# FASCICULE DE BREVET EUROPEEN

(11) **EP 3 319 950 B1**
(45) Date de publication et mention de la délivrance du brevet: **03.11.2021**
(21) Numéro de dépôt: 16750924.9
(22) Date de dépôt: 06.07.2016
(51) Int. Cl.: C07D 295/10, C07C 233/09, C07C 233/65, C07D 213/63, A61K 31/495, A61K 31/4412, A61K 31/16, A61K 8/42, A61P 25/00

(54) **DIAMINES PRIMAIRES VICINALES ASSOCIÉES À DES MOTIFS CHÉLATEURS DE MÉTAUX ET/OU DE RADICAUX LIBRES, ACTIVES CONTRE LES STRESS CARBONYLÉ ET OXYDANT ET LEUR UTILISATION**
VIZINALE PRIMÄRE DIAMINE, DIE MIT CHELATISIERUNGSMOTIVEN VON METALL UND/ODER FREIER RADIKALE ASSOZIIERT UND GEGEN CARBONYLSTRESS UND OXIDATIVEN STRESS AKTIV SIND, UND VERWENDUNG DAVON
VICINAL PRIMARY DIAMINES ASSOCIATED WITH METAL AND/OR FREE RADICAL CHELATION MOTIFS, AND ACTIVE AGAINST CARBONYL AND OXIDATIVE STRESS, AND USE THEREOF

(30) Priorité: 06.07.2015 FR 1556366
(43) Date de publication de la demande: 16.05.2018
(73) Titulaire: Université Amiens Picardie Jules Verne, 80090 Amiens (FR); Centre Hospitalier Universitaire, 80080 Amiens (FR); Centre National de la Recherche Scientifique (CNRS), 75016 Paris (FR); INSERM - Institut National de la Santé et de la Recherche Médicale, 75013 Paris (FR)
(72) Inventeur: SASAKI, Nobumichi André, 80260 Flesselles (FR); SONNET, Pascal, 80480 Pont de Metz (FR); BOULLIER, Agnès, 80000 Amiens (FR); LOHOU, Elodie, 80000 Amiens (FR)
(74) Mandataire: Plasseraud IP
(86) Numéro de dépôt international: PCT/FR2016/051703
(87) Numéro de publication internationale: WO 2017/006048

(56) Documents cités:
- WO-A1-2006/103274
- WO-A1-2013/050721
- WO-A2-00/74664
- US-A1- 2007 155 726
- US-A1- 2010 130 506
- LOHOU ELODIE ET AL: "Multifunctional diamine AGE/ALE inhibitors with potential therapeutical properties against Alzheimer's disease", EUROPEAN JOURNAL OF MEDICINAL CHEMISTRY, EDITIONS SCIENTIFIQUE ELSEVIER, PARIS, FR, vol. 122, 30 April 2016 (2016-04-30), pages 702-722, XP029705907, ISSN: 0223-5234, DOI: 10.1016/J.EJMECH.2016.04.069

## Description

La présente invention concerne de nouveaux dérivés diaminés piégeurs d'alpha-oxoaldéhydes (a-oxoaldéhydes) et d'aldéhydes alpha, béta-insaturés (aldéhydes α,β-insaturés) et chélateurs de métaux ainsi que leurs utilisations, notamment dans le traitement et/ou la prévention de maladies ou affections associées à une accumulation d'AGE (anglais : Advanced Glycation Endproducts) et/ou d'ALE (anglais : Advanced Lipid Peroxydation Endproducts).

### [ETAT DE LA TECHNIQUE]

Lors de la glycolyse et de la peroxydation lipidique, des composés carbonylés sont formés et réagissent avec les groupements nucléophiles des proteines (lysine, arginine, cystéine...) pour donner les AGE ("Advanced Glycation Endproducts" en anglais) (Pathologie, Biologie, 2006, 54, 405-419) et les ALE ("Advanced Lipid Peroxidation Endproducts" en anglais) (Br. J. Pharm., 2008, 153, 6-20). Les AGE apparaissent donc comme des modifications délétères des protéines suite à la formation d'une base de Schiff, suivie d'un réarrangement d'Amadori, de leurs groupements amines libres avec différents dérivés α-oxoaldéhydes tels que par exemple le glyoxal (GO), le méthylglyoxal (MGO) et le déoxyglucosone (3-DG), issus du métabolisme oxydatif des glucides (Diabetes Res. Clin. Pract. 2013, 99, 261-271). De la même façon, les ALE se forment après addition 1,2 ou 1,4 de Michaël de ces groupements aminés des protéines sur des aldéhydes α,β-insaturés tels que le malondialdéhyde (MDA), l'acroléine, le 4-hydroxynonénal (4-HNE) ou le 4-hydroxy-2-hexanal (4-HHE), issus de la dégradation des acides gras polyinsaturés sous l'effet du stress oxydant induit notamment par les métaux de transition (Cu²⁺, Fe³⁺) (Prog. Lipid Res., 2003, 42, 318-343). De plus, ces dérivés carbonylés toxiques peuvent aussi réagir selon un processus non-enzymatique avec les bases de l'ADN pour donner des AGE/ALE ramifiés ou non. Des mécanismes de détoxification enzymatique existent comme le système glyoxalase glutathion (GSH)-dépendant (glyoxalases I et II) qui transforme les composés carbonylés réactifs en D-lactate, glycolate ou acétol, mais leur dysfonctionnement pourra entraîner une accumulation des AGE.

L'accumulation des AGE a deux conséquences biologiques majeures. Tout d'abord, ils peuvent être à l'origine d'une réticulation protéique observée surtout sur les protéines à longue durée de vie telles que par exemple le collagène, les protéines du cristallin, la fibronectine, l'albumine et l'hémoglobine. Ce phénomène joue ainsi un rôle prépondérant dans l'apparition de divers dysfonctionnements (perte de l'élasticité du tissu cutané ou de l'endothélium vasculaire, pigmentation de la peau...) et pathologies liés à l'âge (cataracte, rhumatismes...). Puis, le stress oxydant favoriserait la survenue de réactions inflammatoires et thrombogènes, mais aussi de l'apoptose via une interaction entre les AGE et leurs récepteurs spécifiques (RAGE). Ces mécanismes seraient ainsi notamment impliqués dans l'apparition de l'athérosclérose et de diverses complications notamment microangiopathiques liées au diabète tels que les troubles cardiovasculaires, néphropathies, rétinopathies, neuropathies etc. (Biofactors 2012, 38, 266-274 ; Int. J. Mol, Med., 2010, 26, 813-818 ; Mol. Metabolism, 2014, 3, 94-108). Or, l'accumulation d'AGE associée au déséquilibre oxydatif induit par la surproduction d'espèces réactives de l'oxygène (ERO) et l'essoufflement des systèmes de défense cellulaires antioxydants retrouvé chez le diabétique, sera également à l'origine de l'exacerbation de la peroxydation lipidique (Chem. Biol. Interact., 2008, 171, 251-271). Cette cascade oxydative engendrera donc en parallèle la formation d'ALE tels que le complexe entre l'apolipoprotéine B des LDL (Low Density Lipoproteins) natives et le MDA impliqué dans la génèse des LDL oxydées. Ces dernières seront alors absorbées par des macrophages qui une fois chargés en gouttelettes lipidiques, se transformeront en cellules spumeuses, constitutives de la plaque d'athérome (Int. J. Mol, Med., 2010, 26, 813-818).

De la même façon, suite à l'exacerbation du stress oxydant et de la peroxydation lipidique, on retrouve des taux importants d'ALE associés à des protéines neuronales chez les patients atteints de maladies neurodégénératives telles que par exemple la maladie d'Alzheimer et la maladie de Parkinson. Ainsi, dans le cas de la maladie d'Alzheimer, le modèle physiopathologique de la cascade β-amyloïde tendrait à prouver que l'accumulation des plaques β-amyloïdes qui constituent l'un des marqueurs prédominants de la maladie, serait à l'origine d'une cascade oxydative néfaste (Trends Mol. Med. 2001, 7, 548-554 ; Free Radic. Biol. Med., 2002, 32, 1050-1060). En effet, sous l'influence de divers facteurs génétiques et environnementaux, le clivage par la β-sécrétase du peptide précurseur amyloïde (APP) devient préférentiel au détriment de la voie de dégradation impliquant l'α -sécrétase. Il se forme alors des monomères Aβ non toxiques qui, après agrégation induite par les ions métalliques, se transforment en oligomères toxiques, constitutifs des plaques β-amyloïdes à l'origine d'une exacerbation du stress oxydant (Chem. Eur. J., 2012, 18, 15910-15920 ; Neurochem. Int., 2013, 62, 367-378). Au final, la neurotoxicité des aldéhydes α,β-insaturés alors issus de la peroxydation lipidique, sera largement impliquée dans la survenue de la démence (Curr. Alzheimer Res., 2011, 8, 452-469). Parmi ces derniers, on peut citer l'acroléine qui apparaît comme le composé le plus réactif vis-à-vis des résidus cystéine, histidine et lysine des protéines ou le 4-HNE. L'acroléine entraînera ainsi la formation d'ALE à l'origine d'une altération du cytosquelette et de l'apparition de la dégénérescence neurofibrillaire, autre marqueur important de la maladie d'Alzheimer. Puis, le 4-HNE induira une déstabilisation au niveau de la membrane neuronale notamment après formation d'ALE sur les canaux ioniques et finalement un déséquilibre calcique à l'origine de l'apoptose. De plus, des ALE issus de la condensation du MDA sur des résidus lysine des protéines ont pu être identifiés sous la forme de dérivés dihydropyridine et participeraient à la diminution de la résistance de la peau aux UV qui contribuerait au vieillissement cutané et éventuellement au développement de cancers de la peau (Mech. Ageing Dev., 2001, 122, 735-755). Enfin, le MDA peut également se comporter comme un agent mutagène après réaction sur les fonctions amines des bases de l'ADN et notamment la deoxyguanosine et favoriser ainsi le développement de certains cancers (Toxicology, 2002, 181-182, 219-222). Or, on peut noter que ce type de modification de l'ADN a aussi été décrit dans la littérature sous la forme d'adduits avec le MGO (Chem. Res. Toxicol., 2005, 18, 1586-1592).

Plusieurs agents anti-AGE/ALE ont précédemment été décrits dans la littérature. L'aminoguanidine (Pimagedine®) s'est ainsi révélée être un excellent piégeur de MGO *in vitro* et *in vivo* sur des modèles animaux du diabète (Arch. Biochem. Biophys., 2003, 419, 31-40 ; Diabetes, 2012, 61, 549-559). Cependant, des études cliniques de phase III chez l'homme n'ont pas fourni de résultats concluants montrant de faibles propriétés antioxydantes vasoprotectrices et des effets secondaires hépatiques et gastro-intestinaux (Am. J. Nephrol., 2004, 4, 32-40). Les essais sur cette molécule ont ainsi été abandonnés.

Plusieurs autres composés ont toutefois montré *in vitro* et *in vivo* leur efficacité à ralentir la formation des AGE et des ALE. Nagai et al. (Bioorg. Med. Chem. 2009, 17, 2310-2320) rapportent par exemple les propriétés anti-AGE de la pyridoxamine (Pyridorin®), de la 2,3-diaminophénazine, de la thiamine, de la benfotiamine, du TM-2002, du tenilsetam et des LR-9, 20, 59, 74 et 90 ainsi que le fait que le PTB ("Phenacyl Thiazolium Bromide") et l'ALT-711 se sont montrés capables de détruire les AGE et les ALE. Des effets antiAGE ont également été décrits pour la carnosine (Neurosci. Lett., 1997, 238, 135-13) et l'OPB-9195 (J. Am. Soc. Nephrol., 2000, 11, 1719-1725). On notera que les premiers essais cliniques sur l'OPB-9195 n'ont pas donné de résultats concluants et ont dû être suspendus du fait de la survenue d'importants effets secondaires (Pathologie, Biologie, 2006, 54, 405-419). La plupart des études sur les autres molécules sont toujours au stade expérimental, sauf pour l'ALT-711 pour lequel les essais cliniques ont également été suspendus.

Les demandes de brevets WO 2006/103274 A1 et WO 2013/050721 A1 décrivent des dérivés de l'acide 2,3-diaminopropionique précédemment synthétisés par Sasaki *et al.* ainsi que leurs propriétés anti-AGE/ALE.

Il est important de remarquer que ces différents travaux se sont essentiellement concentrés sur l'élaboration d'agents anti-AGE. En effet, peu d'études concernant le développement de potentiels agents anti-ALE ont jusqu'à présent été réalisées. La conception de composés multipotents à la fois anti-AGE/ALE et chélateurs de métaux n'a à ce jour pas encore été décrite.

Il existe ainsi toujours un besoin de nouveaux composés qui présentent des propriétés anti-AGE/ALE et qui sont de préférence également chélateurs de métaux.

### [RESUME DE L'INVENTION]

Les inventeurs ont maintenant réussi à mettre au point de nouveaux dérivés diaminés piégeurs d'alpha-oxoaldéhydes (α-oxoaldéhydes) et d'aldéhydes alpha, béta-insaturés (aldéhydes α,β-insaturés) et chélateurs de métaux. Ces composés multipotents présentent l'avantage d'associer aux propriétés piégeurs de composés carbonylés, des propriétés chélatrices de métaux et des propriétés antiradicalaires.

L'invention concerne donc des composés de Formule I, leurs sels pharmaceutiquement acceptables ainsi que l'utilisation de ces composés ou leurs sels pharmaceutiquement acceptables dans des compositions pharmaceutiques, agroalimentaires ou cosmétiques.

Dans un premier aspect, l'invention concerne des composés de Formule I : 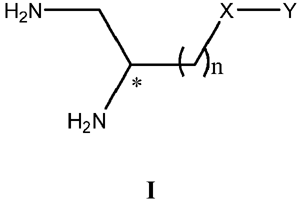
et ses sels, notamment pharmaceutiquement acceptables,
dans laquelle
**n** est un nombre entier de 1 à 6 ;
**X** est CO ou CH₂ ;
**Y** est NR¹R² ou R² ou 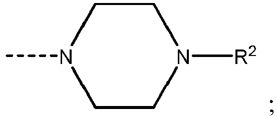
**R¹** est H ou alkyle ou alkyl-aryle ;
**R²** est **Z-L-R³** ;
**Z** est inexistant, CO ou CH₂ ;
**L** est inexistant, CH=CH ou (CH₂)ₘ ;
**m** est un nombre entier de 1 à 6 ;
**R³** est phényle, substitué par au moins un groupe OH et un ou plusieurs substituants choisis parmi OH, alkoxy en C1 à C4 et alkyle en C1 à C4, ou **R³** est N-pyridinonyle, substitué par au moins un groupe OH et éventuellement par un ou plusieurs substituants choisis parmi OH, alkoxy en C1 à C4 et alkyle en C1 à C4.

Les composés de Formule I comportant un atome de carbone asymétrique, ils existent sous forme de deux énantiomères. Ces énantiomères ainsi que leurs mélanges, y compris les mélanges racémiques, font partie de l'invention.

Dans un autre aspect, l'invention concerne des compositions pharmaceutiques, cosmétiques ou agroalimentaires comprenant au moins un composé selon l'invention ou un de ses sels, notamment pharmaceutiquement acceptables et au moins un excipient acceptable au niveau pharmaceutique, cosmétique et/ou agroalimentaire.

Comme indiqué plus haut, les composés de l'invention ainsi que leurs sels pharmaceutiquement acceptables trouvent une application particulière dans le traitement et/ou la prévention de maladies ou affections associées à une accumulation d'AGE (anglais : Advanced Glycation Endproducts) et/ou d'ALE (anglais : Advanced Lipid Peroxydation Endproducts).

### [DESCRIPTION DETAILLEE DE L'INVENTION]

Comme détaillé ci-dessus, l'invention concerne des composés de Formule I ainsi que leurs sels, notamment acceptables au niveau pharmaceutique, cosmétique et/ou agroalimentaire.

Des composés de Formule I et leurs sels préférés sont ceux dans lesquels un, plusieurs ou chacun de **n, Y, R¹**, **R2, Z, L, m** et **R³** sont définis de la manière suivante :
**n** est un nombre entier de 1 à 4 ; de préférence n est 1, 2 ou 3 ; plus préférentiellement encore n est 2 ou 3 ;
**Y** est NR¹R² ou R² ou 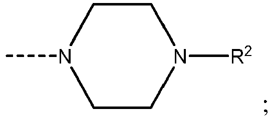
**R¹** est H, méthyle ou benzyle ; de préférence **R¹** est H ou méthyle, plus préferentiellement encore R¹ est H ;
**R²** est **Z-L-R³** ;
**Z** est CO ou CH₂ ;
L est absent, CH=CH ou (CH₂)ₘ ; de préférence L est absent, *trans*-CH=CH ou (CH₂)ₘ ; **m** est un nombre entier de 1 à 6 ; de préférence de 1 à 4, plus préférentiellement encore m est 1, 2 ou 3 ;
**R³** est phényle, substitué par au moins un groupe OH et un ou plusieurs substituants choisis parmi OH, alkoxy en C1 à C4 et alkyle en C1 à C4, ou **R³** est N-pyridinonyle, substitué par au moins un groupe OH et éventuellement par un ou plusieurs substituants choisis parmi OH, alkoxy en C1 à C2 et alkyle en C1 à C2, de préférence choisis parmi OH, méthoxy et méthyle, plus préférentiellement encore parmi OH et méthoxy ou OH et méthyle ou OH, plus préférentiellement encore **R³** est choisi parmi :

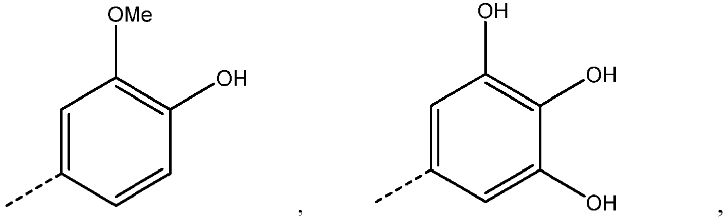

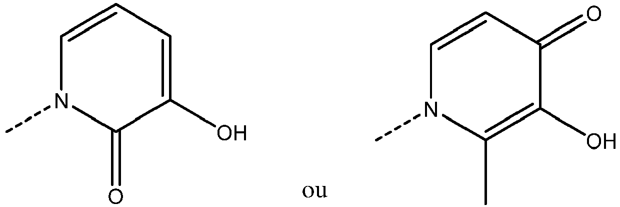

Dans un mode de réalisation particulier, les composés de Formule I et leurs sels sont ceux dans lesquels **R2** est choisi parmi : 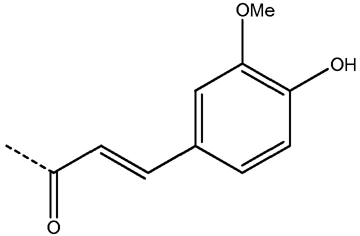 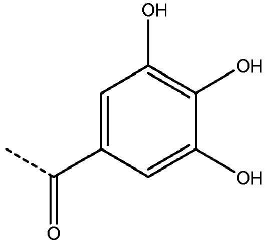 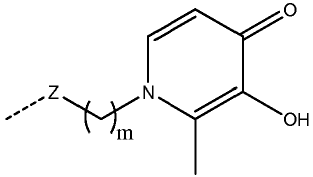 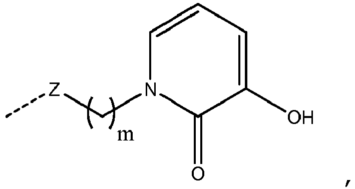 Z étant CO ou CH₂.

En fait et sans vouloir être lié par une quelconque théorie, les inventeurs pensent que la capacité à former des adduits avec le MGO et le MDA et le pouvoir chélateur du cuivre des composés selon l'invention sont obtenus au niveau du groupement diamine. En parallèle, le pouvoir chélateur du fer et les propriétés antiradicalaires des composés selon l'invention sont obtenus grâce aux groupements dérivés de l'acide férulique (4-hydroxy-3-methoxyphényl), de l'acide gallique (3,4,5-trihydroxybenzoyl) et des hydroxypyridinones.

Dans un premier mode de réalisation, les composés de l'invention sont ceux de la Formule II : 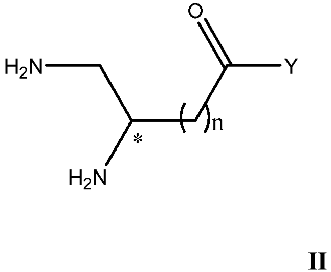
et leurs sels, notamment pharmaceutiquement acceptables,
dans laquelle n et Y sont tels que définis ci-dessus par rapport à la Formule I.

Dans un deuxième mode de réalisation, les composés de l'invention sont ceux de la Formule III 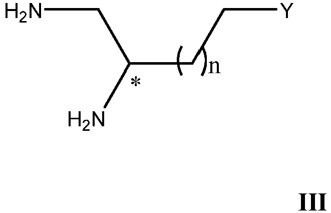
et leurs sels, notamment pharmaceutiquement acceptables,
dans laquelle n et Y sont tels que définis ci-dessus par rapport à la Formule I.

Dans un troisième mode de réalisation, les composés de l'invention sont ceux de la Formule IV 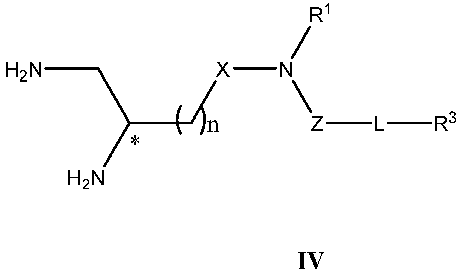
et leurs sels, notamment pharmaceutiquement acceptables,
dans laquelle n, X, R1, Z, L et R3 sont tel que définis par rapport à la Formule I.

Des composés préférés de Formule IV sont ceux dans lesquels Z-L-R³ est choisi parmi : 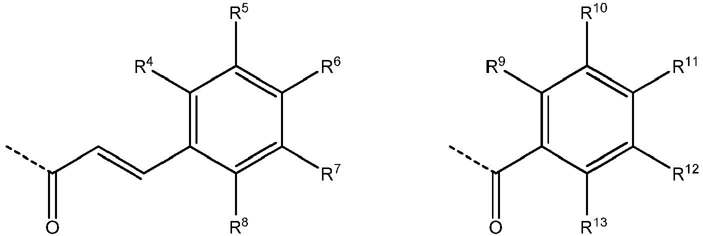 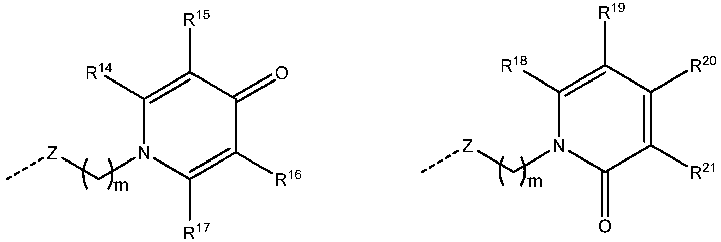 dans lesquels R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ et R²¹ sont choisis, indépendamment les uns des autres, parmi H, OH, alkoxy en C1 à C4 et alkyle en C1 à C4, de préférence parmi H, OH, alkoxy en C1 à C2 et alkyle en C1 à C2, préférentiellement parmi H, OH, méthoxy et méthyle, plus préférentiellement encore parmi H, OH et méthoxy ; H ou OH et H, méthyle ou OH ; à condition qu'au moins un de R⁴, R⁵, R⁶, R⁷ et R⁸, de R⁹, R¹⁰, R¹¹, R¹² et R¹³, de R¹⁴, R¹⁵, R¹⁶ et R¹⁷, de R¹⁸, R¹⁹, R²⁰ et R²¹ est OH.

Des composés particulièrement préférés de Formule IV sont ceux dans lesquels Z-L-R³ est choisi parmi : 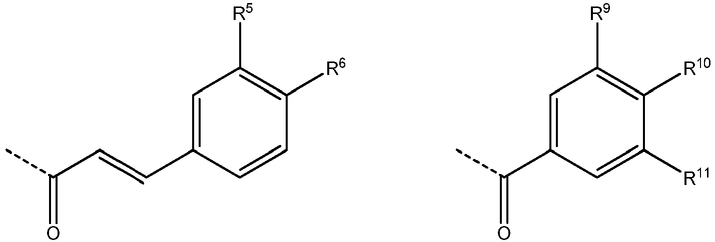 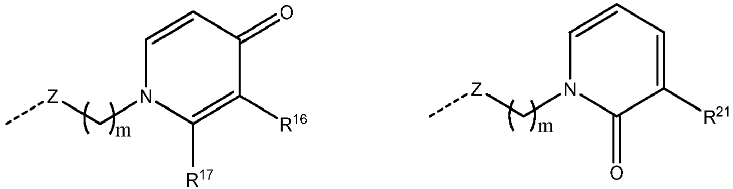 dans lesquels R⁵, R⁶, R⁹, R¹⁰, R¹¹, R¹⁶, R¹⁷ et R²¹ sont choisis, indépendamment les uns des autres, parmi OH, alkoxy en C1 à C4 et alkyle en C1 à C4, de préférence parmi OH, alkoxy en C1 à C2 et alkyle en C1 à C2, préférentiellement parmi OH, méthoxy et méthyle, plus préférentiellement encore parmi OH et méthoxy ou OH et méthyle ou OH ; à condition qu'au moins un de R⁵ et R⁶, de R⁹, R¹⁰ et R¹¹, de R¹⁶ et R¹⁷ et de R²¹ est OH. Des composés particulièrement avantageux de Formule IV sont ceux dans lesquels
- R⁵ et R⁶ sont choisis, indépendamment l'un de l'autre, parmi OH et alkoxy en C1 à C4, de préférence parmi OH et alkoxy en C1 à C2, plus préférentiellement encore parmi OH et méthoxy ; avantageusement R⁵ est alkoxy en C1 à C4, de préférence alkoxy en C1 à C2, plus préférentiellement encore méthoxy et R⁶ est OH ;
- R⁹, R¹⁰ et R¹¹ sont OH ;
- R¹⁶ et R¹⁷ sont choisis, indépendamment l'un de l'autre, parmi OH et alkyle en C1 à C4, de préférence parmi OH et alkyle en C1 à C2, plus préférentiellement encore parmi OH et méthyle ; avantageusement R¹⁶ est OH et R¹⁷ est alkyle en C1 à C4, de préférence alkyle en C1 à C2, plus préférentiellement encore méthyle ;
- R²¹ est OH.

Dans un mode de réalisation, les composés de Formule IV sont ceux dans lesquels Z-L-R³ est choisi parmi : 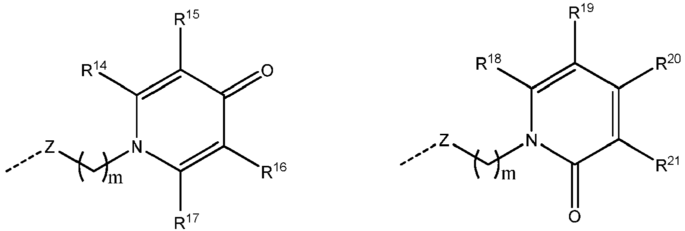 dans lesquels R¹⁴, R¹⁵, R¹⁶, R¹⁷, R¹⁸, R¹⁹, R²⁰ et R²¹ sont choisis, indépendamment les uns des autres, parmi H, OH et alkyle en C1 à C4, de préférence parmi H, OH et alkyle en C1 à C2, préférentiellement parmi H, OH et méthyle ; à condition qu'au moins un de R¹⁴, R¹⁵, R¹⁶ et R¹⁷ et de R¹⁸, R¹⁹, R²⁰ et R²¹ est OH. Avantageusement Z-L-R³ est choisi parmi : 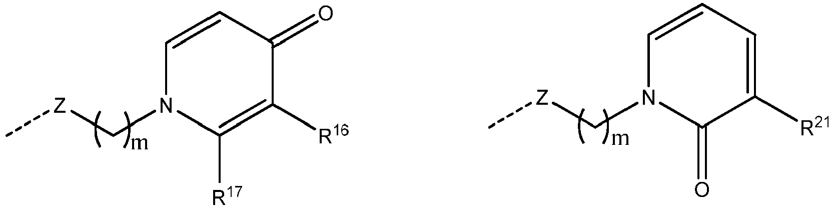 dans lesquels R¹⁶, R¹⁷ et R²¹ sont choisis, indépendamment les uns des autres, parmi OH et alkyle en C1 à C4, de préférence parmi OH et alkyle en C1 à C2, plus préférentiellement encore parmi OH et méthyle ; à condition qu'au moins un de R¹⁶ et R¹⁷ et de R²¹ est OH. Des composés particulièrement avantageux sont ceux dans lesquels
- R¹⁶ et R¹⁷ sont choisis, indépendamment l'un de l'autre, parmi OH et alkyle en C1 à C4, de préférence parmi OH et alkyle en C1 à C2, plus préférentiellement encore parmi OH et méthyle ; avantageusement R¹⁶ est OH et R¹⁷ est alkyle en C1 à C4, de préférence alkyle en C1 à C2, plus préférentiellement encore méthyle ;
- R²¹ est OH.

Dans un quatrième mode de réalisation, les composés de l'invention sont ceux de la Formule V 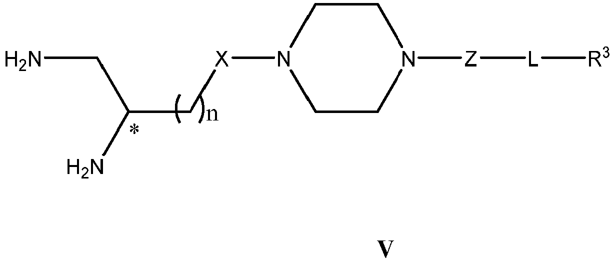
et leurs sels, notamment pharmaceutiquement acceptables,
dans laquelle n, X, Z, L et R³ sont tel que définis par rapport à la Formule I.

Des composés préférés de Formule V sont ceux dans lesquels Z-L-R³ est choisi parmi : 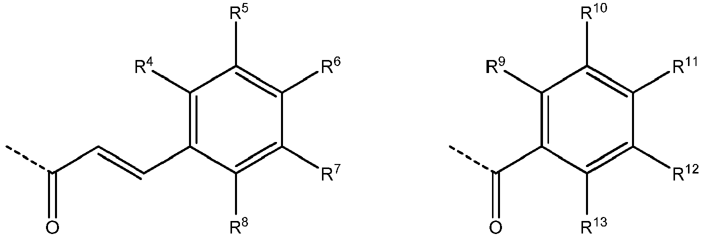 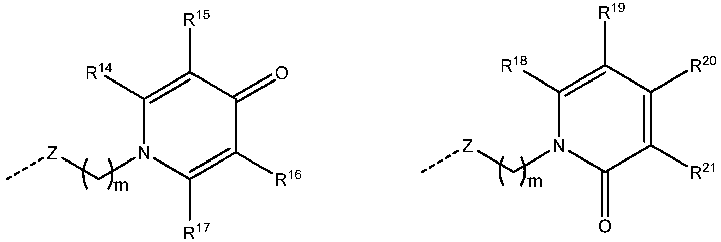 dans lesquels R⁴, R⁵, R⁶, R⁷ R⁸, R⁹, R¹⁰, R¹¹, R¹², R¹³, R¹⁴, R¹⁵, R¹⁶, R¹⁷, R¹⁸, R¹⁹, R²⁰ et R²¹ sont choisis, indépendamment les uns des autres, parmi H, OH, alkoxy en C1 à C4 et alkyle en C1 à C4, de préférence parmi H, OH, alkoxy en C1 à C2 et alkyle en C1 à C2, préférentiellement parmi H, OH, méthoxy et méthyle, plus préférentiellement encore parmi H, OH et méthoxy ; H ou OH et H, méthyle ou OH ; à condition qu'au moins l'un de R⁴, R⁵, R⁶, R⁷ et R⁸, au moins l'un de R⁹, R¹⁰, R¹¹, R¹² et R¹³, au moins l'un de R¹⁴, R¹⁵, R¹⁶ et R¹⁷, et au moins l'un de R¹⁸, R¹⁹, R²⁰ et R²¹ est OH.

Des composés particulièrement préférés de Formule V sont ceux dans lesquels Z-L-R³ est choisi parmi : 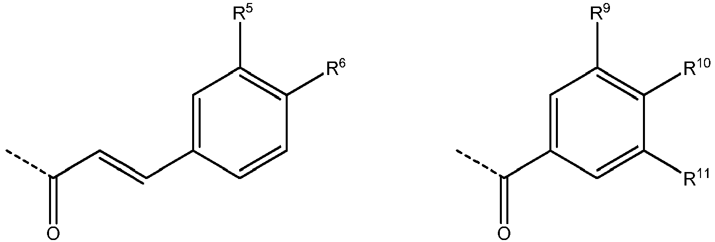 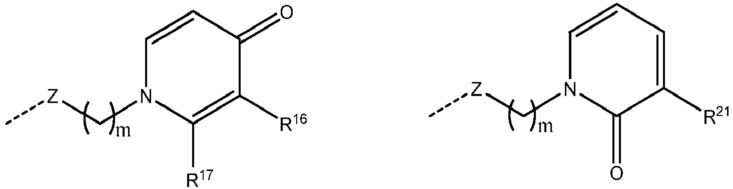 dans lesquels R⁵, R⁶, R⁹, R¹⁰, R¹¹, R¹⁶, R¹⁷ et R²¹ sont choisis, indépendamment les uns des autres, parmi OH, alkoxy en C1 à C4 et alkyle en C1 à C4, de préférence parmi OH, alkoxy en C1 à C2 et alkyle en C1 à C2, préférentiellement parmi OH, méthoxy et méthyle, plus préférentiellement encore parmi OH et méthoxy ou OH et méthyle ou OH ; à condition qu'au moins l'un de R⁵ et R⁶, au moins l'un de R⁹, R¹⁰ et R¹¹, au moins l'un de R¹⁶ et R¹⁷ est OH et R²¹ est OH. Des composés particulièrement avantageux de Formule IV sont ceux dans lesquels
- R⁵ et R⁶ sont choisis, indépendamment l'un de l'autre, parmi OH et alkoxy en C1 à C4, de préférence parmi OH et alkoxy en C1 à C2, plus préférentiellement encore parmi OH et méthoxy ; avantageusement R⁵ est alkoxy en C1 à C4, de préférence alkoxy en C1 à C2, plus préférentiellement encore méthoxy et R⁶ est OH ;
- R⁹, R¹⁰ et R¹¹ sont OH ;
- R¹⁶ et R¹⁷ sont choisis, indépendamment l'un de l'autre, parmi OH et alkyle en C1 à C4, de préférence parmi OH et alkyle en C1 à C2, plus préférentiellement encore parmi OH et méthyle ; avantageusement R¹⁶ est OH et R¹⁷ est alkyle en C1 à C4, de préférence alkyle en C1 à C2, plus préférentiellement encore méthyle ;
- R²¹ est OH.

Dans un mode de réalisation, les composés de Formule V sont ceux dans lesquels Z-L-R³ est choisi parmi : 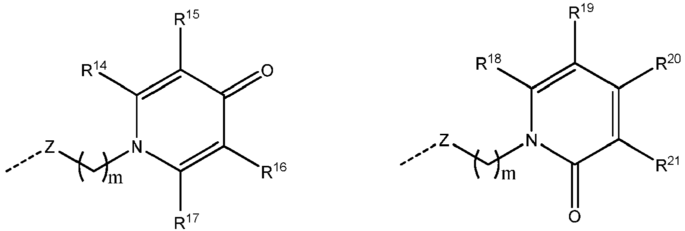 dans lesquels R¹⁴, R¹⁵, R¹⁶, R¹⁷, R¹⁸ , R¹⁹, R²⁰ et R²¹ sont choisis, indépendamment les uns des autres, parmi H, OH et alkyle en C1 à C4, de préférence parmi H, OH et alkyle en C1 à C2, préférentiellement parmi H, OH et méthyle ; à condition qu'au moins l'un de R¹⁴, R¹⁵, R¹⁶ et R¹⁷ et au moins l'un de R¹⁸, R¹⁹, R²⁰ et R²¹ est OH. Avantageusement Z-L-R³ est choisi parmi : 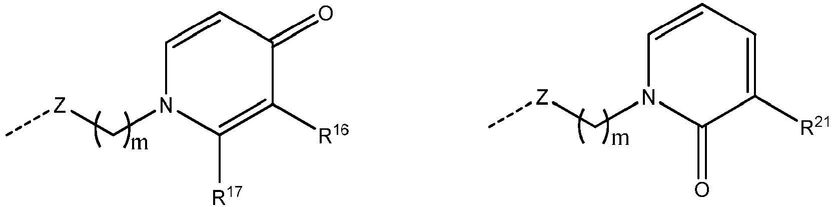 dans lesquels R¹⁶, R¹⁷ et R²¹ sont choisis, indépendamment les uns des autres, parmi OH et alkyle en C1 à C4, de préférence parmi OH et alkyle en C1 à C2, plus préférentiellement encore parmi OH et méthyle ; à condition qu'au moins l'un de R¹⁶ et R¹⁷ est OH et R²¹ est OH. Des composés particulièrement avantageux sont ceux dans lesquels
- R¹⁶ et R¹⁷ sont choisis, indépendamment l'un de l'autre, parmi OH et alkyle en C1 à C4, de préférence parmi OH et alkyle en C1 à C2, plus préférentiellement encore parmi OH et méthyle ; avantageusement R¹⁶ est OH et R¹⁷ est alkyle en C1 à C4, de préférence alkyle en C1 à C2, plus préférentiellement encore méthyle ;
- R²¹ est OH.

Dans un cinquième mode de réalisation, les composés de l'invention sont ceux de la Formule VI 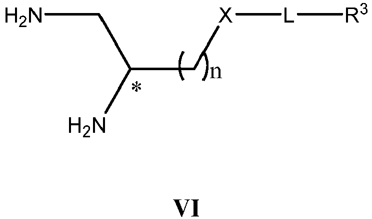
et leurs sels, notamment pharmaceutiquement acceptables,
dans laquelle n, X, L et R³ sont tel que définis par rapport à la Formule I.

Des composés préférés de Formule VI sont ceux dans lesquels n est 1 ou 2, X est CH₂ et/ou L-R³ est 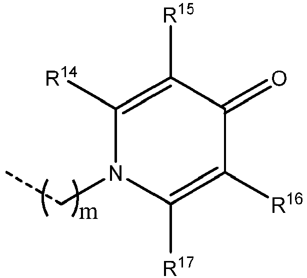 dans lequel R¹⁴, R¹⁵, R¹⁶ et R¹⁷ sont choisis, indépendamment les uns des autres, parmi H, OH et alkyle en C1 à C4, de préférence parmi H, OH et alkyle en C1 à C2, préférentiellement parmi H, OH et méthyle ; à condition qu'au moins l'un de R¹⁴, R¹⁵, R¹⁶ et R¹⁷ est OH. Avantageusement L-R³ est choisi parmi :

| |
|---|
| 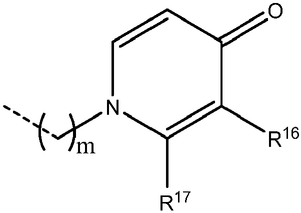 |

dans lesquels R¹⁶ et R¹⁷ sont choisis, indépendamment les uns des autres, parmi OH et alkyle en C1 à C4, de préférence parmi OH et alkyle en C1 à C2, plus préférentiellement encore parmi OH et méthyle ; à condition qu'au moins l'un de R¹⁶ et R¹⁷ est OH. Des composés particulièrement avantageux sont ceux dans lesquels R¹⁶ est OH et R¹⁷ est alkyle en C1 à C4, de préférence alkyle en C1 à C2, plus préférentiellement encore méthyle.

Des composés particulièrement préférés de l'invention sont ceux listés dans le Tableau 1 ci-dessous :

**Tableau 1**

| ***Structure*** | ***Nom*** |
|---|---|
| 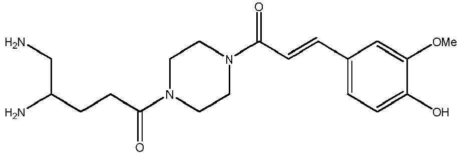 | (E)-4,5-diamino-1-(4-(3-(4-hydroxy-3-méthoxyphényl)acryloyl)pipérazin-1-yl)pentan-1-one |
| 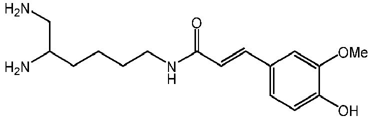 | (E)-*N*-(5,6-diaminohexyl)-3-(4-hydroxy-3-méthoxyphényl)acrylamide |
| 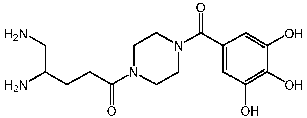 | 4,5-diamino-1-(4-(3,4,5-trihydroxybenzoyl)pipérazin-1-yl)pentan-1-one |
| 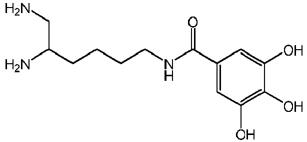 | *N*-(5,6-diaminohexyl)-3,4,5-trihydroxybenzamide |
| 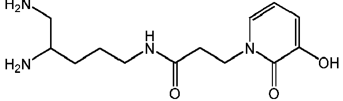 | *N*-(4,5-diaminopentyl)-3-(3-hydroxy-2-oxopyridin-1(2*H*)-yl)propanamide |
| 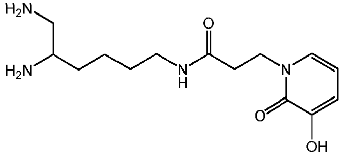 | *N*-(5,6-diaminohexyl)-3-(3-hydroxy-2-oxopyridin-1(2*H*)-yl)propanamide |
| 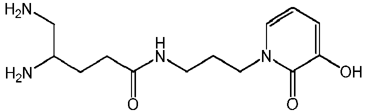 | 4,5-diamino-*N*-(3-(3-hydroxy-2-oxopyridin-1(2*H*)-yl)propyl)pentanamide |
| 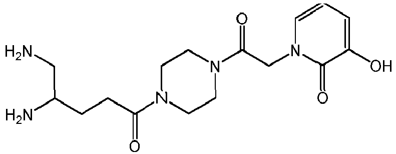 | 1-(2-(4-(4,5-diaminopentanoyl)pipérazin-1-yl)-2-oxoéthyl)-3-hydroxypyridin-2(1*H*)-one |
| 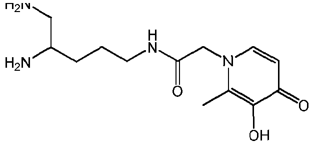 | *N*-(4,5-diaminopentyl)-2-(3-hydroxy-2-méthyl-4-oxopyridin-1(4*H*)-yl)acetamide |
| 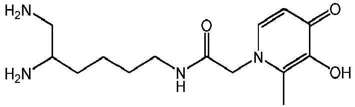 | *N*-(5,6-diaminohexyl)-2-(3-hydroxy-2-méthyl-4-oxopyridin-1(4*H*)-yl)acetamide |
| 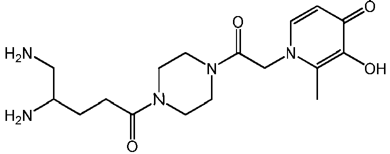 | 1-(2-(4-(4,5-diaminopentanoyl)pipérazin-1 -yl)-2-oxoéthyl)-3-hydroxy-2-méthylpyridin-4(1*H*)-one |
| 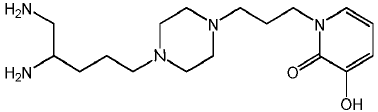 | 1-(3-(4-(4,5-diaminopentyl)pipérazin-1-yl)propyl)-3-hydroxypyridin-2(1*H*)-one |
| 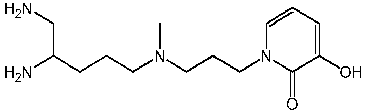 | 1-(3-((4,5-diaminopentyl)(méthyl)amino)propyl)-3-hydroxypyridin-2(1*H*)-one |
| 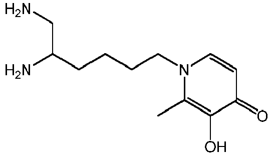 | 1-(5,6-diaminohexyl)-3-hydroxy-2-méthylpyridin-4(1*H*)-one |

Les composes de Formule I peuvent être préparés selon des réactions connues de l'Homme du métier. Les schémas réactionnels décrits dans la partie « Exemples » illustrent des approches de synthèse possibles.

Du fait de leur capacité à piéger des α-oxoaldéhydes et/ou des aldéhydes α,β-insaturés, leur capacité à chélater des métaux et leurs propriétés anti-oxydantes, les composés de l'invention et leurs sels, notamment acceptables au niveau pharmaceutique et/ou cosmétique, trouvent une application dans le domaine pharmaceutique et/ou cosmétique.

Dans un deuxième aspect, l'invention concerne donc les composés de l'invention pour utilisation comme médicament.

Plus particulièrement, les composés de l'invention sont ainsi utiles dans le traitement et/ou la prévention de maladies ou affections associées à une accumulation de produits de glycation avancés (AGE, en anglais : Advanced Glycation Endproducts) et/ou de produits de peroxydation de lipides (ALE, en anglais : Advanced Lipid Peroxydation Endproducts).

Ces maladies ou affections comprennent les maladies neurodégénératives, les micro- et macroangiopathies liées aux stress oxydant et carbonylé, les affections liées au diabète et les pathologies liées à l'âge.

Dans un mode de réalisation particulier, les maladies ou affections sont choisies parmi les maladies neurodégénératives, notamment parmi la maladie d'Alzheimer et la maladie de Parkinson.

Dans un autre mode de réalisation particulier, les maladies ou affections sont choisies parmi les micro- et macroangiopathies liées aux stress oxydant et carbonylé de type athérosclérose.

Dans un autre mode de réalisation particulier, les maladies ou affections sont choisies parmi les affections liées au diabète, notamment parmi l'athérosclérose, la rétinopathie, la néphropathie, la neuropathie, les micro et macroangiopathies, la cataracte, l'amyloïdose, les troubles rhumatismaux et les ulcères variqueux et artériels.

Dans un autre mode de réalisation particulier, les maladies ou affections sont choisies parmi les pathologies liées à l'âge telles que par exemple la cataracte et les rhumatismes.

La présente invention, selon un autre de ses aspects, concerne également une méthode de traitement des maladies et affections ci-dessus indiquées comprenant l'administration, à un patient, d'une dose efficace d'un composé selon l'invention, ou d'un de ses solvates pharmaceutiquement acceptables. De préférence, le patient est un animal à sang chaud, plus préférentiellement un humain.

L'invention concerne également une composition pharmaceutique comprenant au moins un composé de l'invention ou au moins un sel pharmaceutiquement acceptable dudit composé et un excipient pharmaceutiquement acceptable. Lesdits excipients sont choisis selon la forme pharmaceutique et le mode d'administration souhaité, parmi les excipients habituels qui sont connus de l'Homme du métier.

La composition pharmaceutique de la présente invention peut être choisie parmi les compositions pharmaceutiques pour administration orale, sublinguale, sous-cutanée, intramusculaire, intra-veineuse, topique, locale, intratrachéale, intranasale, transdermique ou rectale. Dans ces compositions, le principe actif de Formule I ci-dessus, ou son solvate pharmaceutiquement acceptable, peut être administré sous forme unitaire d'administration, en mélange avec des excipients pharmaceutiques classiques, aux animaux et aux êtres humains pour le traitement et/ou la prévention des maladies ou affections ci-dessus indiquées. Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions. Dans un mode de réalisation préféré, il s'agit d'une composition pharmaceutique pour administration orale. De telles formes d'administration appropriées qui peuvent se présenter sous forme solide, semi-solide ou liquide en fonction du mode d'administration, sont généralement connues de l'Homme du métier, référence étant faite à la dernière édition de l'ouvrage « Remington's Pharmaceutical Sciences ».

L'accumulation des AGE étant également liée à des dysfonctionnements ayant un impact cosmétique, tels que la perte de l'élasticité du tissu cutané ou de l'endothélium vasculaire et la pigmentation de la peau (Dermatoendocrinol. 2012, 4, 259-270), les composés de l'invention sont utiles comme principe actif dans des compositions cosmétiques.

### [DEFINITIONS]

Les définitions et explications ci-dessous concernent les termes et expressions telles qu'utilisés dans la présente demande, comprenant la description ainsi que les revendications.

Pour la description des composés de l'invention, les termes et expressions utilisées doivent, sauf indication contraire, être interprétés selon les définitions ci-après.

Le terme « alkyl(e) », seul ou en tant que partie d'un autre groupement, désigne un radical hydrocarbure de formule CₙH₂ₙ₊₁ dans lequel n est un nombre entier supérieur ou égal à 1.

Le terme « sel » désigne les sels d'addition d'acides des composés de Formule I. Il englobe les sels avec des acides inorganiques et organiques, tels que l'acide chlorhydrique, l'acide nitrique, l'acide sulfurique, l'acide phosphorique, l'acide citrique, l'acide formique, l'acide fumarique, l'acide maléique, l'acide acétique, l'acide succinique, l'acide tartrique, l'acide méthanesulfonique, l'acide p-toluènesulfonique, l'acide oxalique et analogues.

Toutes les références à des composés de Formule I désignent également les sels de ceux-ci.

Le terme « patient » désigne un animal à sang chaud, de préférence un humain, qui attend ou reçoit un traitement médical.

Le terme « humain » désigne des sujets des deux sexes et à tout stade de développement (c'est-à-dire néonatal, infantile, juvénile, adolescent et adulte). Dans un mode de réalisation, il s'agit d'un adolescent ou d'un adulte, de préférence d'un adulte.

Les termes « traiter » et « traitement » doivent être entendus dans leur signification générale et comprennent ainsi l'amélioration et l'abrogation d'un état pathologique.

Les termes « prévenir » et « prévention » désignent le fait d'éviter ou de retarder l'apparition d'une maladie ou affection et des symptômes liés, ainsi excluant un patient de développer une maladie ou affection ou réduisant le risque d'un patient de développer une maladie ou affection.

Le terme « dose thérapeutiquement efficace » ou « dose efficace » désigne la dose de principe actif (composé de Formule I) qui est suffisant pour atteindre le résultat thérapeutique ou prophylactique désiré chez le patient auquel il est administré.

Le terme « pharmaceutiquement acceptable » ou « acceptable au niveau pharmaceutique » désigne qu'un composé ou un composant n'est pas nocif pour le patient et que dans le cadre d'une composition pharmaceutique il est compatible avec les autres composants.

Le terme « acceptable au niveau cosmétique » désigne qu'un composé ou un composant n'est pas nocif pour l'utilisateur, notamment un humain, et que dans le cadre d'une composition cosmétique il est compatible avec les autres composants.

Le terme « acceptable au niveau agroalimentaire » désigne qu'un composé ou un composant n'est pas nocif pour un animal à sang chaud, notamment pour un humain lors de son ingestion et que dans le cadre d'une composition agroalimentaire il est compatible avec les autres composants.

La présente invention sera mieux comprise en référence aux exemples suivants. Ces exemples sont représentatifs de certains modes de réalisation de l'invention et ne limitent en aucun cas la portée de l'invention. Les figures servent à illustrer les résultats expérimentaux.

### FIGURES (les composés 37d, 37f, 37i et 37j sont des composés de référence et ne font pas partie de l'invention)

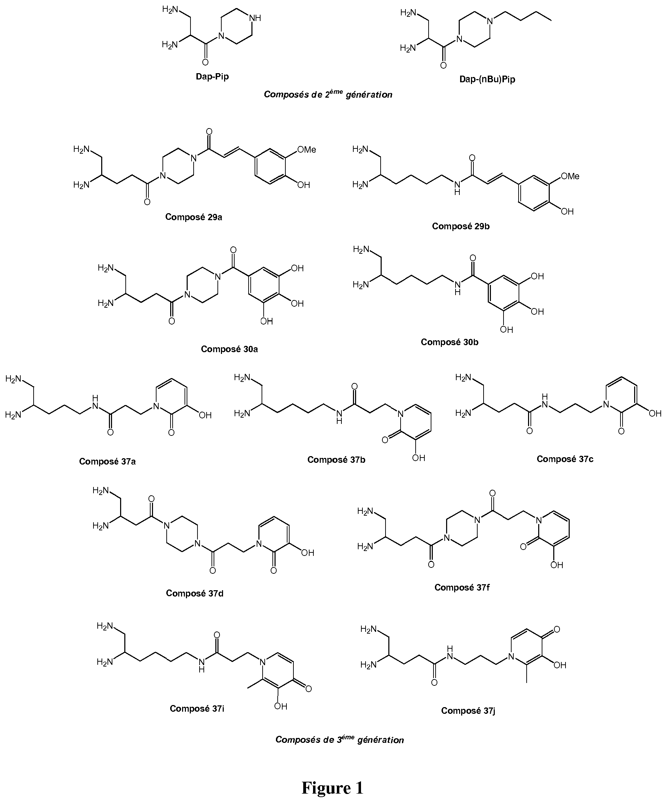

**Figure 1** **:** Structure des différents composés étudiés

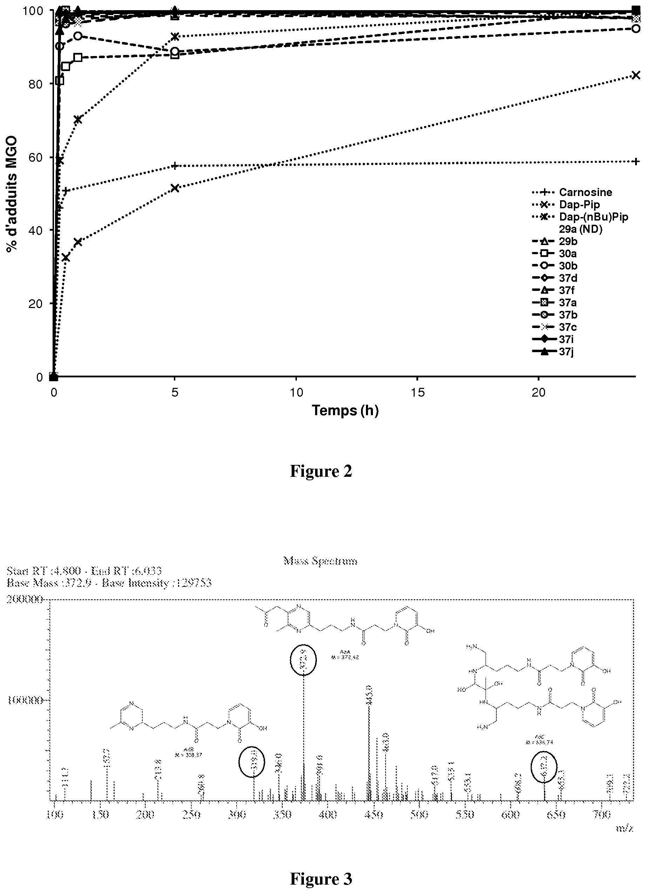

**Figure 2** **:** Étude cinétique de la formation d'adduits entre le MGO et les composés selon l'invention
**Figure 3** **:** Spectre de masse attestant de la formation d'au moins trois types d'adduits entre le MGO et le composé **37a** après 15 min d'incubation à 37°C

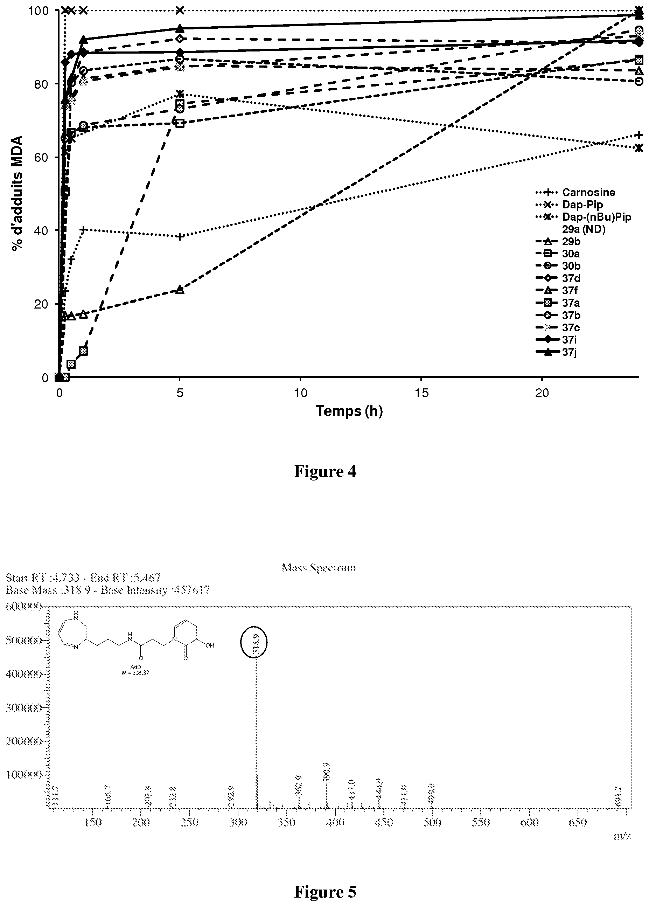

**Figure 4** **:** Étude cinétique de la formation d'adduits entre le MDA et les composés selon l'invention
**Figure 5** **:** Spectre de masse attestant de la formation d'au moins un adduit entre le MDA et le composé **37a** après 5h d'incubation à 37°C

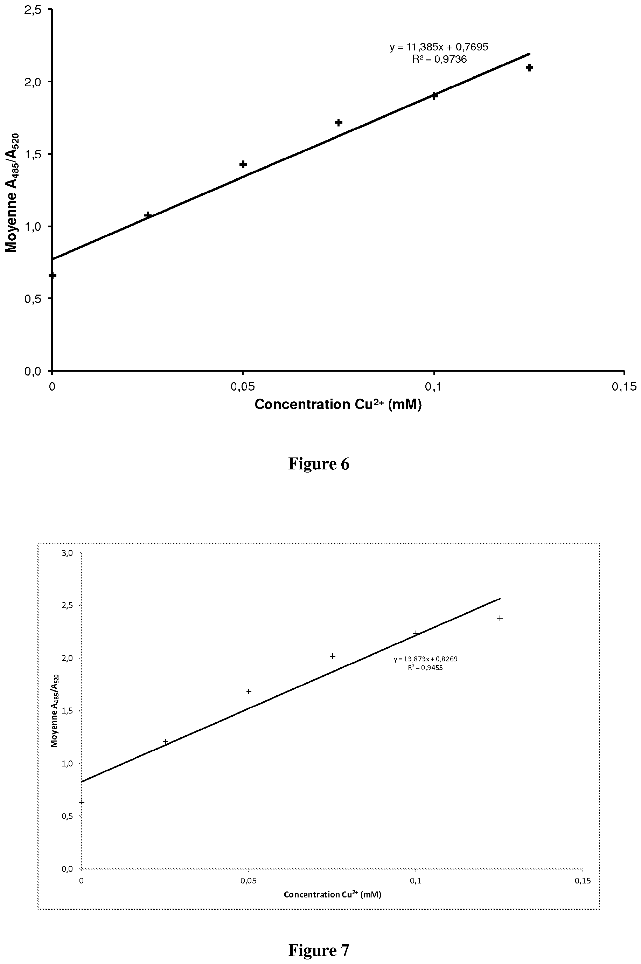

**Figure 6** **:** Courbe de calibration réalisée dans du tampon Hexamine 0,01 M / KCl 0,01 M (pH = 5)
**Figure 7** **:** Courbe de calibration réalisée dans un mélange de tampon Hexamine 0,01 M / KCl 0,01 M (pH = 5) et de MeOH 75/25

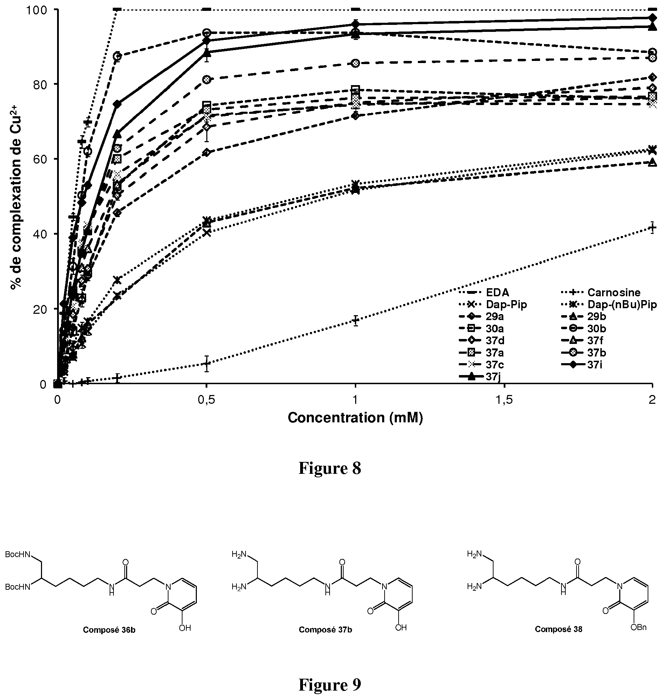

**Figure 8** **:** Comparaison des % de complexation du Cu²⁺ de différents composés selon l'invention en fonction de leur concentration
**Figure 9** **:** Deux composés apparentés au composé **37b** testés pour leurs propriétés chélatrices du Cu²⁺

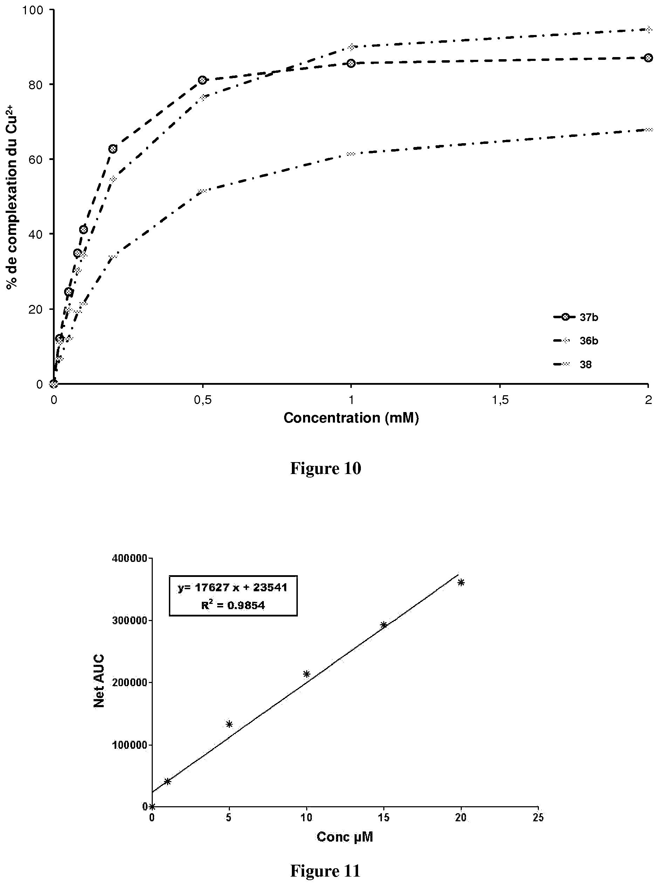

**Figure 10** **:** Comparaison des % de complexation du Cu²⁺ du composé **37b** et de deux composés apparentés présentant seulement une extrémité chélatrice du Cu²⁺ libre en fonction de leur concentration
**Figure 11** **:** Courbe de calibration du trolox (Net AUC *vs* concentration). Les valeurs représentées sont exprimées par la moyenne ± SEM de triplicatas constituant une expérience représentative parmi les trois expériences indépendantes réalisées.

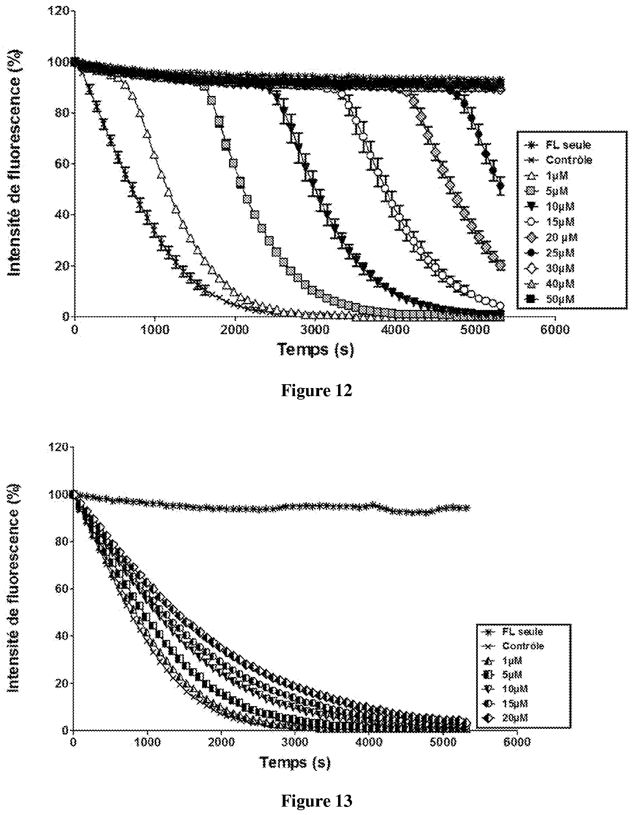

**Figure 12****:** Courbe de décroissance de la fluorescence de la fluorescéine induite par l'AAPH. Les valeurs représentées concernent les résultats obtenus en l'absence (contrôle) ou en présence de trolox à différentes concentrations et sont exprimées par la moyenne ± SEM de triplicatas constituant une expérience représentative parmi les trois expériences indépendantes réalisées.
**Figure 13** **:** Courbe de décroissance de la fluorescence de la fluorescéine induite par l'AAPH. Les valeurs représentées concernent les résultats obtenus en l'absence (contrôle) ou en présence de carnosine à différentes concentrations et sont exprimées par la moyenne ± SEM de triplicatas constituant une expérience représentative parmi les trois expériences indépendantes réalisées.

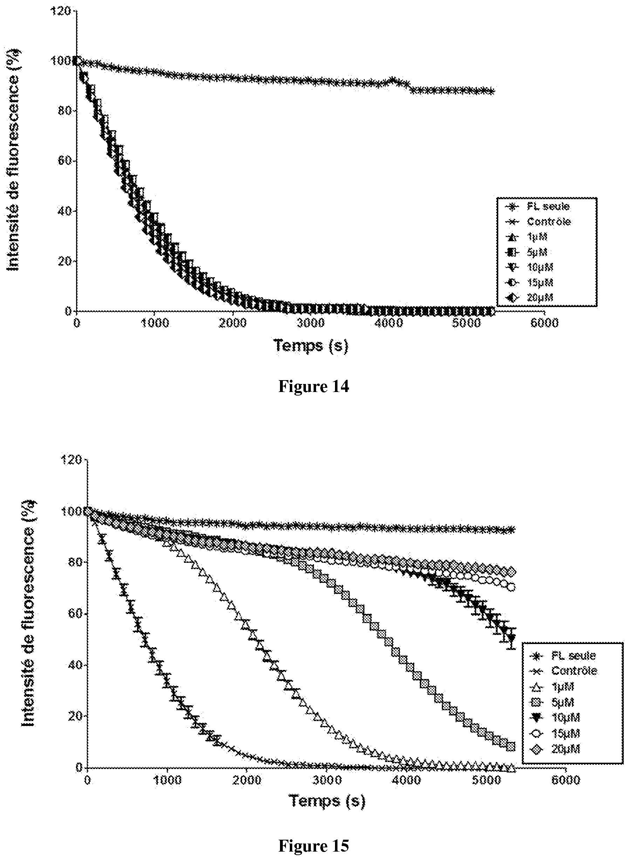

**Figure 14** **:** Courbe de décroissance de la fluorescence de la fluorescéine induite par l'AAPH. Les valeurs représentées concernent les résultats obtenus en l'absence (contrôle) ou en présence de Dap-Pip à différentes concentrations et sont exprimées par la moyenne ± SEM de triplicatas constituant une expérience représentative parmi les trois expériences indépendantes réalisées.
**Figure 15** **:** Courbe de décroissance de la fluorescence de la fluorescéine induite par l'AAPH. Les valeurs représentées concernent les résultats obtenus en l'absence (contrôle) ou en présence du composé **29a** à différentes concentrations et sont exprimées par la moyenne ± SEM de triplicatas constituant une expérience représentative parmi les trois expériences indépendantes réalisées.

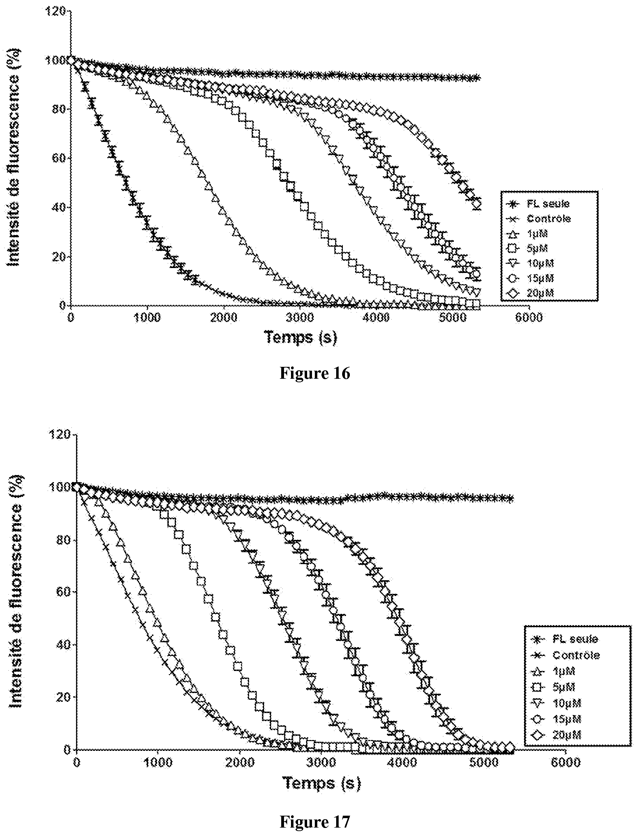

**Figure 16** **:** Courbe de décroissance de la fluorescence de la fluorescéine induite par l'AAPH. Les valeurs représentées concernent les résultats obtenus en l'absence (contrôle) ou en présence du composé **29b** à différentes concentrations et sont exprimées par la moyenne ± SEM de triplicatas constituant une expérience représentative parmi les trois expériences indépendantes réalisées.
**Figure 17** **:** Courbe de décroissance de la fluorescence de la fluorescéine induite par l'AAPH. Les valeurs représentées concernent les résultats obtenus en l'absence (contrôle) ou en présence du composé **30a** à différentes concentrations et sont exprimées par la moyenne ± SEM de triplicatas constituant une expérience représentative parmi les trois expériences indépendantes réalisées.

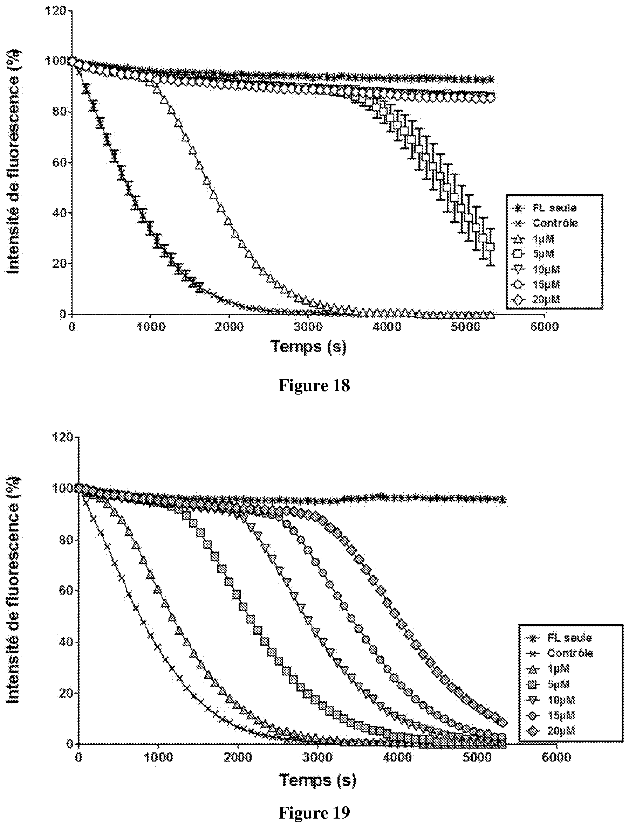

**Figure 18** **:** Courbe de décroissance de la fluorescence de la fluorescéine induite par l'AAPH. Les valeurs représentées concernent les résultats obtenus en l'absence (contrôle) ou en présence du composé **30b** à différentes concentrations et sont exprimées par la moyenne ± SEM de triplicatas constituant une expérience représentative parmi les trois expériences indépendantes réalisées.
**Figure 19** **:** Courbe de décroissance de la fluorescence de la fluorescéine induite par l'AAPH. Les valeurs représentées concernent les résultats obtenus en l'absence (contrôle) ou en présence du composé **37a** à différentes concentrations et sont exprimées par la moyenne ± SEM de triplicatas constituant une expérience représentative parmi les trois expériences indépendantes réalisées.

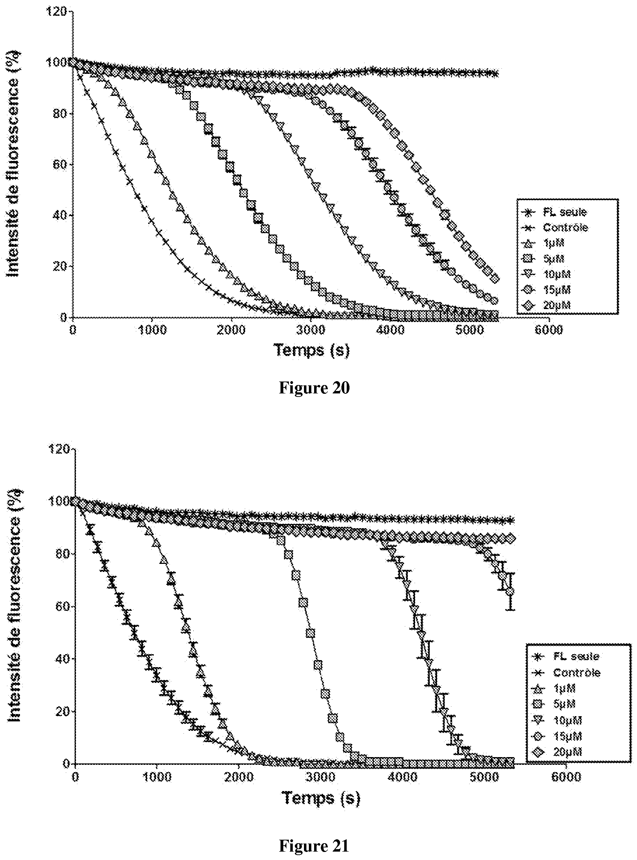

**Figure 20** : Courbe de décroissance de la fluorescence de la fluorescéine induite par l'AAPH. Les valeurs représentées concernent les résultats obtenus en l'absence (contrôle) ou en présence du composé **37b** à différentes concentrations et sont exprimées par la moyenne ± SEM de triplicatas constituant une expérience représentative parmi les trois expériences indépendantes réalisées.
**Figure 21** : Courbe de décroissance de la fluorescence de la fluorescéine induite par l'AAPH. Les valeurs représentées concernent les résultats obtenus en l'absence (contrôle) ou en présence du composé **37c** à différentes concentrations et sont exprimées par la moyenne ± SEM de triplicatas constituant une expérience représentative parmi les trois expériences indépendantes réalisées.

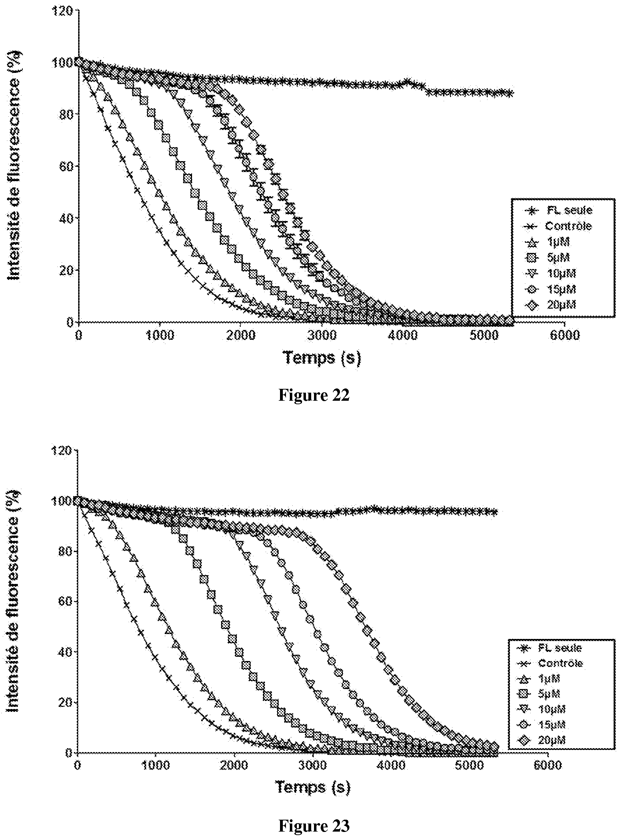

**Figure 22** : Courbe de décroissance de la fluorescence de la fluorescéine induite par l'AAPHLes valeurs représentées concernent les résultats obtenus en l'absence (contrôle) ou en présence du composé **37d** à différentes concentrations et sont exprimées par la moyenne ± SEM de triplicatas constituant une expérience représentative parmi les trois expériences indépendantes réalisées.
**Figure 23** : Courbe de décroissance de la fluorescence de la fluorescéine induite par l'AAPH. Les valeurs représentées concernent les résultats obtenus en l'absence (contrôle) ou en présence du composé **37f** à différentes concentrations et sont exprimées par la moyenne ± SEM de triplicatas constituant une expérience représentative parmi les trois expériences indépendantes réalisées.

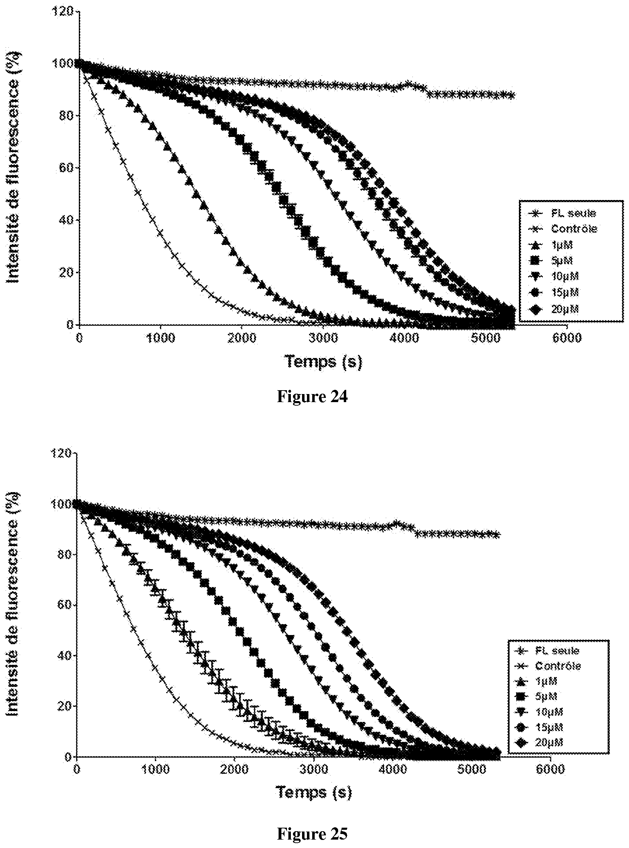

**Figure 24** : Courbe de décroissance de la fluorescence de la fluorescéine induite par l'AAPH. Les valeurs représentées concernent les résultats obtenus en l'absence (contrôle) ou en présence du composé **37i** à différentes concentrations et sont exprimées par la moyenne ± SEM de triplicatas constituant une expérience représentative parmi les trois expériences indépendantes réalisées.
**Figure 25** : Courbe de décroissance de la fluorescence de la fluorescéine induite par l'AAPH. Les valeurs représentées concernent les résultats obtenus en l'absence (contrôle) ou en présence du composé **37j** à différentes concentrations et sont exprimées par la moyenne ± SEM de triplicatas constituant une expérience représentative parmi les trois expériences indépendantes réalisées.

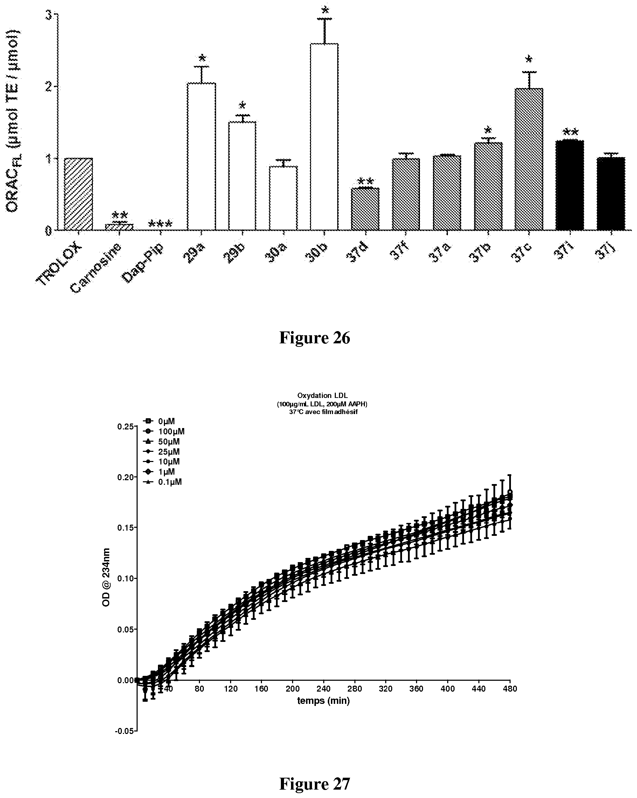

**Figure 26** : Capacité antioxydante (ORAC_{FL}) des composés selon l'invention à 10 µM. Les résultats représentés correspondent à la moyenne ± SEM de trois expériences indépendantes réalisées en triplicatas. * p < 0,05; ** p < 0,01; *** p < 0,001 *vs* Trolox (test *t* de Student : si p < 0,05, la différence est considérée comme significative).
**Figure 27****:** Évaluation des propriétés antiradicalaires du Dap-Pip, composé de 2^{ème} génération

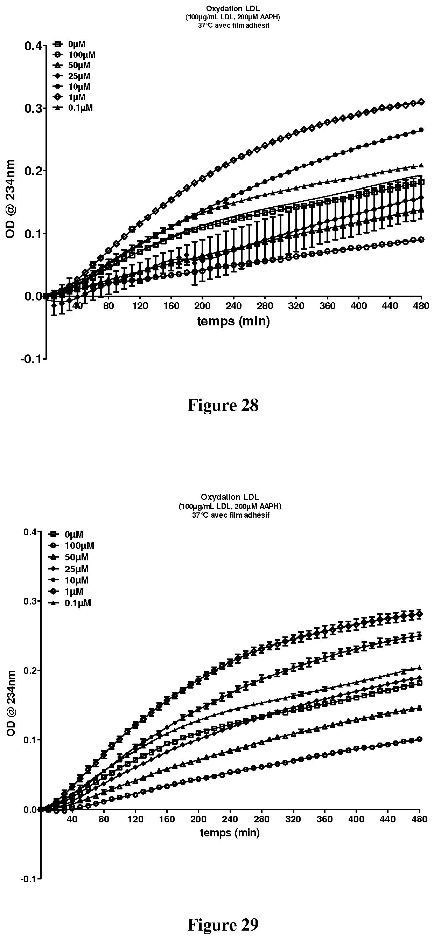

**Figure 28** : Évaluation des propriétés antiradicalaires du composé **29a** selon l'invention
**Figure 29** : Évaluation des propriétés antiradicalaires du composé **29b** selon l'invention

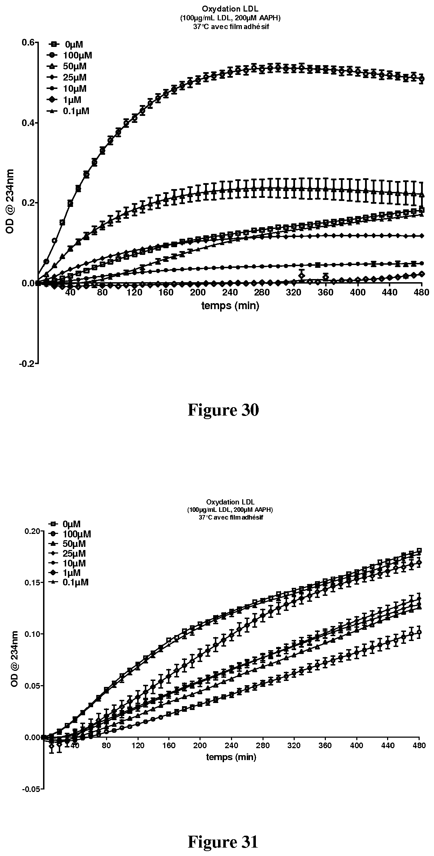

**Figure 30** **:** Évaluation des propriétés antiradicalaires du composé **30b** selon l'invention
**Figure 31** **:** Évaluation des propriétés antiradicalaires du composé **37a** selon l'invention

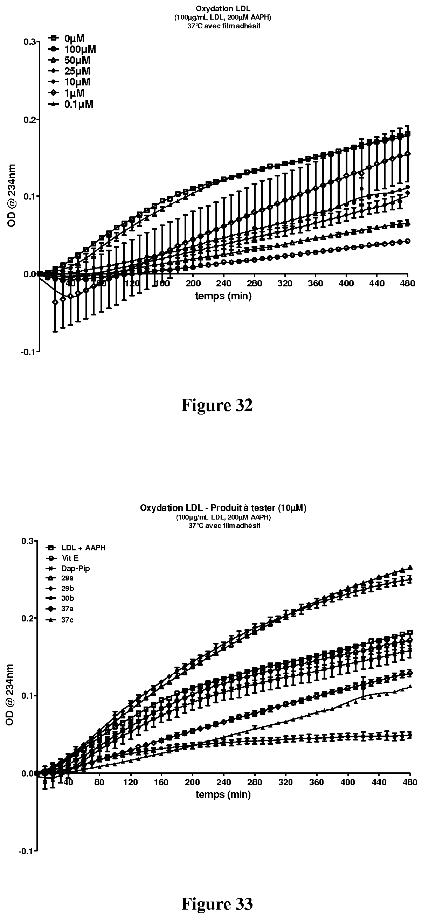

**Figure 32** **:** Évaluation des propriétés antiradicalaires du composé **37c** selon l'invention
**Figure 33** **:** Comparaison des propriétés antiradicalaires de la vitamine E, du Dap-Pip de 2^{ème} génération et des différents composés selon l'invention à une concentration de 10 µM

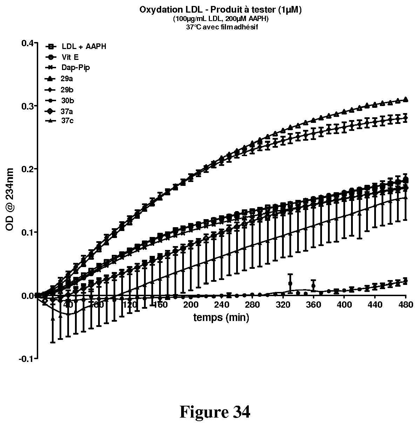

**Figure 34** **:** Comparaison des propriétés antiradicalaires de la vitamine E, du Dap-Pip de 2^{ème} génération et des différents composés selon l'invention à une concentration de 1 µM

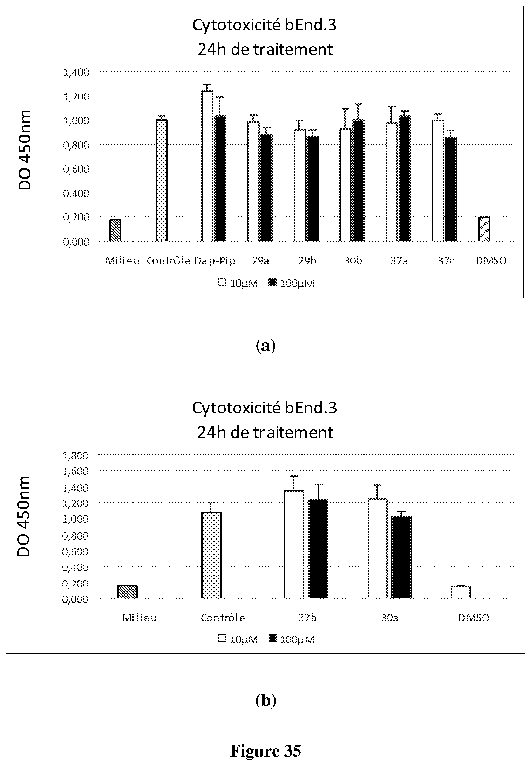

**Figure 35** **:** Étude de la cytotoxicité des composés selon l'invention sur des cellules endothéliales cérébrales murines (bEnd.3) après 24h de traitement. La viabilité des cellules est exprimée par la moyenne ± écart-type des triplicatas. (a) composés **29a, 29b, 30b, 37a** et **37c** ; (b) composés **30a** et **37b.**

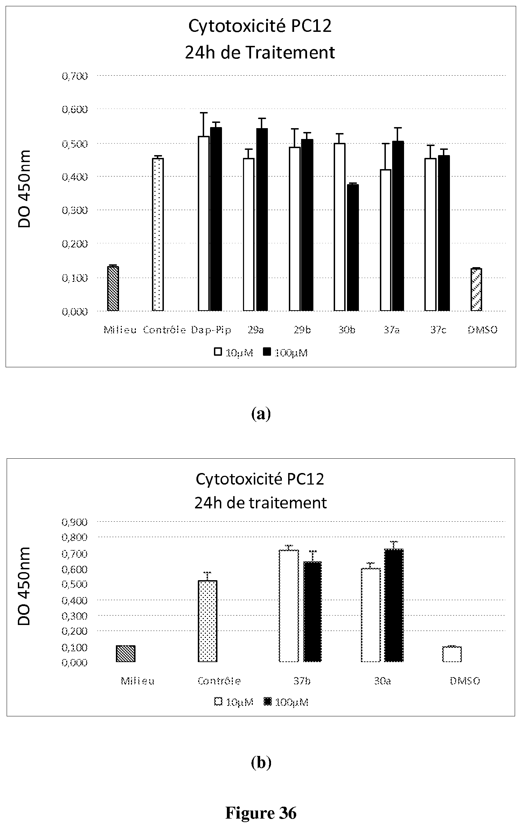

**Figure 36** **:** Étude de la cytotoxicité des composés selon l'invention sur des cellules de phéochromocytome de rat, assimilées à des cellules neuronales (PC12) après 24h de traitement. La viabilité des cellules est exprimée par la moyenne ± écart-type des triplicatas. (a) composés **29a, 29b, 30b, 37a** et **37c** ; (b) composés **30a** et **37b.**

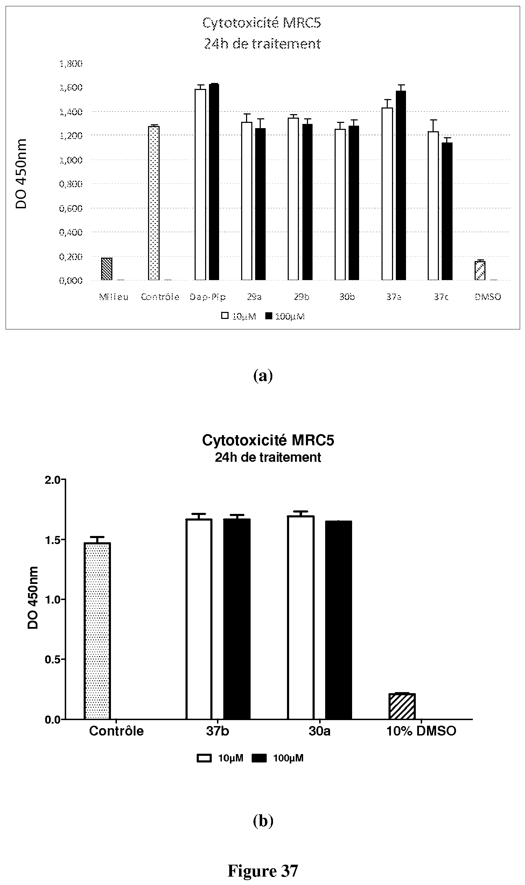

**Figure 37** **:** Étude de la cytotoxicité des composés selon l'invention sur des fibroblastes humains (MRC-5) après 24h de traitement. La viabilité des cellules est exprimée par la moyenne ± écart-type des triplicatas. (a) composés **29a, 29b, 30b, 37a** et **37c** ; (b) composés **30a** et **37b.**

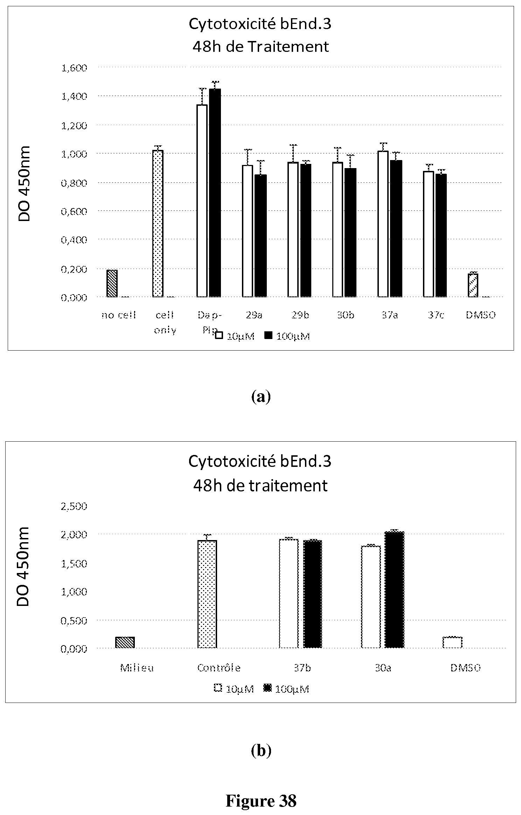

**Figure 38** **:** Étude de la cytotoxicité des composés selon l'invention sur des cellules endothéliales cérébrales murines (bEnd.3) après 48h de traitement. La viabilité des cellules est exprimée par la moyenne ± écart-type des triplicatas. (a) composés **29a, 29b, 30b, 37a** et **37c** ; (b) composés **30a** et **37b.**

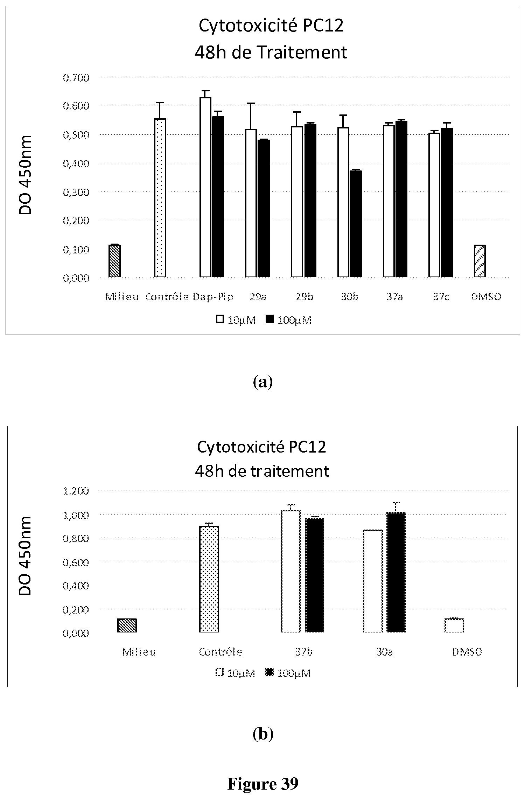

**Figure 39** **:** Étude de la cytotoxicité des composés selon l'invention sur des cellules de phéochromocytome de rat, assimilées à des cellules neuronales (PC12) après 48h de traitement. La viabilité des cellules est exprimée par la moyenne ± écart-type des triplicatas. (a) composés **29a, 29b, 30b, 37a** et **37c** ; (b) composés **30a** et **37b.**

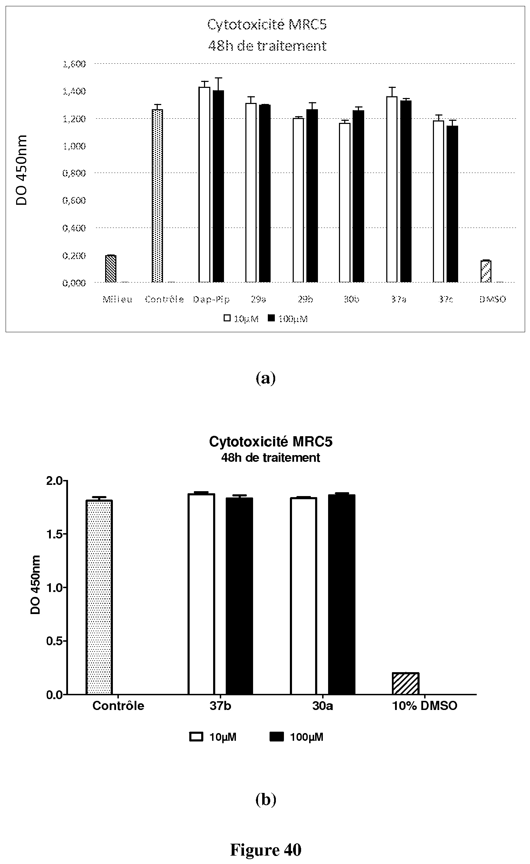

**Figure 40** **:** Étude de la cytotoxicité des composés selon l'invention sur des fibroblastes humains (MRC-5) après 48h de traitement. La viabilité des cellules est exprimée par la moyenne ± écart-type des triplicatas. (a) composés **29a, 29b, 30b, 37a** et **37c** ; (b) composés **30a** et **37b.**

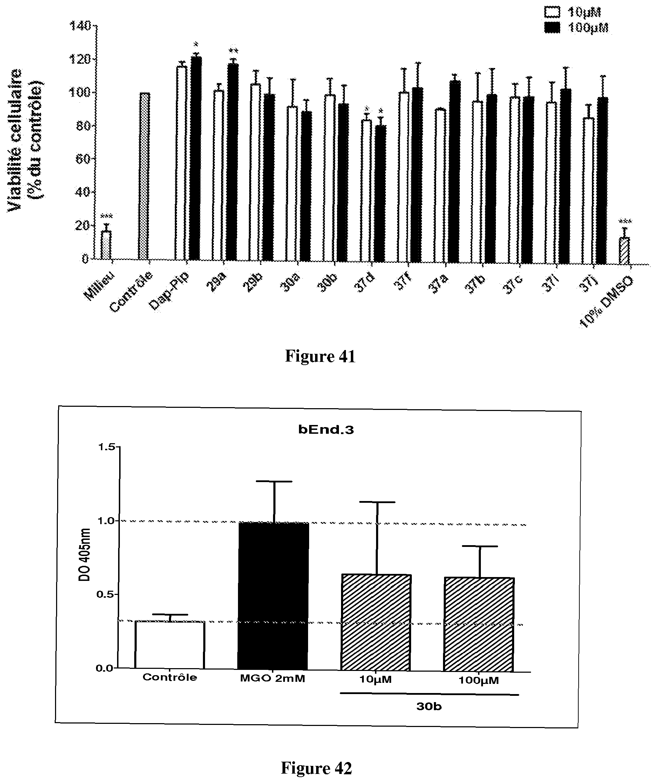

**Figure 41** : Étude de la cytotoxicité des composés selon l'invention **(29a, 29b, 30a, 30b, 37a, 37b, 37c, 37d, 37f, 37i** et **37j)** sur des cellules de phéochromocytome de rat, assimilées à des cellules neuronales (PC12) après 24h de traitement. La viabilité des cellules est exprimée par la moyenne ± SEM de trois expériences indépendantes réalisées en triplicatas.
**Figure 42** **:** Évaluation des propriétés antiapoptotiques du composé **30b** sur des cellules endothéliales cérébrales murines (bEnd.3). Les cellules bEnd.3 ont été prétraitées 30 min avec 10 µM ou 100 µM de **30b** avant l'ajout de 2 mM de MGO, puis incubées pendant 24h. L'apoptose des cellules est exprimée par la moyenne ± écart-type des triplicatas.

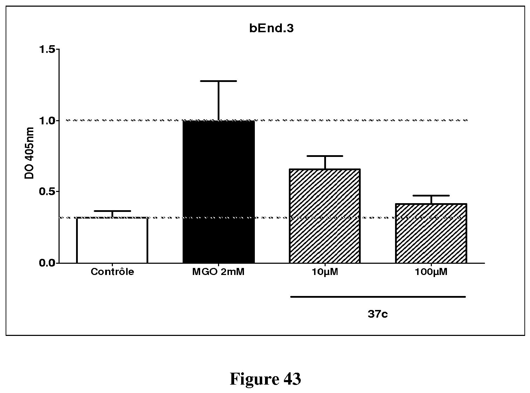

**Figure 43** **:** Évaluation des propriétés antiapoptotiques du composé **37c** sur des cellules endothéliales cérébrales murines (bEnd.3). Les cellules bEnd.3 ont été prétraitées 30 min avec 10 µM ou 100 µM de **37c** avant l'ajout de 2 mM de MGO, puis incubées pendant 24h. L'apoptose des cellules est exprimée par la moyenne ± écart-type des triplicatas.

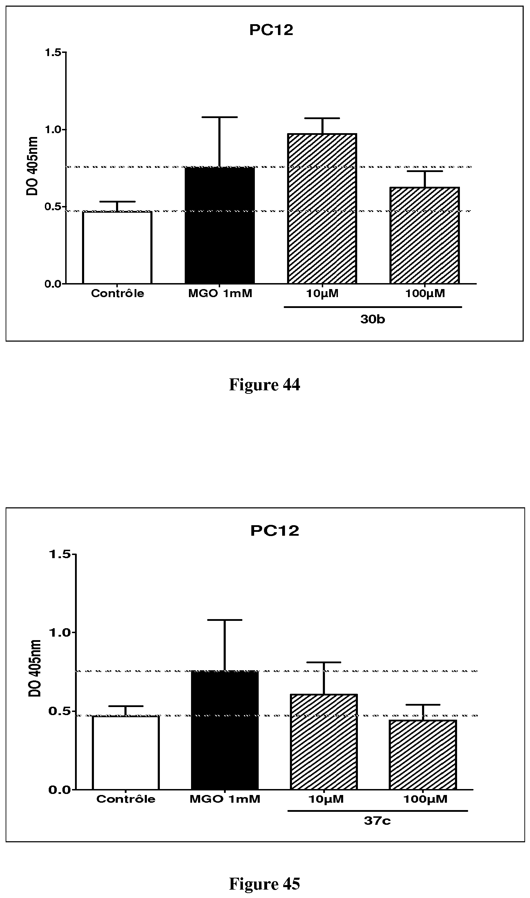

**Figure 44** **:** Évaluation des propriétés antiapoptotiques du composé **30b** sur des cellules de phéochromocytome de rat, assimilées à des cellules neuronales (PC12). Les cellules PC12 ont été prétraitées 30 min avec 10 µM ou 100 µM de **30b** avant l'ajout de 1 mM de MGO, puis incubées pendant 24h. L'apoptose des cellules est exprimée par la moyenne ± écart-type des triplicatas.
**Figure 45** **:** Évaluation des propriétés antiapoptotiques du composé **37c** sur des cellules de phéochromocytome de rat, assimilées à des cellules neuronales (PC12). Les cellules PC12 ont été prétraitées 30 min avec 10 µM ou 100 µM de **37c** avant l'ajout de 1 mM de MGO, puis incubées pendant 24h. L'apoptose des cellules est exprimée par la moyenne ± écart-type des triplicatas.

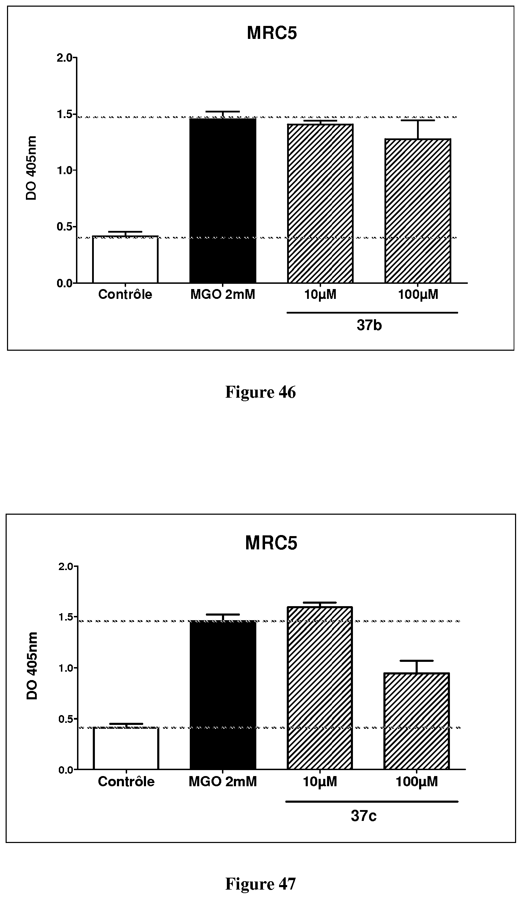

**Figure 46** **:** Évaluation des propriétés antiapoptotiques du composé **37b** sur des fibroblastes humains (MRC-5). Les cellules MRC5 ont été prétraitées pendant 1h avec 10 µM ou 100 µM de 37b avant l'ajout de 2 mM de MGO, puis incubées pendant 24h. L'apoptose des cellules est exprimée par la moyenne ± écart-type des triplicatas.
**Figure 47** **:** Évaluation des propriétés antiapoptotiques du composé **37c** sur des fibroblastes humains (MRC-5). Les cellules MRC5 ont été prétraitées pendant 1h avec 10 µM ou 100 µM de **37c** avant l'ajout de 2 mM de MGO, puis incubées pendant 24h. L'apoptose des cellules est exprimée par la moyenne ± écart-type des triplicatas.

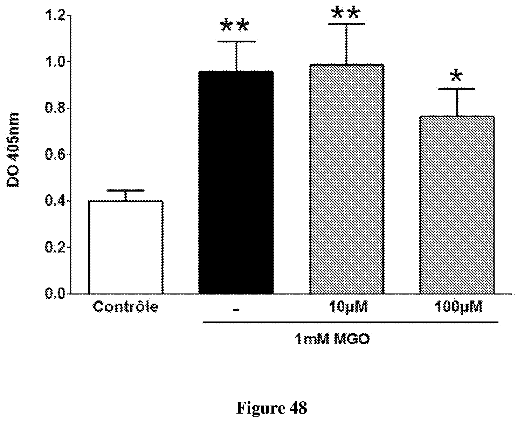

**Figure 48** **:** Évaluation des propriétés antiapoptotiques du composé **37c** sur des cellules de phéochromocytome de rat, assimilées à des cellules neuronales (PC12). Les cellules PC12 ont été prétraitées 1h avec 10 µM ou 100 µM de **37c** avant l'ajout de 1 mM de MGO, puis incubées pendant 24h. L'apoptose des cellules est exprimée par la moyenne ± SEM de trois expériences indépendantes réalisées en triplicatas. * p < 0.05; ** p < 0.01 *vs* contrôle (cellules non traitées par du MGO) (test t de Student : si p < 0,05, la différence est considérée comme significative).

### EXEMPLES

### A. SYNTHÈSE

### I) Matériel et méthodes

Les différents produits réactionnels ont été achetés chez Sigma-Aldrich (Lyon, France), et sont utilisés sans purifications additionnelles. Les CCM ont été réalisées sur plaques de silice Merck 60F254, observées dans l'ultraviolet (λ = 254 nm) avant d'être révélées à l'acide phosphomolybdique dans l'éthanol à 95 % suivi d'un chauffage jusqu'à coloration maximale. Les chromatographies préparatives sur colonne ont été réalisées par la technique chromatographique sur gel de silice Kielselgel 60 (40-63 µm) (Merck) ou à l'aide d'un système de chromatographie flash Reveleris® (Grace) en phase normale.

Les analyses RMN ont été réalisées sur un appareil Bruker AC300, 400 ou 600. Les déplacements chimiques sont exprimés en partie par million (ppm) par rapport au solvant deutéré utilisé comme référence interne. Les constantes de couplage (*J*) sont exprimées en Hertz (Hz) et la multiplicité des signaux est symbolisée comme suit : s (singulet), d (doublet), t (triplet), q (quadruplet) et m (multiplet). Les spectres de masse ont été obtenus sur un appareil Shimadzu LCMS-2020 pour les MS et un appareil Micromass Q-TOF Ultima pour les HRMS, en mode d'ionisation électrospray positif (ESI +).

### II) Synthèse des composés selon l'invention

### 1- Synthèse des synthons diaminés de départ

### a) Dérivés de l'acide aspartique et de l'acide glutamique (Schéma 1)

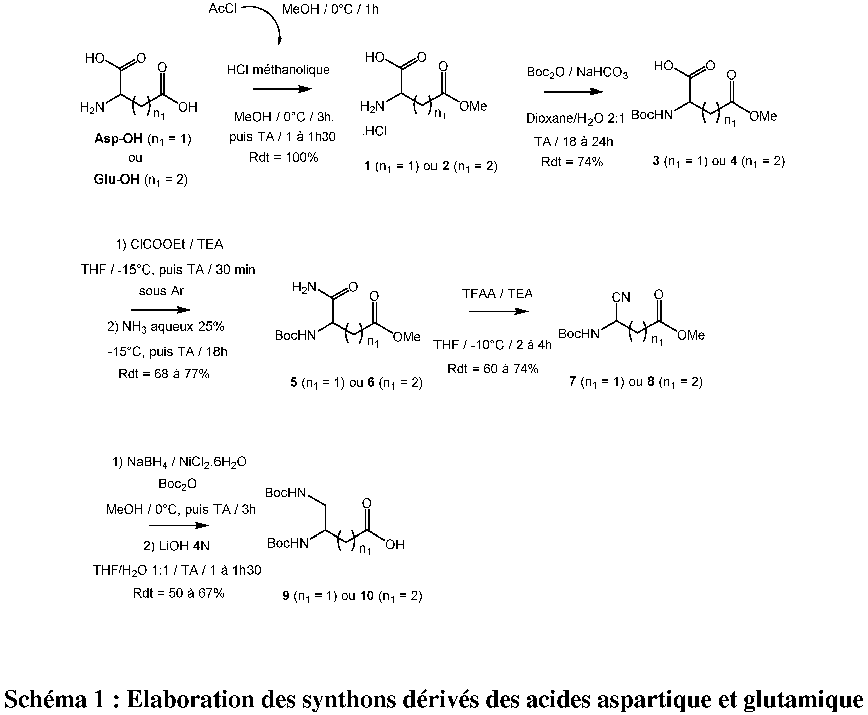

### * Composés 1 (Bioconjug. Chem., 2007, 18, 1625-1636) et 2 (J. Med. Chem., 2009 52, 4650-4656) :

La première étape de synthèse (

Schéma 1) permettant l'obtention d'un ester de méthyle au départ de l'acide aspartique fût précédemment mise au point par Wojciechowski et al. (Bioconjugate Chem., 2007, 18, 1625-1636) et a été dans le présent cas, transposée à l'acide glutamique.

### * Composés 3 (WO 2013/030193 A1) et 4 (J. Med. Chem., 2009 52, 4650-4656) :

Ollivier et al (Tetrahedron Lett., 2010, 51, 4147-4149) et More et al. (J. Med. Chem., 2009, 52, 4650-4656) ont respectivement décrit les conditions de protection utilisées du groupement aminé des esters de méthyle **1** et **2** par un groupement t-butyloxycarbonyle.

### * Composés 5 et 6 (J. Chem. Soc., Perkin Trans. 1, 1999, 2057-2060) :

À une solution du composé **3** ou **4** (38,3-40,4 mmol) dans du THF anhydre (150 mL), placée sous agitation à -15°C et sous Ar, sont additionnés successivement de la triéthylamine (1,1 eq) et du chloroformate d'éthyle (1,4 eq) dissout dans du THF anhydre (50 mL)) en goutte à goutte. Après agitation pendant 30 min à -15°C, une solution d'ammoniaque à 25% (2,5-2,7 eq dans un volume final de 16 mL) est introduite dans le milieu réactionnel qui est alors encore placé sous agitation à -15°C, puis à température ambiante pendant 18h. Le THF est ensuite évaporé sous pression réduite et le produit souhaité est extrait à l'aide d'acétate d'éthyle. La phase organique est lavée avec une solution de KHSO₄ 1N, puis de NaHCO₃ 10% et une solution saturée de NaCl, séchée sur Na₂SO₄ et finalement évaporée sous pression réduite pour donner respectivement le dérivé **5** ou **6** sous la forme d'une poudre blanche (68 ou 77%).
**Composé 5** : ¹H RMN (CDCl₃, 300 MHz): *δ* (ppm) 6,59 (s, 1H); 6,20 (s, 1H); 5,79-5,76 (s, 1H); 4,50-4,49 (m, 1H); 3,65 (s, 3H); 2,91 (dd, *J* = 16,9 Hz et *J'* = 4,8 Hz, 1H); 2,66 (dd, *J* = 18,7 Hz et *J*' = 5,7 Hz, 1H); 1,40 (s, 9H).
¹³C RMN (CDCl₃, 75 MHz): *δ* (ppm) 173,2; 171,9 ; 155,1; 80,1; 51,7; 49,9; 35,6; 27,9 (3C).
MS (ESI +): m/z = [M + H] 247,1; [M + Na] 269,1 ; [M + MeCN + Na] 310,1.
HRMS (ESI +): m/z calculée pour C₁₀H₁₈N₂O₅Na [M + Na] = 269,1113; trouvée = 269,1103.

### * Composés 7 et 8 :

À une solution du composé **5** ou **6** (26,4-28,8 mmol) dans du THF (100 mL), placée sous agitation à -10°C, sont additionnés de l'anhydride trifluoroacétique (1,5 eq) et de la triéthylamine (3 eq) (Synth. Commun., 2009, 39, 395-406). Le milieu réactionnel est alors placé sous agitation à cette même température pendant 2 à 4h. Le THF est ensuite évaporé sous pression réduite et le produit souhaité est extrait à l'aide d'acétate d'éthyle. La phase organique est lavée avec une solution de KHSO₄ 1N, puis de NaHCO₃ 10% et une solution saturée de NaCl, séchée sur Na₂SO₄ et finalement évaporée sous pression réduite pour donner respectivement le dérivé **7** ou **8** sous la forme d'un solide jaune (60 ou 74%) après purification sur colonne de silice à l'aide d'un mélange CH₂Cl₂/MeOH 98:2 ou par chromatographie flash en phase normale à l'aide d'un gradient CH₂Cl₂/MeOH 100:0 à 90:10.
**Composé 7:** ¹H NMR (CDCl₃, 300 MHz) *δ* (ppm): 5,61 (s, 1H); 4,90 (m, 1H); 3,75 (s, 3H); 2,86-2,82 (m, 2H); 1,39 (s, 9H).
¹³C RMN (CDCl₃, 75 MHz) *δ* (ppm): 159,0; 117,9; 80,3; 52,6; 37,4; 28,4 (3C).
MS (ESI +): m/z = [M + H] 229,2; [M + MeCN + Na] 292,1; [2M + Na + H] 480,2. HRMS (ESI +): m/z calculée pour C₁₀H₁₆N₂O₄Na [M + Na] = 251,1008 ; trouvée = 251,1000.
**Composé 8 :** ¹H RMN (CDCl₃, 600 MHz): *δ* (ppm) 5,41 (s, 1H); 4,62 (m, 1H); 3,67 (s, 3H); 2,53-2,46 (m, 2H); 2,13-2,10 (m, 2H); 1,41 (s, 9H).
¹³C RMN (CDCl₃, 150 MHz): *δ* (ppm) 172,6; 154,5; 118,8; 81,2; 52,0; 41,6; 29,6; 28,2 (3C); 28,2.
MS (ESI +): m/z = [M + H] 243,1; [M + Na] 265,1; [M + K] 281,0; [M + MeCN + Na] 306,1.
HRMS (ESI +): m/z calculée pour C₁₁H₁₈N₂O₄Na [M + Na] = 265,1164; trouvée = 265,1159.

### * Composés 9 et 10:

À une solution du composé **7** ou **8** (15,3-19,8 mmol) dans du méthanol (150 mL), placée sous agitation à 0°C, sont additionnés du dicarbonate de di-*t*-butyle (2 eq) et du NiCl₂.6H₂O (0,1 eq). Du borohydrure de sodium (8 eq) est ensuite introduit par petites portions sur une période de 1h et le mélange est placé sous agitation à température ambiante pendant 3h (Tetrahedron, 2003, 59, 5417-5423). Après addition de diéthylènetriamine (2 eq) et agitation à nouveau pendant 30 min à 1h, le méthanol est évaporé sous pression réduite et le produit souhaité est extrait à l'aide d'acétate d'éthyle. La phase organique est lavée avec une solution saturée de NaHCO₃, puis de NaCl, séchée sur Na₂SO₄ et finalement évaporée sous pression réduite. Le composé intermédiaire obtenu est alors dissout dans un mélange THF/H₂0 1:1 (40 mL) et une solution aqueuse de LiOH 4N (4 eq) est ensuite introduite dans le milieu qui est maintenu sous agitation pendant 1h à 1h30. Le THF est évaporé sous pression réduite, puis le mélange est repris dans de l'éther diéthylique ou de l'acétate d'éthyle et alcalinisé avec une solution de Na₂CO₃ 10% afin d'éliminer l'ester de méthyle et le dicarbonate de di-*t*-butyle restant dans la phase organique. La phase aqueuse est ensuite acidifiée à l'aide d'une solution de HCl 6N et le produit souhaité est alors extrait à l'aide d'éther diéthylique ou d'acétate d'éthyle. Après lavage avec une solution saturée de NaCl, la phase organique est finalement séchée sur Na₂SO₄ et évaporée sous pression réduite pour donner respectivement le dérivé **9** ou **10** sous la forme d'une poudre blanche (50 ou 67%).
**Composé 9 :** ¹H RMN (CDCl₃, 300 MHz): *δ* (ppm) 8,70-8,61 (s, 1H); 5,54-5,41 (s, 1H); 5,15 (s, 1H); 4.00-3.98 (m, 1H); 3,37-3,25 (m, 2H); 2,65-2,59 (m, 2H); 1,43 (s, 18H). ¹³C RMN (CDCl₃, 75 MHz): *δ* (ppm) 174,7; 174,6; 146,2 (2C); 79,9; 79,8; 48,0; 43,4; 36,4; 28,1 (6C).
MS (ESI +): m/z = [M + H] 319,2; [M + Na] 341,2; [M + MeCN + Na] 382,2.
HRMS (ESI +): m/z calculée pour C₁₄H₂₆N₂O₆Na [M + Na] = 341,1689; trouvée = 341,1676.
**Composé 10 :** ¹H RMN (CDCl₃, 300 MHz): *δ* (ppm) 8,35-8,28 (s, 1H); 5,06 (s, 1H); 4,99-4,96 (s, 1H); 3,72-3,66 (m, 1H); 3,18-3,16 (m, 2H); 2,43-2,37 (m, 2H); 1,87-1,76 (m, 2H); 1,43 (s, 18H).
¹³C RMN (CDCl₃, 75 MHz): *δ* (ppm) 177,4; 156,8; 156,5; 79,6 (2C); 51,0; 44,6; 30,5; 28,3 (6C), 27,7.
MS (ESI +): m/z = [M + Na] 355,9; [M + MeCN + Na] 396,2.
HRMS (ESI +): m/z calculée pour C₁₅H₂₈N₂O₆Na [M + Na] = 355,1845; trouvée = 355,1855.

### b) Dérivés de l'ornithine et de la lysine (Schéma 2)

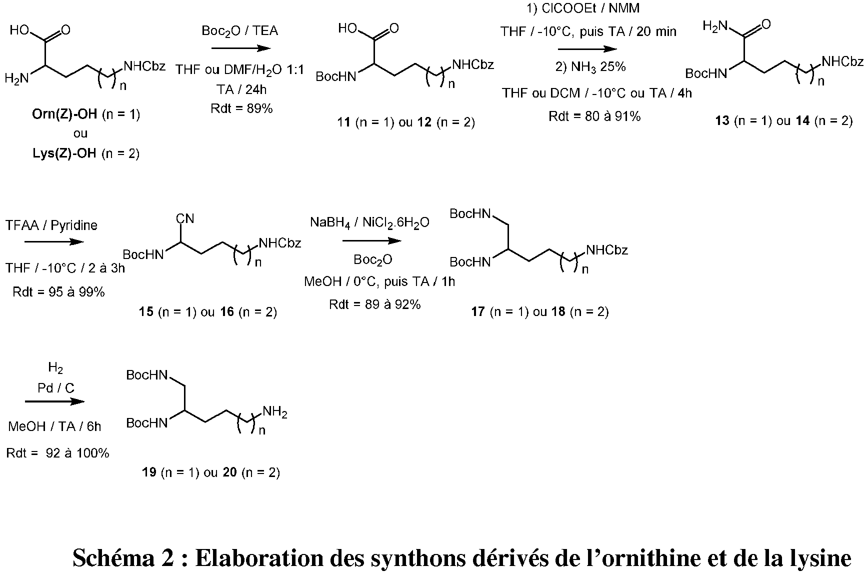

### * Composé 11 et 12 (commerciaux)

### * Composés 13 (WO 07/011623 A1) et 14 (WO 00/64865 A1) :

À une solution du composé **11** ou **12** (1 eq) (13,7-24,5 mmol) dans du THF (50-150 mL), placée sous agitation à -10°C, sont additionnés successivement de la *N-*méthylmorpholine (1,1 eq) et du chloroformate d'éthyle (1,1 eq). Après agitation pendant 20 min à -10°C, une solution d'ammoniaque à 25% (2,5 eq dans un volume final de 16 mL) est introduite dans le milieu réactionnel qui est alors placé sous agitation pendant 4h. Le THF est ensuite évaporé sous pression réduite et le produit souhaité est extrait à l'aide d'acétate d'éthyle. La phase organique est lavée avec une solution de KHSO₄ 1N, puis de NaHCO₃ 10% et une solution saturée de NaCl, séchée sur Na₂SO₄ et finalement évaporée sous pression réduite pour donner respectivement le dérivé **13** ou **14** sous la forme d'une poudre blanche (91 ou 80%) après recristallisation à l'aide d'un mélange AcOEt/Cyclohexane 20:80.

### * Composés 15 et 16 (WO 2000/64865 A1) :

À une solution du composé **13** ou **14** (8,1-10 mmol) dans du THF (60-70 mL), placée sous agitation à -10°C, sont additionnés de l'anhydride trifluoroacétique (1,5 eq) et de la pyridine (3 eq). Le milieu réactionnel est alors placé sous agitation à cette même température pendant 2h. Le THF est ensuite évaporé sous pression réduite et le produit souhaité est extrait à l'aide d'acétate d'éthyle. La phase organique est lavée avec une solution de KHSO₄ 1N, puis de NaHCO₃ 10% et une solution saturée de NaCl, séchée sur Na₂SO₄ et finalement évaporée sous pression réduite pour donner respectivement le dérivé **15** ou **16** sous la forme d'une poudre blanche (99 ou 95%) après recristallisation à l'aide d'un mélange AcOEt/Cyclohexane 20:80.
**Composé 15 :** ¹H RMN (CDCl₃, 600 MHz): *δ* (ppm) 7,36-7,29 (m, 5H); 5,11 (s, 1H); 5,08 (s, 2H); 4,96 (s, 1H); 4,55 (m, 1H); 3,24-3,23 (m, 2H); 1,81-1,79 (m, 2H); 1,68-1,65 (m, 2H); 1,44 (s, 9H).
¹³C RMN (CDCl₃, 150 MHz): *δ* (ppm) 156,6 (2C); 136,4; 128,5 (2C); 128,2; 128,1 (2C); 118,7; 79,1; 66,8; 42,0; 40,0; 29,6; 28,2 (3C); 26,1.
MS (ESI +): m/z = [M + Na] 370,2; [2M + Na] 717,3.
HRMS (ESI +): m/z calculée pour C₁₈H₂₅N₃O₄Na [M + Na] = 370,1743; trouvée = 370,1749.

### * Composés 17 et 18 :

A une solution du composé **15** ou **16** (8-10 mmol) dans du méthanol (60-80 mL), placée sous agitation à 0°C, sont additionnés du dicarbonate de di-*t*-butyle (2 eq) et du NiCl₂.6H₂O (0,1 eq). Du borohydrure de sodium (7 eq) est ensuite introduit par petites portions sur une période de 30 min à 1h et le mélange est placé sous agitation à température ambiante pendant 1h. Après addition de diéthylènetriamine (1 eq) et agitation à nouveau pendant 30 min à 1h, le méthanol est évaporé sous pression réduite et le produit souhaité est extrait à l'aide d'acétate d'éthyle. La phase organique est lavée avec une solution saturée de NaHCO₃, puis de NaCl, séchée sur Na₂SO₄ et finalement évaporée sous pression réduite pour donner respectivement le dérivé **17** ou **18** sous la forme d'une poudre blanche (92 ou 89%).
**Composé 17 :** ¹H RMN (CDCl₃, 300 MHz): *δ* (ppm) 7,34-7,31 (m, 5H); 5,06 (s, 2H); 5,06 (s, 1H); 4,94 (s, 1H); 4,80-4,77 (s, 1H); 3,58 (m, 1H); 3,21-3,12 (m, 4H); 1,56-1,52 (m, 2H); 1,45-1,44 (m, 2H); 1,43 (s, 18H).
¹³C RMN (CDCl₃, 75 MHz): *δ* (ppm) 172,0; 156,9 (2C); 136,9; 128,8 (2C); 127,4 (3C); 79,7 (2C); 67,0; 51,5; 44,8; 41,1; 30,4; 28,7 (6C); 26,5.
MS (ESI +): m/z = [M + H] 452,1; [M + Na] 474,0.
HRMS (ESI +): m/z calculée pour C₂₃H₃₇N₃O₆Na [M + Na] = 474,2580; trouvée = 474,2560.
**Composé 18 :** ¹H RMN (CDCl₃, 300 MHz): *δ* (ppm) 7,34-7,29 (m, 5H); 5,07 (s, 2H); 4,98-4,91 (s, 2H); 4,72 (s, 1H); 3,56 (m, 1H); 3,17-3,13 (m, 4H); 1,46-1,41 (m, 6H); 1,41 (m, 18H).
¹³C RMN (CDCl₃, 75 MHz): *δ* (ppm) 176,3; 155,8 (2C); 136,0; 127,8 (2C); 127,4 (3C); 78,7 (2C); 65,9; 50,5; 43,9; 39,8; 31,6; 29,1; 27,7 (6C); 22,1.
MS (ESI +): m/z = [M + H] 466,3; [M + Na] 488,3.
HRMS (ESI +): m/z calculée pour C₂₄H₃₉N₃O₆Na [M + Na] = 488,2737; trouvée = 488,2730.

### * Composés 19 et 20 :

À une solution du composé **17** ou **18** (1,1-5,7 mmol) dans du méthanol (10-40 mL), est additionné du Pd/C (10% m/m). Le milieu réactionnel est placé sous vide et sous agitation à température ambiante pendant 30 min, puis maintenu sous un flux d'H₂ pendant 6h. Il est ensuite filtré sur papier et le méthanol est évaporé sous pression réduite pour donner respectivement le dérivé **19** ou **20** sous la forme d'une poudre blanche (92 ou 100%).
**Composé 19 :** ¹H RMN (CDCl₃, 600 MHz): *δ* (ppm) 4,98 (s, 1H); 4,96 (s, 1H); 3,56 (m, 1H); 3,18-3,12 (m, 2H); 2,74 (s, 2H); 2,74 (m, 2H); 1,48-1,43 (m, 4H); 1,41 (s, 18H).
¹³C RMN (CDCl₃, 150 MHz): *δ* (ppm) 156,7; 156,3; 79,3 (2C); 51,2; 44,7; 41,6; 30,1; 29,3; 28,4 (6C).
MS (ESI +): m/z = [M + H] 318,2; [M + Na] 340,2.
HRMS (ESI +): m/z calculée pour C₁₅H₃₁N₃O₄ [M + H] = 318,2393; trouvée = 318,2379.
**Composé 20 :** ¹H RMN (CDCl₃, 300 MHz): *δ* (ppm) 4,99 (s, 2H); 4,76-4,73 (s, 2H); 3,55 (m, 1H); 3,10 (m, 2H); 2,61-2,59 (m, 2H); 1,57 (m, 2H); 1,37 (m, 22H).
¹³C RMN (CDCl₃, 75 MHz): *δ* (ppm) 170,8; 167,9; 78,9 (2C); 51,0; 44,4; 41,5; 33,1; 32,4; 28,0 (6C); 22,7.
MS (ESI +): m/z = [M + H] 332,2; [M + Na] 354,2.
HRMS (ESI +): m/z calculée pour C₁₆H₃₃N₃O₄ [M + H] = 332,2549; trouvée = 332,2564.

### 2- Couplage du groupement Rₐ ou NR'ₐR_{b}

### a) Couplage pseudo-peptidique

### α- Méthode A (Schéma 3)

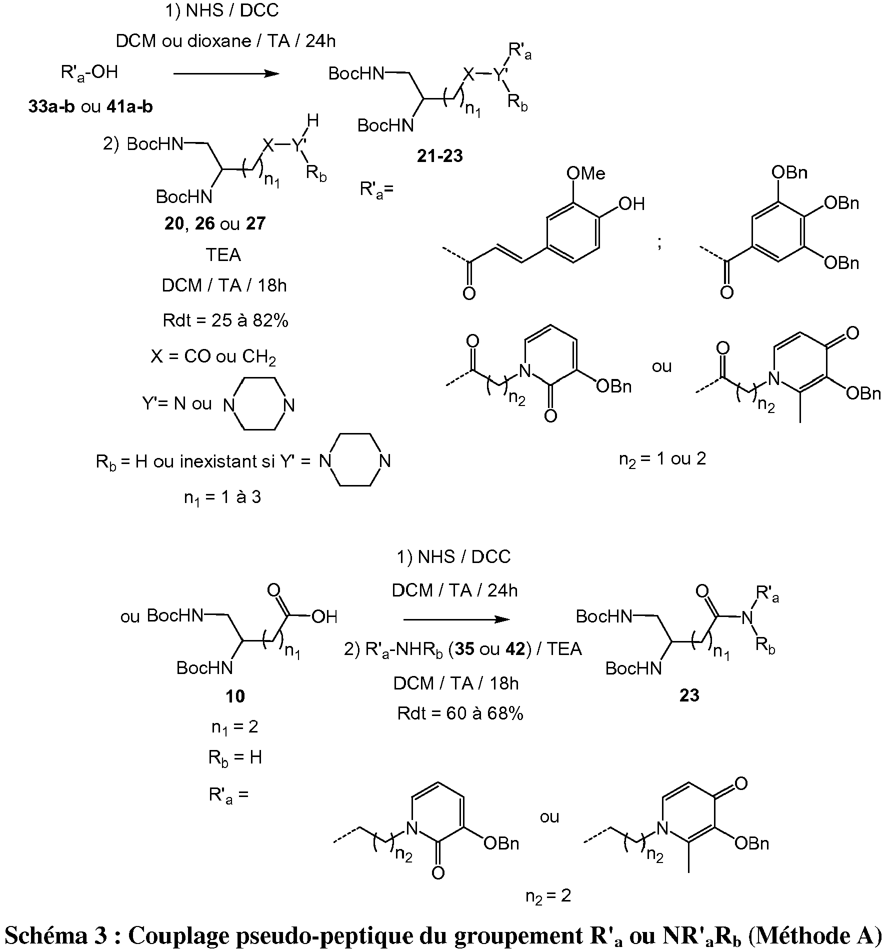

* 1^{ère} étape : A une solution du dérivé **10, 33a-b, 41a-b,** porteur de la fonction acide (0,4-3,0 mmol) dans du dichlorométhane ou du 1,4-dioxane (10-25 mL), sont additionnés successivement de la N-hydroxysuccinimide (1,1 à 1,5 eq) et du DCC (1 à 1,2 eq). Le milieu réactionnel est maintenu sous agitation à température ambiante pendant 24h, puis filtré sous vide. Le filtrat est ensuite évaporé sous pression réduite pour donner l'intermédiaire activé de l'acide.

* 2^{ème} étape : Le dérivé aminé **20, 26, 27, 35** ou **42** (1 à 1,7 eq) est dissous dans du dichlorométhane (8-30 mL) et mis à réagir avec de la triéthylamine (0 à 4 eq) à température ambiante pendant 30 min. L'intermédiaire activé de l'acide (1 eq) est ensuite introduit dans le milieu réactionnel qui est alors est placé sous agitation à température ambiante pendant 18h. La phase organique est ensuite lavée avec une solution de HCl 1N, puis de NaHCO₃ et de NaCl saturée, séchée sur Na₂SO₄ et finalement évaporée sous pression réduite pour donner le composé **21a-b, 22a-b, 23c-f** ou **23j** (25 à 82%) après purification sur colonne de silice (CH₂Cl₂/MeOH 98:2 à 95:5) (Tableau 2).

### β- Méthode B (Schéma 4)

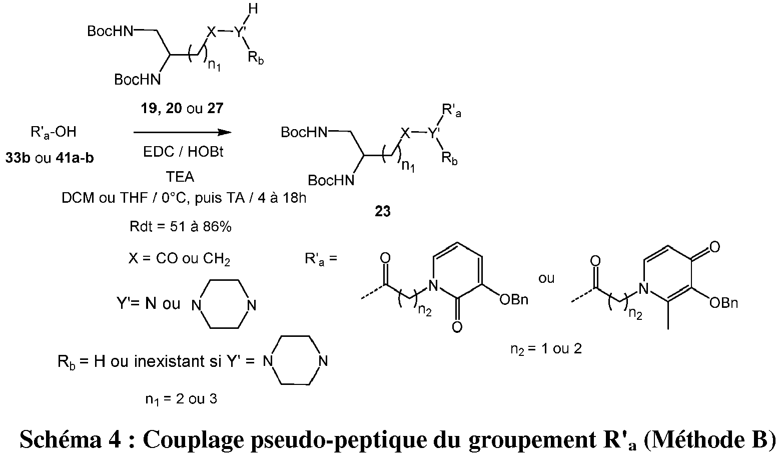

À une solution du dérivé porteur de la fonction acide **33b, 41a-b** (1-6,5 mmol) dans du dichlorométhane, DMF ou THF (25-100 mL), placée sous agitation à 0°C, sont additionnés successivement du chlorhydrate de 1-éthyl-3-(3-diméthylaminopropyl)-carbodiimide (1,2 eq), du 1-hydroxybenzotriazole monohydrate (1,1-1,2 eq). Après 30 min d'agitation à 0°C, le dérivé aminé **19, 20** ou **27** (1 eq) est introduit avec de la triéthylamine (1,2 eq) dans le milieu réactionnel qui est alors maintenu sous agitation à température ambiante pendant 4 à 18h. Après évaporation du DMF ou du THF et reprise du résidu dans du dichlorométhane, la phase organique est lavée avec une solution saturée de NaCl, séchée sur Na₂SO₄ et finalement évaporée sous pression réduite pour donner après purification sur colonne de silice (AcOEt/Cyclohexane 60:40 ou AcOEt/MeOH 80:20) les composés **23a-b, 23g-i** ou **23k** (51 à 86%) (Tableau 2).

**Tableau 2 : Composés 21-23**

| **Composé** | **n₁** | **X** | **Y'** | **R'ₐ** | **R_{b}** | **Rdt (%) (Méthode)** | **Aspect** |
|---|---|---|---|---|---|---|---|
| **21a** | 2 | CO | 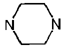 | 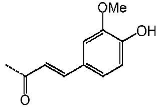 | Inexistant | 55 (A) | Poudre blanche |
| **21b** | 3 | CH₂ | N | 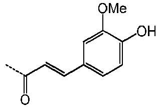 | H | 71 (A) | Poudre blanche |
| **22a** | 2 | CO | 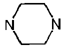 | 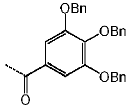 | Inexistant | 81 (A) | Huile incolore |
| **22b** | 3 | CH₂ | N | 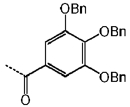 | H | 58 (A) | Poudre jaune |
| **23a** | 2 | CH₂ | N | 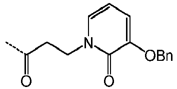 | H | 92 (B) | Poudre brune |
| **23b** | 3 | CH₂ | N | 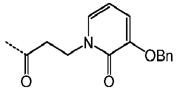 | H | 86 (B) | Poudre brune |
| **23c** | 2 | CO | N | 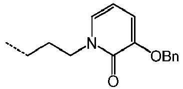 | H | 68 (A) | Poudre blanche |
| **23d** | 1 | CO | 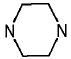 | 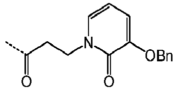 | Inexistant | 39 (A) | Poudre brune |
| **23e** | 2 | CO | 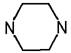 | 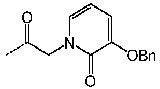 | Inexistant | 56 (A) | Poudre blanche |
| **23f** | 2 | CO | 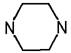 | 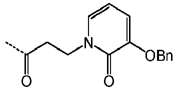 | Inexistant | 25 (A) | Poudre blanche |
| **23g** | 2 | CH₂ | N | 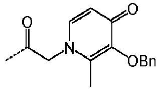 | H | 65 (B) | Poudre blanche |
| **23h** | 3 | CH₂ | N | 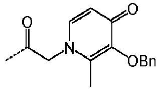 | H | 62 (B) | Poudre blanche |
| **23i** | 3 | CH₂ | N | 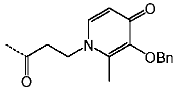 | H | 69 (B) | Solide pâteux blanc |
| **23j** | 2 | CO | N | 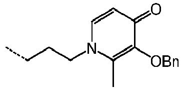 | H | 60 (A) | Poudre jaune |
| **23k** | 2 | CO | 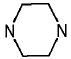 | 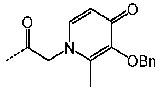 | Inexistant | 51 (B) | Poudre blanche |

**Composé 21a :** ¹H RMN (CDCl₃, 300 MHz): *δ* (ppm) 7,63 (d, *J* = 15,0 Hz, 1H); 7,09 (dd, *J₁* = 8,4 Hz, *J*₂ = 1,8 Hz, 1H); 6,99 (d, *J* = 1,8 Hz, 1H); 6,91 (d, *J* = 8,1 Hz, 1H); 6,67 (d, *J* = 15,3 Hz, 1H); 6,08 (s, 1H); 4,93 (s, 2H); 3,92 (s, 3H); 3,73-3,50 (m, 9H); 3,20 (t, *J* = 5,4 Hz, 2H); 2,45-2,39 (m, 2H); 1,86-1,80 (m, 2H); 1,42 (s, 18H).
¹³C RMN (CDCl₃, 75 MHz): *δ* (ppm) 170,8; 170,5 ; 165,5 (2C); 147,2; 146,4; 143,4; 127,1; 121,7; 114,4; 113,3; 109,5; 77,5 (2C); 55,6; 51,0; 44,8; 44,2 (2C); 41,3 (2C); 29,1; 27,9 (6C); 27,5.
MS (ESI +): m/z = [M + H] 577,3; [M + Na] 599,3.
HRMS (ESI +): m/z calculée pour C₂₉H₄₄N₄O₈ [M + Na] = 599,3057; trouvée = 599,3043.
**Composé 21b** : ¹H RMN (CDCl₃, 300 MHz): *δ* (ppm) 7,52 (d, *J* = 15,6 Hz, 1H); 7,02 (dd, *J₁* = 8,1 Hz, *J*₂ = 1,8 Hz, 1H); 6,96 (d, *J* = 1,8 Hz, 1H); 6,87 (d, *J* = 8,1 Hz, 1H); 6,31 (d, *J* = 15,6 Hz, 1H); 6,28 (s, 1H); 5,01-4,99 (s, 1H); 4,84-4,82 (s, 1H); 3,86 (s, 3H); 3,59-3,58 (m, 1H); 3,34-3,33 (m, 2H); 3,16 (m, 2H); 1,57-1,55 (m, 2H); 1,42 (m, 22H).
¹³C RMN (CDCl₃, 75 MHz): *δ* (ppm) 167,4 (2C); 157,3; 148,3; 147,8; 141,6; 128,4; 122,9; 119,5; 115,8; 110,8; 80,4 (2C); 56,8; 52,2; 45,3; 39,9; 33,1; 30,6; 29,3 (6C); 23,7.
MS (ESI +): m/z = [M + H] 508,2; [M + Na] 530,2.
HRMS (ESI +): m/z calculée pour C₂₆H₄₁N₃O₇Na [M + Na] = 530,2842; trouvée = 530,2856.
**Composé 22a :** ¹H RMN (CDCl₃, 300 MHz): *δ* (ppm) 7,40-7,33 (m, 12H); 7,28-7,26 (m, 3H); 6,65 (s, 2H); 5,12 (s, 4H); 5,10 (s, 2H); 3,65-3,19 (m, 11H); 2,42-2,39 (m, 2H); 1,90-1,84 (m, 2H); 1,70-1,64 (m, 2H); 1,43 (s, 18H).
¹³C RMN (CDCl₃, 75 MHz): *δ* (ppm) 171,0; 169,9; 156,5; 156,2; 152,5 (2C); 139,7; 137,3; 136,5 (2C); 129,9; 128,4 (6C); 128,0; 127,8 (2C); 127,1 (6C); 107,1 (2C); 79,2 (2C); 75,0; 71,0 (2C); 51,2; 45,0 (2C); 44,4; 41,5 (2C); 33,7; 29,3; 28,2 (6C).
MS (ESI +): m/z = [M + H] 823,3; [M + Na] 845,3.
HRMS (ESI +): m/z calculée pour C₄₇H₅₈N₄O₉Na [M + Na] = 845,4101; trouvée = 845,4138.
**Composé 22b** : ¹H RMN (CDCl₃, 300 MHz): *δ* (ppm) 7,38-7,28 (m, 12H); 7,28-7,26 (m, 3H); 7,17 (s, 2H); 6,62 (s, 1H); 5,13 (s, 4H); 5,09 (s, 2H); 4,97 (s, 1H); 4,78-4,75 (s, 1H); 3,63-3,62 (m, 1H); 3,40 (m, 2H); 3,20-3,18 (m, 2H); 1,62 (m, 2H); 1,46-1,44 (m, 22H). ¹³C RMN (CDCl₃, 75 MHz): *δ* (ppm) 167,2; 156,2 (2C); 152,8 (2C); 141,1; 137,6; 136,9 (2C); 130,3; 128,6 (6C); 128,3; 128,1 (2C); 127,7 (6C); 107,0 (2C); 79,6 (2C); 75,3 (2C); 71,5; 51,4; 44,2; 39,5; 32,1; 29,2; 28,5 (6C); 22,8.
MS (ESI +): m/z = [M - 3Bn + 3H + Na] 506,3; [M + H + Na] 777,2.
HRMS (ESI +): m/z calculée pour C₄₄H₅₅N₃O₈Na [M + Na] = 776,3887; trouvée = 776,3905.
**Composé 23a :** ¹H RMN (CDCl₃, 300 MHz): *δ* (ppm) 7,38-7,28 (m, 5H); 7,22-7,19 (s, 1H); 7,08 (dd, *J₁* = 6,9 Hz, *J*₂ = 1,5 Hz, 1H); 6,70 (dd, *J₁* = 7,5 Hz, *J*₂ = 1,2 Hz, 1H); 6,04 (t, *J* = 7,2 Hz, 1H); 5,07-5,01 (s, 2H); 4,93-4,90 (s, 1H); 4,28-4,18 (m, 2H); 3,51-3,47 (m, 1H); 3,05-3,03 (m, 4H); 2,63 (t, *J* = 6,0 Hz, 2H); 2,27-1,40 (s, 9H); 1,38 (s, 9H); 1,32-1,17 (m, 4H).
¹³C RMN (CDCl₃, 75 MHz): *δ* (ppm) 169,7 (2C); 157,7; 156,1; 148,1; 135,4; 130,0; 128,1 (2C); 127,7; 127,2 (2C); 115,1; 104,4; 78,7 (2C); 70,3; 50,5; 46,5; 44,6; 38,6; 34,5; 29,4; 27,9 (6C); 25,0.
MS (ESI +): m/z = [M + H] 573,2; [M + Na] 595,1.
HRMS (ESI +): m/z calculée pour C₃₀H₄₄N₄O₇Na [M + Na] = 595,3108; trouvée = 595,3098.
**Composé 23b :** ¹H RMN (CDCl₃, 300 MHz): *δ* (ppm) 7,36-7,25 (m, 5H); 7,06 (dd, *J₁* = 6,9 Hz, *J*₂ = 1,5 Hz, 1H); 6,99 (s, 1H); 6,66 (dd, *J₁* = 7,5 Hz, *J*₂ = 1,5 Hz, 1H); 6,00 (t, *J* = 7,2 Hz, 1H); 5,00 (s, 1H); 5,00 (s, 2H); 4,83-4,80 (s, 1H); 4,21 (t, *J* = 6,3 Hz, 2H); 3,50-3,48 (m, 1H); 3,07-3,00 (m, 4H); 2,65 (t, *J* = 6,0 Hz, 2H); 1,38 (s, 18H); 1,33-1,23 (m, 6H).
¹³C RMN (CDCl₃, 75 MHz): *δ* (ppm) 170,1 (2C); 158,1 (2C); 148,5; 135,9; 130,3; 128,5 (2C); 128,1; 127,4 (2C); 115,6; 104,7; 79,2 (2C); 70,6; 51,1; 47,0; 44,6; 38,8; 35,0; 32,0; 28,9; 28,3 (6C); 22,7.
MS (ESI +): m/z = [M + H] 587,5; [M + Na] 609,3.
HRMS (ESI +): m/z calculée pour C₃₁H₄₆N₄O₇Na [M + Na] = 609,3264; trouvée = 609,3254.
**Composé 23c :** ¹H RMN (CDCl₃, 300 MHz) : *δ* (ppm) 7,40 (s, 1H); 7,38-7,25 (m, 5H); 6,95 (d, *J* = 6,0 Hz, 1H); 6,67 (d, *J* = 6,8 Hz, 1H); 6,10 (t, *J* = 7,2 Hz, 1H); 5,40-5,23 (s, 2H); 5,03 (s, 2H); 4,00 (t, *J* = 5,1 Hz, 2H); 3,61-3,59 (m, 1H); 3,11 (m, 4H); 2,33-2,26 (m, 2H); 1,87-1,61 (m, 4H); 1,35 (s, 18H).
¹³C RMN (CDCl₃, 75 MHz): *δ* (ppm) 172,3 (2C); 156,4; 156,2; 148,2; 135.6; 128,5; 128,2 (2C); 128,0; 127,8 (2C); 115,5; 105,8; 79,0 (2C); 70,4; 50,9; 46,8; 44,2; 35,7; 32,6; 30,3; 28,8; 28,0 (6C).
MS (ESI +): m/z = [M + H] 573,5; [M + Na] 595,4.
HRMS (ESI +): m/z calculée pour C₃₀H₄₄N₄O₇Na [M + Na] = 595,3108; trouvée = 595,3078.
**Composé 23d** : ¹H RMN (CDCl₃, 300 MHz): *δ* (ppm) 7,32-7,18 (m, 5H); 7,01 (d, *J* = 6,1 Hz, 1H); 6,56 (d, *J* = 5,4 Hz, 1H); 5,92 (t, *J* = 6,9 Hz, 1H); 5,62-5,60 (s, 1H); 5,03-4,97 (s, 1H); 4,97 (s, 2H); 4,14 (t, *J* = 6,3 Hz, 2H); 3,81-3,79 (m, 1H); 3,54-3,32 (m, 8H); 3,23-3,13 (m, 2H); 2,79 (t, *J* = 6,0 Hz, 2H); 2,57-2,38 (m, 2H); 1,30 (s, 18H). ¹³C RMN (CDCl₃, 75 MHz): *δ* (ppm) 168,7; 168,6; 157,8; 156,2; 155,2; 142,0; 135,6; 129,8; 128,1 (2C); 127,5; 126,8 (2C); 115,1; 104,1, 79,1 (2C); 70,2; 48,6; 46,7; 44,8 (2C) ; 43,1; 41,0 (2C); 34,7; 31,2; 27,9 (6C). MS (ESI +): m/z = [M + H] 642,3; [M + Na] 664,3.
HRMS (ESI +): m/z calculée pour C₃₃H₄₇N₅O₈Na [M + Na] = 664,3322; trouvée = 664,3322.
**Composé 23e** : ¹H RMN (CDCl₃, 400 MHz): *δ* (ppm) 7,38 (d, *J* = 7,1 Hz, 2H); 7,32 (t, *J* = 7,5 Hz, 2H); 7,26 (t, *J* = 7,8 Hz, 1H); 7,27 (m, 1H); 6,65 (d, *J* = 7,5 Hz, 1H); 6,05 (t, *J* = 7,0 Hz, 1H); 5,06 (s, 2H); 5,04-5,02 (s, 2H); 4,76-4,69 (m, 2H); 3,75-3,41 (m, 9H); 3,17-3,14 (m, 2H); 2,40-2,34 (m, 2H); 1,85-1,63 (m, 2H); 1,39 (s, 18H).
¹³C RMN (CDCl₃, 100 MHz): *δ* (ppm) 171,3; 165,6 (2C); 158,0; 156,1; 148,3; 135,9; 129,6; 128,5 (2C); 127,9; 127,2 (2C); 115,9; 104,8; 79,3 (2C); 70,7; 51,4; 49,2; 45,2; 44,8; 44,4; 42,0; 41,4; 29,7; 28,3 (6C); 27,8.
MS (ESI +): m/z = [M - Boc + 2H] 542,3; [M + H] 642,3; [M + Na] 664,3.
HRMS (ESI +): m/z calculée pour C₃₃H₄₇N₅O₈Na [M + Na] = 664,3322; trouvée = 664,3324.
**Composé 23f :** ¹H RMN (CDCl₃, 300 MHz): *δ* (ppm) 7,42 (d, *J* = 6,6 Hz, 2H); 7,37-7,28 (m, 3H); 7,11 (d, *J* = 5,9 Hz, 1H); 6,65 (d, *J* = 6,2 Hz, 1H); 6,02 (t, *J* = 7,2 Hz, 1H); 5,08 (s, 2H); 4,94-4,93 (s, 2H); 4,24 (t, *J* = 6,3 Hz, 2H); 3,54-3,32 (m, 9H); 3,17 (t, *J* = 5,4 Hz, 2H); 2,89 (t, *J* = 6,3 Hz, 2H); 2,41-2,34 (m, 2H); 1,94-1,82 (m, 2H); 1,40 (s, 18H).
¹³C RMN (CDCl₃, 75 MHz): *δ* (ppm) 171,4 (2C); 158,6; 156,7; 149,1; 140,1; 138,2; 130,7; 128,9 (2C); 128,4; 127,7 (2C); 115,9; 104,9; 87,1; 87,0; 71,1; 51,8; 47,6; 45,9 (2C); 45,0; 41,9 (2C); 32,0; 30,1; 28,7 (6C); 28,3.
MS (ESI +): m/z = [M + H] 656,3; [M + Na] 678,3.
HRMS (ESI +): m/z calculée pour C₃₄H₄₉N₅O₈Na [M + Na] = 678,3479; trouvée = 678,3474.
**Composé 23g** : ¹H RMN (CD₃OD, 300 MHz): *δ* (ppm) 7,61 (d, *J* = 7,5 Hz, 1H); 7,41-7,31 (m, 5H); 6,46 (d, *J* = 7,5 Hz, 1H); 5,05 (s, 2H); 4,68 (s, 2H); 3,58-3,54 (m, 1H); 3,23-3,18 (m, 2H); 3,12-2,97 (m, 2H); 2,07 (s, 3H); 1,58-1,30 (m, 22H).
¹³C RMN (CD₃OD, 75 MHz): *δ* (ppm) 175,6; 168,5; 163,7; 158,7; 147,3; 145,9; 143,0; 138,8; 130,4 (2C); 129,8 (2C); 129,6; 117,4; 80,4; 80,3; 75,0; 57,2; 52,2; 45,8; 40,9; 31,2; 29,1 (6C); 27,2; 13,3.
MS (ESI +): m/z = [M + Na] 595,3.
HRMS (ESI +): m/z calculée pour C₃₀H₄₄N₄O₇Na [M + Na] = 595,3108; trouvée = 595,3121.
**Composé 23h** : ¹H RMN (CD₃OD, 300 MHz): *δ* (ppm) 7,62 (d, *J* = 7,2 Hz, 1H); 7,42-7,32 (m, 5H); 6,47 (d, *J* = 7,2 Hz, 1H); 5,06 (s, 2H); 4,68 (s, 2H); 3,56-3,54 (m, 1H); 3,23-3,18 (m, 2H); 3,14-2,98 (m, 2H); 2,08 (s, 3H); 1,49-1,35 (m, 24H).
¹³C RMN (CD₃OD, 75 MHz): *δ* (ppm) 175,3; 168,2; 161,1; 158,7; 147,0; 145,6; 142,7; 138,5; 130,2 (2C); 129,5 (2C); 129,3; 117,1; 80,1; 80,0; 74,7; 56,9; 52,1; 45,5; 40,6; 33,1; 30,1; 28,9 (6C); 24,4; 13,0.
MS (ESI +): m/z = [M + Na] 609,3.
HRMS (ESI +): m/z calculée pour C₃₁H₄₆N₄O₇Na [M + Na] = 609,3264; trouvée = 609,3271.
**Composé 23i :** ¹H RMN (CDCl₃, 400 MHz): *δ* (ppm) 7,70 (s, 1H); 7,37-7,30 (m, 6H); 6,28 (d, *J* = 5,6 Hz, 1H); 5,40 (s, 1H); 5,18 (s, 1H); 5,06 (s, 2H); 4,10-4,06 (m, 2H); 3,51-3,49 (m, 1H); 3,24-3,04 (m, 4H); 2,65-2,50 (m, 4H); 2,12 (s, 3H); 1,44-1,37 (m, 4H); 1,37 (s, 18H).
¹³C RMN (CDCl₃, 75 MHz): *δ* (ppm) 173,6; 169,1; 157,0; 156,4; 145,8; 141,6; 139,2; 137,2; 128,9; 128,4 (2C); 128,3 (2C); 116,9; 79,2 (2C); 72,9; 52,3; 50,1; 44,4; 39,1; 36,2; 35,0; 31,9; 28,4 (6C); 22,7; 12,4.
MS (ESI +): m/z = [M + H] 601,3; [M + Na] 623,3.
HRMS (ESI +): m/z calculée pour C₃₂H₄₉N₄O₇ [M + H] = 601,3601; trouvée = 601,3621.
**Composé 23j** : ¹H RMN (CDCl₃, 300 MHz): *δ* (ppm) 7,58-7,55 (s, 1H); 7,37-7,24 (m, 6H); 6,32 (d, *J* = 5,6 Hz, 1H); 5,47-5,45 (s, 2H); 5,08 (s, 2H); 3,80-3,77 (m, 2H); 3,54-3,53 (m, 1H); 3,24-3,10 (m, 4H); 2,22 (t, *J* = 4,9 Hz, 2H); 2,03 (s, 3H); 1,93-1,70 (m, 4H); 1,36 (s, 18H).
¹³C RMN (CDCl₃, 75 MHz): *δ* (ppm) 173,8; 173,3; 156,9 (2C); 146,3; 141,4; 139,0; 137,4; 129,0 (2C); 128,4 (2C); 128,2; 117,1; 79,6 (2C); 73,1; 51,9; 51,3; 44,4; 36,2; 32,9; 30,7; 29,2; 28,4 (6C), 12,4.
MS (ESI +): m/z = [M + H] 587,3; [M + Na] 609,3.
HRMS (ESI +): m/z calculée pour C₃₁H₄₆N₄O₇Na [M + Na] = 609,3264; trouvée = 609,3265.
**Composé 23k** : ¹H RMN (CDCl₃, 400 MHz): *δ* (ppm) 7,35-7,28 (m, 6H); 6,42 (d, *J* = 7,2 Hz, 1H); 5,17 (s, 2H); 5,03 (s, 2H); 4,83 (m, 2H); 3,60-3,42 (m, 9H); 3,19 (m, 2H); 2,43-2,37 (m, 2H); 2,04 (s, 3H); 1,88-1,67 (m, 2H); 1,43 (s, 18H).
¹³C RMN (CDCl₃, 100 MHz): *δ* (ppm) 173,1; 171,4; 164,5; 157,0; 156,3; 145,6; 144,1; 140,8; 137,6; 129,0 (2C); 128,7 (2C); 128,4; 116,3; 79,6 (2C); 73,8; 55,3; 51,6; 45,1; 44,7; 42,5; 41,7; 41,4; 29,7; 28,6 (6C); 28,0; 13,0.
MS (ESI +): m/z = [M + H] 656,4; [M + Na] 678,3.
HRMS (ESI +): m/z calculée pour C₃₄H₄₉N₅O₈Na [M + Na] = 678,3479; trouvée = 678,3464.

### γ- Couplage des synthons issus des acides aspartique et glutamique avec la pipérazine (Schéma 5)

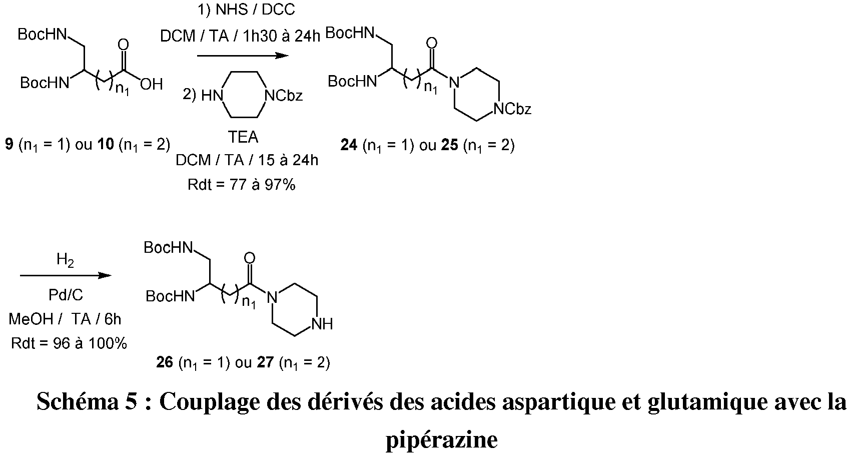

Le pipérazine-1-carboxylate de benzyle a été synthétisé en adaptant une méthode mise au point par Dener et al (WO 1998/04537 A1) en passant par le 4-benzyloxycarbonylpipérazine-1-carboxylate de *t*-butyle. Ainsi, le pipérazine-1-carboxylate de *t*-butyle est obtenu selon une procédure décrite par Moussa et al. (J. Med. Chem., 2010, 53, 6228-6239). Puis, il est mis à réagir (18,8 mmol) avec de la triéthylamine (1,2 eq) dans du dichlorométhane (50 mL) à 0°C pendant 10 min. Une solution de chloroformate de benzyle (1,2 eq) dans du dichlorométhane (30 mL) est ensuite introduite dans le milieu qui est placé sous agitation à température ambiante pendant 18h. Le dichlorométhane est alors évaporé sous pression réduite et le résidu est repris dans de l'acétate d'éthyle. La phase organique est finalement lavée avec une solution de KHSO₄ 1M et de NaHCO₃ 5%, puis une solution saturée de NaCl, séchée sur Na₂SO₄ et finalement évaporée sous pression réduite pour donner le 4-benzyloxycarbonylpipérazine-1-carboxylate de *t*-butyle sous la forme d'une poudre blanche (87%) après purification sur colonne de silice (CH₂Cl₂/MeOH 99:1). Cet intermédiaire (10,9 mmol) est finalement dissous dans du 1,4-dioxane (15 mL) en présence d'une solution commerciale de HC1 4N dans du 1,4-dioxane (20 eq). Le milieu réactionnel est alors placé sous agitation à température ambiante pendant 45 min. Le 1,4-dioxane est évaporé sous pression réduite et le résidu est trituré dans de l'éther pour donner le pipérazine-1-carboxylate de benzyle sous la forme d'une poudre blanche (92%), après filtration sous vide.

### * Composés 24 et 25 :

1 ^{ère} étape : A une solution du composé **9** ou **10** (3,1-4,5 mmol) dans du dichlorométhane (25-30 mL), sont additionnés successivement de la *N*-hydroxysuccinimide (1,2-1,5 eq) et du DCC (1,1-1,2 eq). Le milieu réactionnel est maintenu sous agitation à température ambiante pendant 1h30 à 24h, puis filtré sous vide. Le filtrat est ensuite évaporé sous pression réduite pour donner l'intermédiaire activé de l'acide.

2^{ème} étape : Le pipérazine-1-carboxylate de benzyle (1,2 eq) est dissous dans du dichlorométhane (40 mL) et mis à réagir avec de la triéthylamine (3 eq) à température ambiante pendant 30 min. L'intermédiaire activé de l'acide (1 eq) est ensuite introduit dans le milieu réactionnel qui est alors placé sous agitation à température ambiante pendant 15 à 24h. La phase organique est ensuite lavée avec une solution de HCl 1N, puis une solution saturée de NaHCO₃ et de NaCl, séchée sur Na₂SO₄ et finalement évaporée sous pression réduite pour donner le composé **24** ou **25** sous la forme d'une poudre blanche (77 ou 97%) après purification sur colonne de silice (CH₂Cl₂/MeOH 98:2).
**Composé 24 :** ¹H RMN (CDCl₃, 300 MHz): *δ* (ppm) 7,33 (m, 5H); 5,69 (s, 1H); 5,12 (s, 2H); 5,03 (s, 1H); 3,89 (m, 1H); 3,62-3,45 (m, 10H); 2,65-2,41 (m, 2H); 1,40 (s, 18H).
¹³C RMN (CDCl₃, 75 MHz): *δ* (ppm) 173,0; 163,7; 161,1; 147,7; 132,4; 128,9 (2C); 128,5; 128,3 (2C); 81,2 (2C); 67,8; 49,7; 45,9; 44,1 (2C); 41,8 (2C); 35,3; 28,7 (6C).
MS (ESI +): m/z = [M + H] 521,3; [M + Na] 543,2.
HRMS (ESI +): m/z calculée pour C₂₆H₄₀N₄O₇Na [M + Na] = 543,2795; trouvée = 543,2794.
**Composé 25 :** ¹H RMN (CDCl₃, 300 MHz): *δ* (ppm) 7,36-7,32 (m, 5H); 5,13 (s, 2H); 4,92 (s, 2H); 3,60-3,44 (m, 9H); 3,20-3,16 (m, 2H); 2,41-2,38 (m, 2H); 1.83-1,81 (m, 2H); 1,40 (s, 18H).
¹³C RMN (CDCl₃, 75 MHz): *δ* (ppm) 170,8; 164,1; 163,8; 154,7; 136,0; 128,2 (2C); 127,8; 127,6 (2C); 79,0 (2C); 67,1; 51,1; 44,8; 44,3; 43,4; 41,1 (2C); 29,2; 28,0 (6C); 27,5. MS (ESI +): m/z = [M + H] 535,3; [M + Na] 557,3.
HRMS (ESI +): m/z calculée pour C₂₇H₄₂N₄O₇Na [M + Na] = 557,2951; trouvée = 557,2945.

### * Composés 26 et 27 :

À une solution du composé **24** ou **25** (0,7-2,7 mmol) dans du méthanol (8-30 mL), est additionné du Pd/C (10% m/m). Le milieu réactionnel est placé sous vide et sous agitation à température ambiante pendant 30 min, puis maintenu sous un flux d'H₂ pendant 6h. Il est ensuite filtré sur papier et le méthanol est évaporé sous pression réduite pour donner respectivement le dérivé **26** ou **27** sous la forme d'une poudre blanche (100 ou 96%).
**Composé 26 :** ¹H RMN (CDCl₃, 300 MHz): *δ* (ppm) 7,33 (s, 1H); 5,69-5,66 (s, 1H); 5,12 (s, 1H); 3,89-3,88 (m, 1H); 3,66-3,23 (m, 8H); 2,93-2,83 (m, 2H); 2,67-2,43 (m, 2H); 1,41 (s, 18H).
¹³C RMN (CDCl₃, 75 MHz): *δ* (ppm) 167,1; 157,5 ; 155,4; 79,1 (2C); 48,6 ; 45,0; 44,6; 43,1; 40,9; 34,8; 27,9 (6C).
MS (ESI +): m/z = [M + H] 387,3; [M + Na] 409,3.
HRMS (ESI +): m/z calculée pour C₁₈H₃₄N₄O₅Na [M + Na] = 409,2427; trouvée = 409,2418.
**Composé 27 :** ¹H RMN (CDCl₃, 300 MHz): *δ* (ppm) 5,13-5,09 (s, 2H); 3,56-3,52 (m, 3H); 3,39-3,36 (m, 2H); 3,15-3,12 (m, 2H); 2,80-2,76 (m, 4H); 2,37-2,31 (m, 2H); 1,81-1,63 (m, 2H); 1,37 (s, 18H).
¹³C RMN (CDCl₃, 75 MHz): *δ* (ppm) 170,8; 156,5; 156,2; 80,3; 79,1; 51,3; 46,0 ; 45,3 ; 44,5; 42,6; 41,5; 29,4; 28,2 (6C); 27,7.
MS (ESI +): m/z = [M + H] 401,4; [M + Na] 423,3.
HRMS (ESI +): m/z calculée pour C₁₉H₃₆N₄O₅Na [M + Na] = 423,2583; trouvée = 423,2573.

### b) Composés porteurs d'un groupement dérivé de l'acide férulique ou de l'acide gallique

### α- Préparation du synthon R'ₐ-OH issu de l'acide gallique

La triple O-benzylation des groupements phénoliques de l'acide gallique est décrite dans la littérature (Carbohydr. Res., 2007, 342, 1510-1513).

### β- Débenzylation du groupement R'ₐ issu de l'acide gallique (Schéma 6)

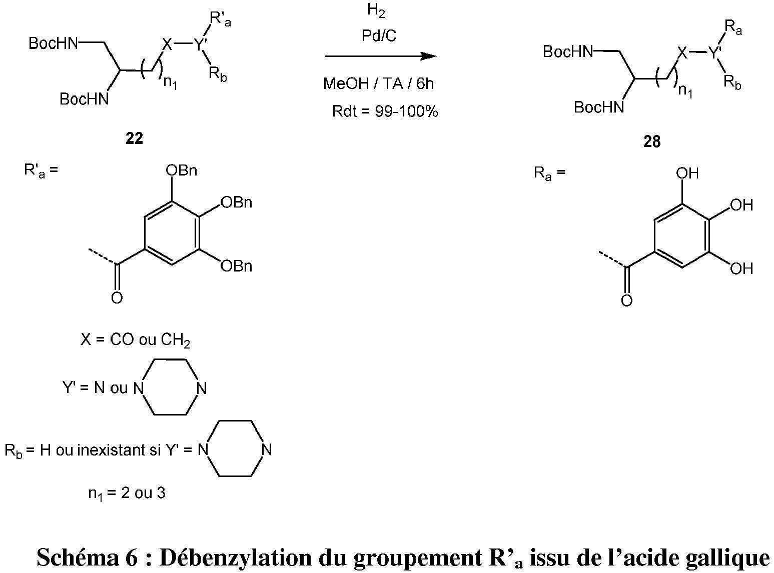

À une suspension du composé **22a** ou **22b** (0,5 mmol) dans du méthanol (20 mL), est additionné du Pd/C (10% m/m). Le milieu réactionnel est placé sous vide et sous agitation à température ambiante pendant 30 min, puis maintenu sous un flux d'H₂ pendant 6h. Il est ensuite filtré sur papier et le méthanol est évaporé sous pression réduite pour donner respectivement le dérivé **28a** ou **28b** (soluble dans le méthanol, contrairement au produit de départ) sous la forme d'une poudre blanche ou d'une huile orangée (99 ou 100%).
**Composé 28a :** ¹H RMN (CD₃OD, 400 MHz): *δ* (ppm) 6,45 (s, 2H); 3,59-3,43 (m, 8H); 3,32-3,31 (m, 1H); 3,06-3,01 (m, 2H); 2,47-2,45 (m, 2H); 2,01-1,70 (m, 2H); 1,43 (s, 18H).
¹³C RMN (CD₃OD, 100 MHz): *δ* (ppm) 172,4; 171,9; 157,3 ; 157,0; 145,8 (2C); 135,3; 125,0; 106,3 (2C); 78,7 (2C); 50,5; 45,1 (2C); 43,8; 42,3 (2C); 33,5; 29,1; 27,4 (6C).
MS (ESI +): m/z = [M + H] 553,1; [M + Na] 575,1.
HRMS (ESI +): m/z calculée pour C₂₆H₄₀N₄O₉Na [M + Na] = 575,2693; trouvée = 575,2719.
**Composé 28b :** ¹H RMN (CDCl₃, 300 MHz): *δ* (ppm) 7,25 (s, 3H); 6,84 (s, 2H); 5,43 (s, 1H); 5,24-5,21 (s, 2H); 3,15-3,04 (m, 5H); 1,41-1,35 (m, 22H); 1,20-1,16 (m, 2H).
¹³C RMN (CDCl₃, 75 MHz): *δ* (ppm) 168,4; 156,7; 156,4; 144,3 (2C); 135,7; 124,6; 106,9 (2C); 79,1 (2C); 50,7; 44,2; 39,5; 31,9; 29,2; 27,9 (6C); 22,6.
MS (ESI +): m/z = [M + H] 484,2; [M + Na] 506,3.
HRMS (ESI +): m/z calculée pour C₂₃H₃₇N₃O₈Na [M + Na] = 506,2478; trouvée = 506,2501.

### γ- Déprotection finale du groupement diamine (Schéma 7)

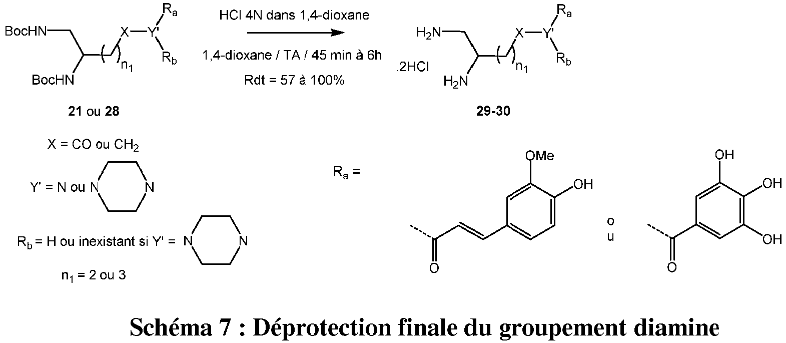

Le composé **21a, 21b, 28a** ou **28b** (0,3-0,4 mmol) est dissous dans du 1,4-dioxane (7-12 mL) en présence d'une solution commerciale de HCl 4N dans du 1,4-dioxane (20 eq). Le milieu réactionnel est alors placé sous agitation à température ambiante pendant 45 min à 6h. Le 1,4-dioxane est évaporé sous pression réduite et le résidu est trituré dans de l'éther. Après évaporation sous vide (produits très hygroscopiques), le précipité obtenu est lyophilisé pour donner respectivement le dérivé **29a, 29b, 30a** ou **30b** sous la forme d'une poudre jaune ou beige (75, 66, 100 ou 57%). **Composé 29a :** ¹H RMN (d₆-DMSO, 300 MHz): *δ* (ppm) 9,52 (s, 1H); 8,57 (s, 2H); 8,46 (s, 2H); 7,44 (d, *J* = 15,0 Hz, 1H); 7,33 (s, 1H); 7,10 (m, 2H); 6,79 (d, *J* = 8,4 Hz, 1H); 3,83 (s, 3H); 3,71-3,45 (m, 9H); 3,12 (m, 2H); 2,60-2,58 (m, 2H); 1,89 (m, 2H).
¹³C RMN (d₆-DMSO, 75 MHz): *δ* (ppm) 169,7; 165,0; 162,9; 148,5; 142,4; 126,4; 122,5; 115,3; 114,2; 111,2; 55,7; 48,7; 39,7 (5C); 28,1; 25,3.
MS (ESI +): m/z = [M + H] 377,2.
HRMS (ESI +): m/z calculée pour C₁₉H₂₉N₄O₄ [M + H] = 377,2189; trouvée = 377,2198.
**Composé 29b :** ¹H RMN (d₆-DMSO, 300 MHz): *δ* (ppm) 8,49 (s, 4H); 8,11-8,09 (s, 1H); 7,30 (d, *J* = 15,9 Hz, 1H); 7,11 (d, *J* = 1.8 Hz, 1H); 6,97 (dd, *J₁* = 8,4 Hz et *J₂* = 1.8 Hz, 1H); 6,79 (d, *J* = 8,1 Hz, 1H); 6,49 (d, *J* = 15,9 Hz, 1H); 3,78 (s, 3H); 3,42-3,39 (m, 1H); 3,17 - 3,05 (m, 4H); 1,64-1,62 (m, 2H); 1,46-1,40 (m, 4H).
¹³C RMN (d₆-DMSO, 75 MHz): *δ* (ppm) 165,3; 148,2; 147,7; 138,7; 126,3; 121,4; 119,0; 115,6; 110,7; 55,4; 48,9; 38,1 (2C); 29,5; 28,6; 21,7.
MS (ESI +): m/z = [M + H] 308,1.
HRMS (ESI +): m/z calculée pour C₁₆H₂₆N₃O₃ [M + H] = 308,1974; trouvée = 308,1986.
**Composé 30a :** ¹H RMN (d₆-DMSO, 300 MHz): *δ* (ppm) 8,65 (s, 2H); 8,55 (s, 2H); 6,37 (s, 2H); 3,49-3,48 (m, 9H); 3,13 (m, 2H); 2,59 (t, *J* = 4,8 Hz, 2H); 1,90-1,88 (m, 2H). ¹³C RMN (d₆-DMSO, 75 MHz): *δ* (ppm) 169,5; 169,3; 145,3 (2C); 134,5; 124,9; 106,3 (2C); 48,5; 40,0 (2C); 39,7; 38,3; 27,8; 25,0.
MS (ESI +): m/z = [M + H] 352,9.
HRMS (ESI +): m/z calculée pour C₁₆H₂₅N₄O₅ [M + H] = 353,1825; trouvée = 353,1813.
**Composé 30b :** ¹H RMN (d₆-DMSO, 300 MHz): *δ* (ppm) 8,47 (s, 4H); 8,11 (s, 1H); 6,82 (s, 2H); 3,24-3,07 (m, 5H); 1,65-1,35 (m, 6H).
¹³C RMN (d₆-DMSO, 75 MHz): *δ* (ppm) 166,5; 145,4; 136,1; 125,0; 106,8 (2C); 49,0; 40,4 (2C); 29,6; 28,8; 21,8.
MS (ESI +): m/z = [M + H] 284,2.
HRMS (ESI +): m/z calculée pour C₁₃H₂₂N₃O₄ [M + H] = 284,1610; trouvée = 284,1612.

### c) Composés porteurs d'un groupement hydroxypyridinone

### α- Préparation des synthons R'ₐ-OH et R'ₐ-NHR_{b}

### * Préparation des synthons R'ₐ-OH et R'ₐ-NHR_{b} de type 3-benzyloxypyridin-2-one (Schéma 7) :

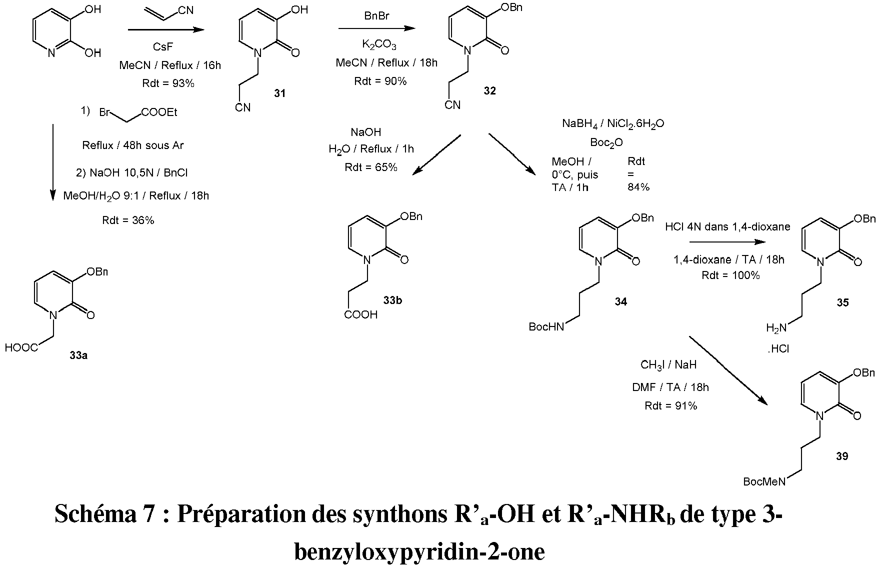

### * Composé 31 (Synthesis, 2011, 57-64) :

À une solution de 2,3-dihydroxypyridine (135 mmol) dans de l'acétonitrile (120 mL), sont additionnés du fluorure de césium (0,1 eq) et de l'acrylonitrile (3 eq) (Tetrahedron Lett., 2002, 43, 7379-7383 ; Synthesis, 2011, 57-64). Le milieu réactionnel est chauffé à reflux pendant 16h. L'acétonitrile est alors évaporé sous pression réduite et le produit souhaité est extrait à l'aide d'acétate d'éthyle. La phase organique est lavée avec une solution de Na₂CO₃ 10%, puis une solution saturée de NaCl, séchée sur Na₂SO₄ et finalement évaporée sous pression réduite pour donner le dérivé 31 sous la forme d'une poudre blanche (93%) après recristallisation dans un mélange AcOEt/Cyclohexane 50:50.

### * Composé 32 (Synthesis, 2011, 57-64) :

Le dérivé **32** a été préparé selon la synthèse décrite par Arumugam et al. (Synthesis, 2011, 57-64) en 2011.

### * Composé 33a (J. Med. Chem., 1990, 33, 1749-1755, Molecules, 2015, 20, 19393-19405) :

1 ^{ère} étape : De la 2,3-dihydroxypyridine (50 mmol) est additionnée à du bromoacétate d'éthyle (5 eq). Le milieu réactionnel est alors chauffé au reflux pendant 48h sous Ar et le produit souhaité est finalement obtenu après précipitation dans l'acétate d'éthyle et filtration sous vide.

2^{ème} étape : A une solution de cet intermédiaire (18 mmol) dans un mélange MeOH/H₂O 9:1 (150 mL), est additionnée une solution de NaOH 10,5N (2 eq). Le milieu réactionnel est alors chauffé au reflux pendant 30 min. Du chlorure de benzyle (2 eq) est ensuite introduit en goutte à goutte sur une période de 30 min à température ambiante et le mélange est à nouveau chauffé au reflux pendant 18h. Après filtration sous vide et évaporation du méthanol sous pression réduite, la phase aqueuse est extraite à l'aide de dichlorométhane, puis acidifiée à l'aide de HCl 6N. Le produit souhaité est finalement extrait à l'aide de dichlorométhane. La phase organique est lavée avec une solution saturée de NaCl, séchée sur Na₂SO₄ et évaporée sous pression réduite pour donner le dérivé **33a** sous la forme d'une poudre blanche (36%).

### ° Composé 33b :

À une solution du composé **32** (1 eq) dans de l'eau (20 mL/mmol), est additionné de l'hydroxyde de sodium (12,5 eq). Le milieu réactionnel est alors chauffé au reflux pendant 1h, puis extrait à l'aide d'acétate d'éthyle afin d'éliminer la matière première restante. La phase aqueuse est ensuite acidifiée à l'aide d'une solution d'HCl 6N et le composé souhaité est extrait à l'aide d'acétate d'éthyle. La phase organique est lavée à l'aide d'une solution de NaCl saturée, séchée sur Na₂SO₄ et finalement évaporée sous pression réduite pour donner le dérivé **33** sous la forme d'une poudre jaunâtre (65%).
**Composé 33b :** ¹H RMN (CDCl₃, 300 MHz): *δ* (ppm) 7,43-7,32 (m, 5H); 7,25 (dd, *J₁* = 6,9 Hz et *J₂* = 1,5 Hz, 1H); 6,87 (dd, *J₁* = 7,3 Hz et *J₂* = 1,8 Hz, 1H); 6,09 (t, *J* = 7,2 Hz, 1H); 4,99 (s, 2H); 4,07 (t, *J* = 6,9 Hz, 2H); 2,65 (t, *J* = 6,9 Hz, 2H).
¹³C RMN (CDCl₃, 75 MHz): *δ* (ppm) 171,9; 156,5; 147,5; 136,2; 130,1; 128,0 (2C); 127,5; 127,4 (2C); 115,1; 103,4; 69,4; 45,0; 32,5.
MS (ESI +): m/z = [M + H] 273,8.
HRMS (ESI +): m/z calculée pour C₁₅H₁₅NO₄Na [M + Na] = 296,0899; trouvée = 296,0893.

### * Composé 34 :

À une solution du composé **32** (7,9 mmol) dans du méthanol (80 mL), placée sous agitation à 0°C, sont additionnés du dicarbonate de di-*t*-butyle (2 eq) et du NiCl₂.6H₂O (0,1 eq). Du borohydrure de sodium (7 eq) est ensuite introduit par petites portions sur une période de 30 min et le mélange est placé sous agitation à température ambiante pendant 1h. Après addition de diéthylènetriamine (1 eq) et agitation à nouveau pendant 1h, le méthanol est évaporé sous pression réduite et le produit souhaité est extrait à l'aide d'acétate d'éthyle. La phase organique est lavée avec une solution saturée de NaHCO₃, puis de NaCl, séchée sur Na₂SO₄ et finalement évaporée sous pression réduite pour donner le dérivé **34** sous la forme d'une poudre blanchâtre (84%).
**Composé 34 :** ¹H RMN (CDCl₃, 400 MHz): *δ* (ppm) 7,33 (d, *J* = 7,6 Hz, 2H); 7,25 (t, *J* = 6,8 Hz, 2H); 7,19 (t, *J* = 6,8 Hz, 1H); 6,80 (d, *J* = 6,3 Hz, 1H); 6,55 (d, *J* = 7,4 Hz, 1H); 5,96 (t, *J* = 7,3 Hz, 1H); 5,49 (s, 1H); 5,01 (s, 2H); 3,95 (t, *J* = 6,3 Hz, 2H); 3,01-2,96 (m, 2H); 1,81-1,75 (m, 2H); 1,33 (s, 9H).
¹³C RMN (CDCl₃, 100 MHz): *δ* (ppm) 158,3; 155,9; 148,5; 135,8; 128,4; 128,3 (2C); 127,8; 127,1 (2C); 115,1; 105,2; 78,6; 70,5; 46,0; 36,3; 29,7; 28,3 (3C).
MS (ESI +): m/z = [M + H] 358,9; [M + Na] 380,9.
HRMS (ESI +): m/z calculée pour C₂₀H₂₆N₂O₄Na [M + Na] = 381,1790; trouvée = 381,1782.

### * Composé 35 :

Le composé **34** (2,4 mmol) est dissous dans du 1,4-dioxane (10 mL) en présence d'une solution commerciale de HCl 4N dans du 1,4-dioxane (20 eq). Le milieu réactionnel est alors placé sous agitation à température ambiante pendant 18h. Le 1,4-dioxane est évaporé sous pression réduite et le résidu est trituré dans de l'éther pour donner le dérivé **35** sous la forme d'une poudre beige (100%), après filtration sous vide.
**Composé 35 :** ¹H RMN (d₆-DMSO, 300 MHz): *δ* (ppm) 8,06-8,05 (s, 2H); 7,45-7,33 (m, 6H); 6,93 (d, *J* = 7,5 Hz, 1H); 6,18 (t, *J* = 7,2 Hz, 1H); 5,01 (s, 2H); 4,00 (t, *J* = 6,9 Hz, 2H); 2,75 (t, *J* = 7,2 Hz, 2H); 1,98-1,93 (m, 2H).
¹³C RMN (d₆-DMSO, 75 MHz): *δ* (ppm) 175,1; 147,6; 136,1; 129,5; 128,0 (2C); 127,5 (3C); 115,3; 104,1; 69,4; 45,4; 35,9; 26,5.
MS (ESI +): m/z = [M + H] 259,1; [M + H + MeCN] 300,2.
HRMS (ESI +): m/z calculée pour C₁₅H₁₉N₂O₂ [M + H] = 259,1436; trouvée = 259,1447.

### ° Composé 39 :

À une solution du composé **34** (1,5 mmol) dans du DMF (3 mL), est additionné de l'hydrure de sodium (1,4 eq). Après 10 min d'agitation à température ambiante, de l'iodure de méthyle (1,2 eq) est introduit et le mélange est placé sous agitation à température ambiante pendant 18h (Org. Lett., 2014, 16, 3196-3199). Après évaporation du DMF sous pression réduite, le résidu est repris dans l'acétate d'éthyle. La phase organique est lavée avec une solution saturée de NaCl, séchée sur Na₂SO₄ et finalement évaporée sous pression réduite pour donner le dérivé **39** sous la forme d'une huile jaune (91%) après purification sur colonne de silice (AcOEt/MeOH 50:50).
**Composé 39 :** ¹H RMN (CDCl₃, 400 MHz): *δ* (ppm) 7,40 (d, *J* = 8,0 Hz, 2H); 7,31 (t, *J =* 8,5 Hz, 2H); 7,26 (t, *J* = 7,2 Hz, 1H); 6,93-6,86 (m, 1H); 6,61 (d, *J₁* = 7,4 Hz et *J₂* = 1,6 Hz, 1H); 5,99 (t, *J* = 7,1 Hz, 1H); 5,07 (s, 2H); 3,92 (t, *J* = 7,2 Hz, 2H); 3,27 (m, 2H); 2,82 (s, 3H); 1,96 (t, *J* = 7,2 Hz, 2H); 1,41 (s, 9H).
¹³C RMN (CDCl₃, 100 MHz): *δ* (ppm) 158,2; 149,0; 136,4; 128,6 (2C); 128,0; 127,4 (2C); 115,5; 104,8; 79,6; 70,8; 47,8; 45,8; 34,1; 28,5 (3C); 27,1.
MS (ESI +): m/z = [M + H] 373,2; [M + Na] 395,2.
HRMS (ESI +): m/z calculée pour C₂₁H₂₉N₂O₄ [M + H] = 373,2127; trouvée = 373,2137.

### * Préparation des synthons R'ₐ-OH et R'ₐ-NHR_{b} de type 3-benzyloxy-2-méthylpyridin-4-one (Schéma 8) :

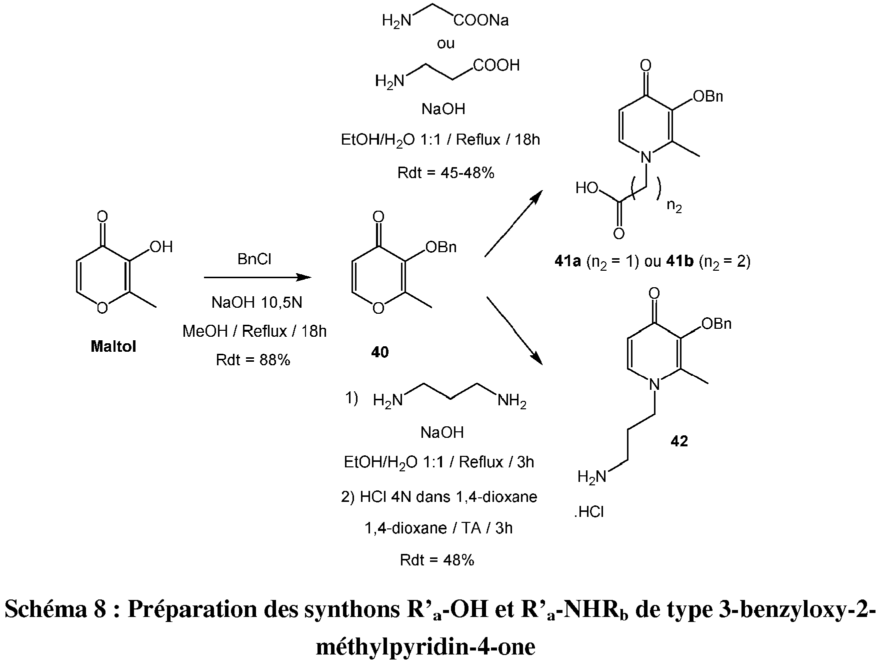

### ° Composé 40 (J. Med. Chem., 1998, 41, 3347-3359, J. Inorg. Biochem., 2000, 78, 303-311) :

À une solution de maltol (79,3 mmol) dans du méthanol (80 mL), est additionnée une solution de NaOH 10,5N (1,1 eq). Le milieu réactionnel est alors chauffé au reflux pendant 30 min. Du chlorure de benzyle (1,2 eq) est ensuite introduit en goutte à goutte sur une période de 30 min à température ambiante et le mélange est à nouveau chauffé au reflux pendant 18h. Après filtration sous vide, le méthanol est évaporé sous pression réduite, le résidu est ensuite repris dans l'eau et le produit souhaité est extrait à l'aide de dichlorométhane. La phase organique est lavée avec une solution de NaOH 5%, puis une solution saturée de NaCl, séchée sur Na₂SO₄ et finalement évaporée sous pression réduite pour donner le dérivé **40** sous la forme d'une huile jaune (88%).

### ° Composés 41a (J. Heterocyclic Chem., 1994, 31, 947-56) et 41b (J. Inorg. Biochem., 2000, 78, 303-311) :

À une solution de NaOH (3 eq) dans un mélange EtOH/H₂O 1:1 (100 mL), sont additionnés le composé **40** (18,5 mmol) et du glycinate de sodium ou de la *β-*alanine (2 eq) (Bioconjugate Chem. 2005, 16, 1597-1609). Le milieu réactionnel est alors chauffé au reflux pendant 18h. Après évaporation sous vide de l'éthanol, le résidu est repris dans l'eau et extrait à l'aide d'acétate d'éthyle afin d'éliminer la matière première restante. La phase aqueuse est ensuite concentrée sous pression réduite et acidifiée à l'aide d'une solution d'HCl 6N et le dérivé **41a** ou **41b** est finalement obtenu après précipitation, filtration sous vide et lavage à l'eau, sous la forme d'une poudre blanchâtre (48 ou 45%).

### ° Composé 42 (Dalton Trans., 2004, 3772-3781) :

À une solution de NaOH (0,5 eq) dans un mélange EtOH/H₂O 1:1 (20 mL), sont additionnés le composé **40** (18,5 mmol) et du 1,3-diaminopropane (1,1 eq). Le milieu réactionnel est alors chauffé au reflux pendant 18h. Après évaporation sous vide de l'éthanol, la phase aqueuse est acidifiée à l'aide de HCl 6N et extraite à l'aide d'acétate d'éthyle afin d'éliminer la matière première restante. La phase aqueuse est ensuite neutralisée à l'aide d'une solution de NaOH 6N et le produit souhaité est extrait à l'aide d'acétate d'éthyle. La phase organique est lavée avec une solution saturée de NaCl, séchée sur Na₂SO₄ et finalement évaporée sous pression réduite. Après agitation du composé intermédiaire à température ambiante pendant 2h dans du 1,4-dioxane (20 mL) en présence d'une solution commerciale de HCl 4N dans du 1,4-dioxane (5 eq) et évaporation sous pression réduite du 1,4-dioxane, le résidu obtenu est trituré dans de l'éther pour donner le dérivé **42** sous la forme d'une poudre blanche (48%).

### β - Différentes pharmacomodulations réalisées au niveau du « linker »

### * Réduction des groupements carbonylés du composé 23f (Schéma 9) :

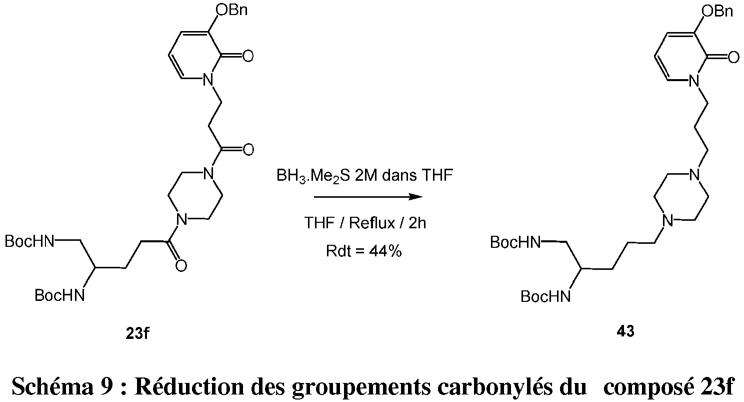

À une solution du composé **23f** (1 mmol) dans du THF (100 mL), est additionnée goutte à goutte une solution de complexe borane/sulfure de diméthyle 2M dans du THF (5 eq) (J. Med. Chem., 2005, 48, 3891-3902). Le milieu réactionnel est alors chauffé au reflux pendant 2h. Après refroidissement, le borane est piégé à l'aide de MeOH et le mélange est à nouveau chauffé au reflux pendant 18h. Après évaporation sous vide du MeOH, le résidu est repris dans un mélange EtOH/NaOH 1N 5:1 porté au reflux pendant 2h. La phase aqueuse est finalement extraite à l'aide d'acétate d'éthyle après évaporation de l'éthanol. La phase organique est lavée avec une solution saturée de NaCl, séchée sur Na₂SO₄ et évaporée sous pression réduite pour donner le dérivé **43** sous la forme d'une huile incolore (44%) après purification sur colonne de silice (AcOEt/MeOH 50:50).
**Composé 43 :** ¹H RMN (CDCl₃, 400 MHz): *δ* (ppm) 7,40 (d, *J* = 7,3 Hz, 2H); 7,32 (t, *J* = 7,2 Hz, 2H); 7,26 (t, *J* = 7,8 Hz, 1H); 6,93 (d, *J* = 6,8 Hz, 1H); 6,61 (d, *J* = 7,4 Hz, 1H); 5,96 (t, *J* = 7,1 Hz, 1H); 5,27 (s, 3H); 5,08 (m, 2H); 4,98 (s, 1H); 3,99 (t, *J* = 6,8 Hz, 2H); 3,57 (m, 1H); 3,14 (m, 2H); 2,43-2,31 (m, 10H); 1,92 (t, *J* = 6,8 Hz, 2H); 1,54-1,52 (m, 2H); 1,40 (m, 20H).
¹³C RMN (CDCl₃, 100 MHz): *δ* (ppm) 158,5 (2C); 149,0; 136,8; 129,8; 128,7 (2C); 128,1; 127,5 (2C); 115,7; 104,5; 79,4 (2C); 70,9; 58,2; 54,8 (2C); 53,4; 53,0; 51,2; 48,2; 45,1; 30,7; 29,9; 28,6 (6C); 26,0; 23,2.
MS (ESI +): m/z = [M + H] 628,3; [M + Na] 650,3.
HRMS (ESI +): m/z calculée pour C₃₄H₅₄N₅O₆ [M + H] = 628,4074; trouvée = 628,4080.

### * Préparation d'un analogue N-méthylé et décarbonylé du composé 23c (Schéma 10) :

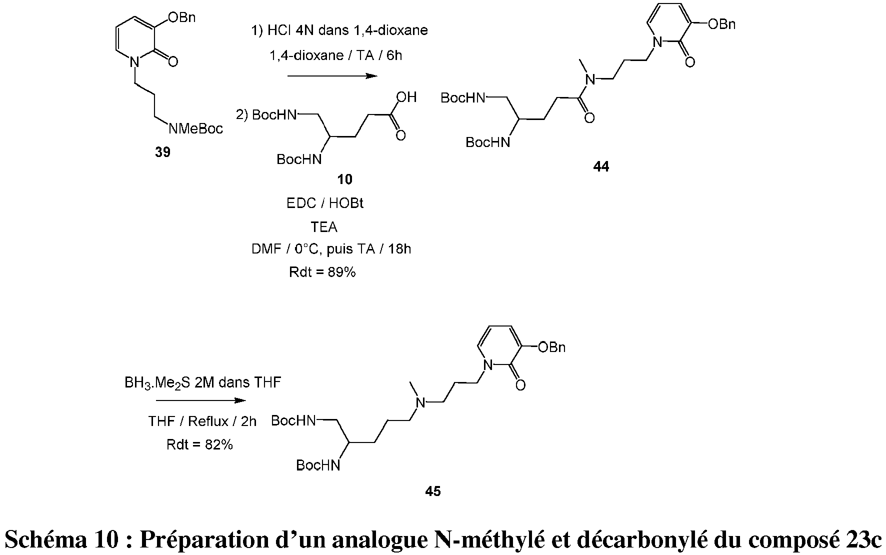

### ° Composé 44

1 ^{ère} étape : Le composé **39** (0,7 mmol) est dissous dans du 1,4-dioxane (2 mL) en présence d'une solution commerciale de HCl 4N dans du 1,4-dioxane (20 eq). Le milieu réactionnel est alors placé sous agitation à température ambiante pendant 6h. Le dioxane est évaporé sous pression réduite et le résidu est trituré dans de l'éther pour donner le chlorhydrate d'ammonium correspondant sous la forme d'un précipité.

2^{ème} étape : A une solution du composé **10** (0,8 mmol) dans du DMF (20 mL), placée sous agitation à 0°C, sont additionnés successivement du chlorhydrate de 1-éthyl-3-(3-diméthylaminopropyl)-carbodiimide (1,2 eq), du 1-hydroxybenzotriazole monohydrate (1,1 eq). Après 30 min d'agitation à 0°C, le dérivé aminé intermédiaire (1 eq) est introduit avec de la triéthylamine (1,2 eq) dans le milieu réactionnel qui est alors maintenu sous agitation à température ambiante pendant 18h. Après évaporation du DMF et reprise du résidu dans l'acétate d'éthyle, la phase organique est lavée avec une solution de HCl 1N, de NaHCO₃, puis de NaCl saturée, séchée sur Na₂SO₄ et finalement évaporée sous pression réduite pour donner après purification sur colonne de silice (AcOEt/MeOH 50:50) le composé **45** (89%).
**Composé 44 :** ¹H RMN (CDCl₃, 400 MHz): *δ* (ppm) 7,39 (d, *J* = 7,1 Hz, 2H); 7,31 (t, *J =* 7,6 Hz, 2H); 7,27-7,25 (m, 1H); 7,01 (d, *J* = 6,5 Hz, 1H); 6,61 (d, *J* = 7,5 Hz, 1H); 5,99 (t, *J* = 7,1 Hz, 1H); 5,06 (s, 2H); 5,02 (m, 2H); 3,95-3,88 (m, 2H); 3,60-3,59 (m, 1H); 3,46-3,29 (m, 2H); 3,17-3,15 (m, 2H); 2,95 (s, 3H); 2,43-2,29 (m, 2H); 2,02-1,92 (m, 2H); 1,85-1,77 (m, 2H); 1,39 (s, 18H).
¹³C RMN (CDCl₃, 100 MHz): *δ* (ppm) 173,0; 158,4 (2C); 149,2; 136,6; 129,7; 128,8 (2C); 128,3; 127,6 (2C); 115,9; 104,9; 79,5; 79,4; 71,0; 51,7; 48,1; 45,3; 45,1; 35,4; 30,1; 28,6 (6C); 27,9; 27,0.
MS (ESI +): m/z = [M + Na] 609,3.
HRMS (ESI +): m/z calculée pour C₃₁H₄₆N₄O₇Na [M + Na] = 609,3264; trouvée = 609,3268.

### ° Composé 45

À une solution du composé **44** (0,5 mmol) dans du THF (30 mL), est additionnée goutte à goutte une solution de complexe borane/sulfure de diméthyle 2M dans du THF (2,5 eq). Le milieu réactionnel est alors chauffé au reflux pendant 2h. Après refroidissement, le borane est piégé à l'aide de MeOH et le mélange est à nouveau chauffé au reflux pendant 18h. Après évaporation sous vide du MeOH, le résidu est repris dans un mélange EtOH/NaOH 1N 5:1 porté au reflux pendant 2h. La phase aqueuse est finalement extraite à l'aide d'acétate d'éthyle après évaporation de l'éthanol. La phase organique est lavée avec une solution saturée de NaCl, séchée sur Na₂SO₄ et évaporée sous pression réduite pour donner le dérivé **45** sous la forme d'une poudre brune (82%) après purification sur colonne de silice (AcOEt/MeOH 70:30).
**Composé 45 :** ¹H RMN (CDCl₃, 400 MHz): *δ* (ppm) 7,41 (d, *J* = 7,3 Hz, 2H); 7,33 (t, *J* = 6,7 Hz, 2H); 7,26 (t, *J* = 6,8 Hz, 1H); 6,94 (d, *J* = 6,9 Hz, 1H); 6,63 (d, *J* = 7,4 Hz, 1H); 6,00 (t, *J* = 7,2 Hz, 1H); 5,08 (s, 2H); 5,08 (s, 2H); 5,06-5,01 (m, 2H); 3,99 (t, *J* = 6,7 Hz, 2H); 3,58 (m, 1H); 3,16-3,15 (m, 2H); 2,37-2,30 (m, 4H); 2,17 (s, 3H); 1,92 (t, *J* = 7,1 Hz, 2H); 1,51-1,50 (m, 2H); 1,40 (m, 20H).
¹³C RMN (CDCl₃, 100 MHz): *δ* (ppm) 158,2 (2C); 149,0; 136,5; 129,6; 128,6 (2C); 128,0; 127,4 (2C); 115,7; 104,6; 79,3 (2C); 70,8; 57,2; 54,4; 51,3; 48,1; 45,0; 41,6; 30,7; 28,5 (6C); 23,4; 21,1.
MS (ESI +): m/z = [M + H] 573,4; [M + Na] 595,3.
HRMS (ESI +): m/z calculée pour C₃₁H₄₉N₄O₆ [M + H] = 573,3652; trouvée = 573,3664.

### * Préparation d'un dérivé porteur d'un « linker » aliphatique dépourvu de fonction amide (Schéma 11) :

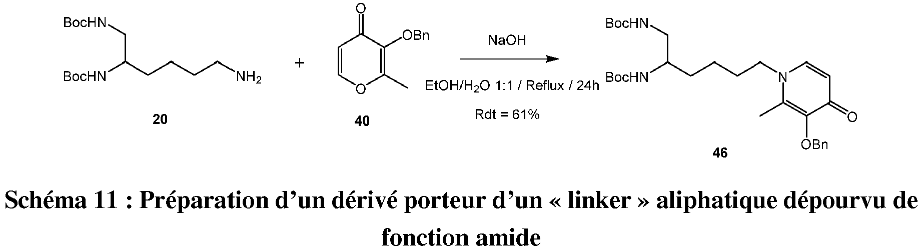

### ° Composé 46:

À une solution de NaOH (0,5 eq) dans un mélange EtOH/H₂O 1:1 (120 mL), sont additionnés le composé **40** (5,1 mmol) et le composé **20** (0,7 eq). Le milieu réactionnel est alors chauffé au reflux pendant 24h (Dalton Trans., 2004, 3772-3781). Après évaporation sous vide de l'éthanol, la phase aqueuse est neutralisée à l'aide de HCl 6N et extraite à l'aide d'acétate d'éthyle. La phase organique est lavée avec une solution saturée de NaCl, séchée sur Na₂SO₄ et finalement évaporée sous pression réduite pour donner le dérivé **46** sous la forme d'une poudre jaune (61%) après purification sur colonne de silice (AcOEt/MeOH 90:10).
**Composé 46 :** ¹H RMN (CDCl₃, 400 MHz): *δ* (ppm) 7,38 (d, *J* = 6,7 Hz, 2H); 7,32-7,26 (m, 3H); 7,16 (d, *J* = 7,5 Hz, 1H); 6,39 (d, *J* = 7,5 Hz, 1H); 5,03 (s, 2H); 4,95-4,93 (s, 2H); 3,71-3,76 (m, 2H); 3,59 (m, 1H); 3,14 (m, 2H); 2,06 (s, 3H); 1,66-1,55 (m, 2H); 1,40-1,35 (m, 22H).
¹³C RMN (CDCl₃, 100 MHz): *δ* (ppm) 173,5; 156,6 (2C); 146,4; 141,0; 138,3; 137,9; 129,3 (2C); 128,4 (2C); 128,1; 117,5; 79,7 (2C); 73,1; 53,9; 51,2; 44,7; 32,6; 30,9; 28,5 (6C); 23,0; 12,5.
MS (ESI +): m/z = [M + H] 530,3.
HRMS (ESI +): m/z calculée pour C₂₉H₄₄N₃O₆ [M + H] = 530,3230; trouvée = 530,3245.

### γ - Débenzylation du groupement R'ₐ (Schéma 12)

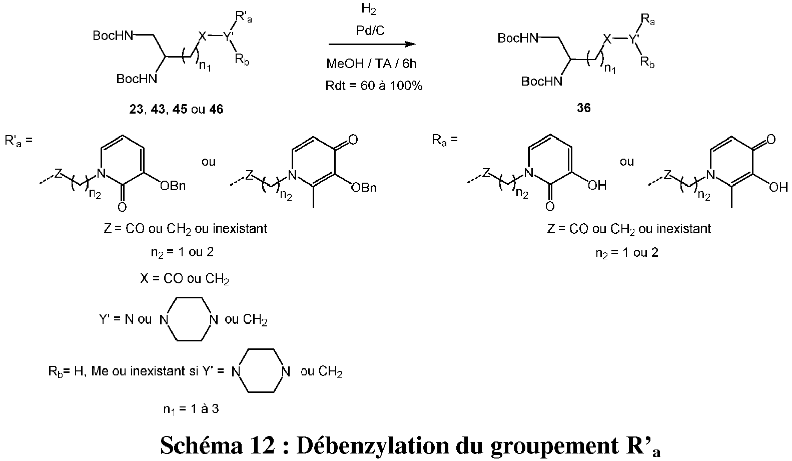

À une solution du composé **23a-k, 43, 45** ou **46** (0,2-1,6 mmol) dans du méthanol (5-40 mL), est additionné du Pd/C (10% m/m). Le milieu réactionnel est placé sous vide et sous agitation à température ambiante pendant 30 min, puis maintenu sous un flux d'H₂ pendant 6h. Il est ensuite filtré sur papier et le méthanol est évaporé sous pression réduite pour donner le dérivé **36a-n** (60 à 100%) (Tableau 3).

**Tableau 3 : Composés 36**

| **Composé** | **n₁** | **X** | **Y'** | **R'ₐ** | **R_{b}** | **Rdt (%)** | **Aspect** |
|---|---|---|---|---|---|---|---|
| **36a** | 2 | CH₂ | N | 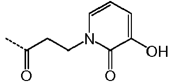 | H | 100 | Poudre blanche |
| **36b** | 3 | CH₂ | N | 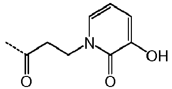 | H | 87 | Poudre jaune |
| **36c** | 2 | CO | N | 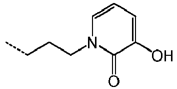 | H | 100 | Huile brune |
| **36d** | 1 | CO | 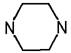 | 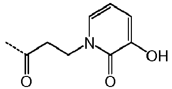 | Inexistant | 100 | Poudre brune |
| **36e** | 2 | CO | 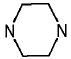 | 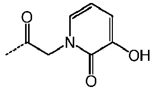 | Inexistant | 95 | Poudre blanche |
| **36f** | 2 | CO | 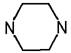 | 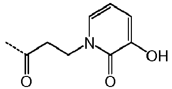 | Inexistant | 82 | Poudre brune |
| **36g** | 2 | CH₂ | N | 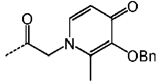 | H | 78 | Poudre blanche |
| **36h** | 3 | CH₂ | N | 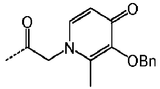 | H | 71 | Poudre blanche |
| **36i** | 3 | CH₂ | N | 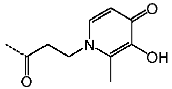 | H | 100 | Huile orange |
| **36j** | 2 | CO | N | 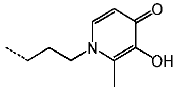 | H | 60 | Poudre blanche |
| **36k** | 2 | CO | 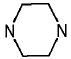 | 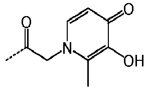 | Inexistant | 100 | Poudre blanche |
| **36l** | 2 | CH₂ | 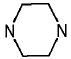 | 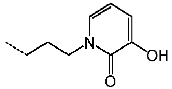 | Inexistant | 94 | Poudre brune |
| **36m** | 2 | CH₂ | N | 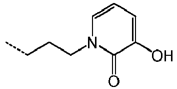 | CH₃ | 96 | Poudre brune |
| **36n** | 1 | CH₂ | CH₂ | 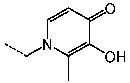 | Inexistant | 91 | Poudre brune |

**Composé 36a :** ¹H RMN (CDCl₃, 600 MHz): *δ* (ppm) 6,95 (d, *J* = 6,7 Hz, 1H); 6,92 (s, 1H); 6,82 (d, *J* = 7,2 Hz, 1H); 6,14 (t, *J* = 7,1 Hz, 1H); 5,18 (s, 1H); 5,04-5,03 (s, 1H); 4,25 (m, 2H); 3,56-3,54 (m, 1H); 3,17-3,16 (m, 2H); 3,09 (m, 2H); 2,68 (t, *J* = 6,4 Hz, 2H); 1,50-1,40 (m, 22H).
¹³C RMN (CDCl₃, 150 MHz): *δ* (ppm) 170,3 ; 158,7; 157,0; 156,7; 146,7; 128,6; 115,2; 107,2; 79,7 (2C); 50,8; 47,3; 44,8; 39,5; 35,5; 29,9; 28,6 (6C); 25,7.
MS (ESI +): m/z = [M + H] 483,3; [M + Na] 505,3.
HRMS (ESI +): m/z calculée pour C₂₃H₃₈N₄O₇Na [M + Na] = 505,2638; trouvée = 505,2644.
**Composé 36b** : ¹H RMN (CDCl₃, 300 MHz): *δ* (ppm) 6,96 (dd, *J₁* = 6,9 Hz et *J₂* = 1.5 Hz, 1H); 6,80 (dd, *J₁* = 7,4 Hz et *J₂* = 1.5 Hz, 1H); 6,52-6,51 (s, 1H); 6,12 (t, *J* = 7,2 Hz, 1H); 5,04 (s, 1H); 4,88-4,86 (s, 1H); 4,26 (t, *J* = 6,3 Hz, 2H); 3,55-3,53 (m, 1H); 3,13-3,08 (m, 4H); 2,68 (t, *J* = 6,3 Hz, 2H); 1,79-1,50 (m, 2H); 1,40 (s, 18H); 1,35-1,23 (m, 4H).
¹³C RMN (CDCl₃, 75 MHz): *δ* (ppm) 169,7; 158,3 (2C); 146,2; 128,2; 114,6; 106,7; 79,5 (2C); 51,1; 46,9; 44,3; 38,8; 35,1; 31,9; 28,8; 28,2 (6C); 22,6.
MS (ESI +): m/z = [M - 2Boc + 3H] 296,9; [M - Boc + 2H] 397,0; [M + H] 497,1; [M + Na] 519,1.
HRMS (ESI +): m/z calculée pour C₂₄H₄₀N₄O₇Na [M + Na] = 519,2795; trouvée = 519,2801.
**Composé 36c** : ¹H RMN (CDCl₃, 300 MHz): *δ* (ppm) 7,34 (s, 1H); 6,89 (dd, *J₁* = 6,9 Hz et *J₂* = 1,2 Hz, 1H); 6,83 (d, *J* = 7,2 Hz, 1H); 6,19 (t, *J* = 6,9 Hz, 1H); 5,33-5,23 (s, 2H); 4,06 (t, *J* = 5,2 Hz, 2H); 3,67-3,64 (m, 1H); 3,28-3,17 (m, 4H); 2,30 (t, *J* = 7,2 Hz, 2H); 1,93 (t, *J* = 6,3 Hz, 2H); 1,95-1,60 (m, 2H); 1,40 (s, 18H).
¹³C RMN (CDCl₃, 75 MHz): *δ* (ppm) 173,3; 172,4; 158,6 ; 156,8; 146,5; 127,0; 114,6; 107,6; 79,4 (2C); 51,2; 47,1; 44,5; 35,9; 33,0; 29,3; 28,2 (6C); 25,4.
MS (ESI +): m/z = [M + H] 483,4; [M + Na] 505,3.
HRMS (ESI +): m/z calculée pour C₂₃H₃₈N₄O₇Na [M + Na] = 505,2638; trouvée = 505,2652.
**Composé 36d :** ¹H RMN (CDCl₃, 600 MHz): *δ* (ppm) 7,03-7,01 (m, 1H); 6,80 (d, *J* = 7,3 Hz, 1H); 6,10 (t, *J* = 7,1 Hz, 1H); 5,66 (s, 1H); 4,98 (s, 1H); 4,27 (t, *J* = 7,1 Hz, 2H); 3,89 (m, 1H); 3,66-3,31 (m, 8H); 3,23-3,21 (m, 2H); 2,87-2,85 (m, 2H); 2,67-2,46 (m, 2H); 1,39 (s, 18H).
¹³C RMN (CDCl₃, 150 MHz): *δ* (ppm) 169,5; 169,0; 158,6; 157,0; 156,1; 146,5; 128,9; 115,1; 107,0; 79,8; 79,7; 49,5; 47,3; 45,8 (2C); 43,9; 41,8 (2C); 35,4; 32,1; 28,6 (6C).
MS (ESI +): m/z = [M - 2Boc + 3H] 352,1; [M - Boc + 2H] 452,2; [M + H] 552,2; [M + Na] 574,2.
HRMS (ESI +): m/z calculée pour C₂₆H₄₁N₅O₈Na [M + Na] = 574,2853; trouvée = 574,2849.
**Composé 36e** : ¹H RMN (CDCl₃, 400 MHz): *δ* (ppm) 6,80 (d, *J* = 6,4 Hz, 2H); 6,18 (t, *J* = 7,1 Hz, 1H); 5,06-4,99 (s, 2H); 4,85-4,71 (m, 2H); 3,86-3,38 (m, 9H); 3,21-3,15 (m, 2H); 2,46-2,32 (m, 2H); 1,92-1,64 (m, 2H); 1,40 (s, 18H).
¹³C RMN (CDCl₃, 100 MHz): *δ* (ppm) 171,6; 165,4; 158,9; 157,2; 156,6; 146,7; 128,1; 114,9; 107,2; 79,8 (2C); 51,6; 49,9; 45,2; 44,8; 42,5; 41,8; 41,5; 30,0; 28,6 (6C); 28,2. MS (ESI +): m/z = [M + H] 552,3; [M + Na] 574,3.
HRMS (ESI +): m/z calculée pour C₂₆H₄₁N₅O₈Na [M + Na] = 574,2853; trouvée = 574,2832.
**Composé 36f :** ¹H RMN (CDCl₃, 400 MHz): *δ* (ppm) 7,01-7,00 (m, 1H); 7,76 (d, *J* = 7,1 Hz, 1H); 6,09 (t, *J* = 6,9 Hz, 1H); 5,04 (s, 2H); 4,23 (t, *J* = 5,9 Hz, 2H); 3,67-3,36 (m, 9H); 3,12 (m, 2H); 2,83 (m, 2H); 2,36-2,30 (m, 2H); 1,80-1,62 (m, 2H); 1,35 (s, 18H).
¹³C RMN (CDCl₃, 100 MHz): *δ* (ppm) 171,3; 169,0; 158,6; 156,7; 156,4; 146,5; 128,6; 114,8; 106,7; 79,3 (2C); 51,4; 47,1; 45,2; 44,4 (2C); 41,6 (2C); 31,7; 29,4; 28,4 (6C); 27,7. MS (ESI +): m/z = [M + H] 566,3; [M + Na] 588,3.
HRMS (ESI +): m/z calculée pour C₂₇H₄₃N₅O₈Na [M + Na] = 588,3009; trouvée = 588,2980.
**Composé 36g** : ¹H RMN (CDCl₃, 400 MHz): *δ* (ppm) 7,49 (d, *J* = 4,8 Hz, 1H); 6,33 (d, *J* = 4,8 Hz, 1H); 4,70 (s, 2H); 3,53-3,51 (m, 1H); 3,19 - 3,17 (m, 2H); 3,07-2,91 (m, 2H); 2,26 (s, 3H); 1,56-1,44 (m, 4H); 1,42 (s, 18H).
¹³C RMN (CDCl₃, 100 MHz): *δ* (ppm) 169,9; 167,1; 157,3; 157,0; 145,5; 139,0; 131,9; 111,1; 78,6 (2C); 55,5; 50,5; 44,1; 39,2; 29,6; 27,4 (6C); 25,4; 10.7.
MS (ESI +): m/z = [M + H] 483,2.
HRMS (ESI +): m/z calculée pour C₂₃H₃₈N₄O₇Na [M + Na] = 505,2638; trouvée = 505,2636.
**Composé 36h** : ¹H RMN (CDCl₃, 400 MHz): *δ* (ppm) 7,57 (d, *J* = 4,8 Hz, 1H); 6,41 (d, *J* = 4,8 Hz, 1H); 4,77 (s, 2H); 3,57 (m, 1H); 3,14-3,11 (m, 2H); 3,02-2,98 (m, 2H); 2,34 (s, 3H); 1,58-1,49 (m, 2H); 1,40 (s, 20H); 1,39-1,35 (m, 2H).
¹³C RMN (CDCl₃, 100 MHz): *δ* (ppm) 169,9; 167,1; 157,3; 157,0; 145,5; 139,0; 131,9; 111,1; 78,6 (2C); 55,5; 50,7; 44,0; 39,1; 31,6; 28,7; 27,4 (6C); 22,9; 10.7.
MS (ESI +): m/z = [M + H] 497,3; [M + Na] 519,2.
HRMS (ESI +): m/z calculée pour C₂₄H₄₀N₄O₇Na [M + Na] = 519,2795; trouvée = 519,2789.
**Composé 36i :** ¹H RMN (CDCl₃, 400 MHz): *δ* (ppm) 7,56 (d, *J* = 7,2 Hz, 1H); 6,37 (d, *J* = 7,2 Hz, 1H); 4,37-4,33 (m, 2H); 3,53 (m, 1H); 3,13-3,07 (m, 2H); 3,00-2,95 (m, 2H); 2,64 (t, *J* = 6,5 Hz, 2H); 2,46 (s, 3H); 1,44 (s, 18H); 1,44-1,40 (m, 4H); 1,40-1,23 (m, 2H).
¹³C RMN (CDCl₃, 100 MHz): *δ* (ppm) 171,5; 170,8; 158,5; 158,2; 147,1; 139,0; 132,5; 112,5; 80,0 (2C); 51,4; 50,3; 45,4; 40,2; 37,6; 32,9; 30,0; 28,8 (6C); 24,3; 11,8.
MS (ESI +): m/z = [M + H] 511,3; [M + Na] 533,2.
HRMS (ESI +): m/z calculée pour C₂₅H₄₂N₄O₇Na [M + Na] = 533.2951; trouvée = 533.2935.
**Composé 36j** : ¹H RMN (CDCl₃, 400 MHz): *δ* (ppm) 7,39 (m, 2H); 6,31 (m, 1H); 5,40-5,33 (s, 2H); 3,95 (m, 2H); 3,55 (m, 1H); 3,40-3,13 (m, 4H); 2,35 (s, 3H); 2,24 (m, 2H); 1,96-1,60 (m, 4H), 1,40 (s, 18H).
¹³C RMN (CDCl₃, 100 MHz): *δ* (ppm) 174,2; 169,8; 157,7 (2C); 146,8; 137,5; 129,3; 111,6; 80,2 (2C); 52,2; 51,6; 44,9; 36,8; 33,5; 31,4; 30,2; 28,7 (6C); 12.1.
MS (ESI +): m/z = [M + H] 497,2; [M + Na] 519,3.
HRMS (ESI +): m/z calculée pour C₂₄H₄₀N₄O₇Na [M + Na] = 519,2795; trouvée = 519,2795.
**Composé 36k** : ¹H RMN (CDCl₃, 400 MHz): *δ* (ppm) 7,26 (m, 1H); 6,35 (m, 1H); 5,98 (s, 2H); 5,13 (m, 2H); 4,88 (m, 1H); 3,67-3,66 (m, 8H); 3,14 (m, 2H); 2,35 (m, 2H); 2,17 (s, 3H); 1,80-1,63 (m, 2H), 1,40 (s, 18H).
¹³C RMN (CDCl₃, 100 MHz): *δ* (ppm) 171,6; 168,9; 164,5; 156,9 (2C); 142,8; 138,6; 128,5; 111,5; 79,6 (2C); 55,0; 50,7; 45,2; 44,9; 44,6; 42,1; 41,5; 29,9; 28,4 (6C); 28,1; 12,2.
MS (ESI +): m/z = [M + H] 566,3; [M + Na] 588,3.
HRMS (ESI +): m/z calculée pour C₂₇H₄₃N₅O₈Na [M + Na] = 588,3009; trouvée = 588,3002.
**Composé 36l:** ¹H RMN (CDCl₃, 400 MHz): *δ* (ppm) 6,83 (d, *J* = 6,8 Hz, 1H); 6,76 (d, *J* = 7,3 Hz, 1H); 6,11 (t, *J* = 7,1 Hz, 1H); 5,17-5,08 (s, 2H); 4,02 (t, *J* = 6,9 Hz, 2H); 3,56 (m, 1H); 3,15-3,12 (m, 4H); 2,91-2,83 (m, 6H); 2,57-2,56 (m, 2H); 2,12 (m, 2H); 1,80-1,78 (m, 2H); 1,37 (m, 22H).
¹³C RMN (CDCl₃, 100 MHz): *δ* (ppm) 159,0; 157,2; 156,3; 147,0; 127,4; 114,3; 107,1; 79,7 (2C); 56,9; 54,4; 51,1; 50,2 (2C); 48,4; 44,7 (2C); 30,1; 28,6 (6C); 27,3; 25,5; 21,2. MS (ESI +): m/z = [M + H] 538,3.
HRMS (ESI +): m/z calculée pour C₂₇H₄₈N₅O₆ [M + H] = 538,3605; trouvée = 538,3611.
**Composé 36m :** ¹H RMN (CDCl₃, 400 MHz): *δ* (ppm) 6,85 (d, *J* = 6,6 Hz, 1H); 6,77 (dd, *J₁* = 7,3 Hz et *J₂* = 1,5Hz, 1H); 6,12 (t, *J* = 7,1 Hz, 1H); 5,07 (s, 2H); 4,02-3,88 (m, 2H); 3,59 (m, 1H); 3,16 (m, 2H); 2,34-2,32 (m, 4H); 2,16 (s, 3H); 1,90 (t, *J* = 6,6 Hz, 2H); 1,51 (m, 4H); 1,41 (s, 18H).
¹³C RMN (CDCl₃, 100 MHz): *δ* (ppm) 159,3; 156,9 (2C); 147,1; 127,6; 113,7; 106,8; 79,5 (2C); 57,5; 54,5; 51,4; 48,3; 45,1; 42,0; 31,4; 28,6 (6C); 26,8; 24,4.
MS (ESI +): m/z = [M + H] 483,4; [M + Na] 505,3.
HRMS (ESI +): m/z calculée pour C₂₄H₄₃N₄O₆ [M +] = 483,3183; trouvée = 483,3172. **Composé 36n :** ¹H RMN (CDCl₃, 400 MHz): *δ* (ppm) 7,86 (m, 1H); 7,03-7,02 (m, 2H); 5,19-5,10 (m, 2H); 4,19 (m, 2H); 3,59 (m, 1H); 3,13 (m, 2H); 2,51 (s, 3H); 1,84-1,78 (m, 2H); 1,39-1,38 (m, 22H).
¹³C RMN (CDCl₃, 100 MHz): *δ* (ppm) 161,5; 157,1; 156,6; 144,5; 138,4; 137,5; 112,3; 79,6 (2C); 56,3; 51,2; 44,6; 32,3; 30,3; 28,6 (6C); 22,8; 12,8.
MS (ESI +): m/z = [M + H] 440,3.
HRMS (ESI +): m/z calculée pour C₂₂H₃₈N₃O₆ [M + H] = 440,2761; trouvée = 440,2749.

### δ - Déprotection finale du groupement diamine (Schéma 13)

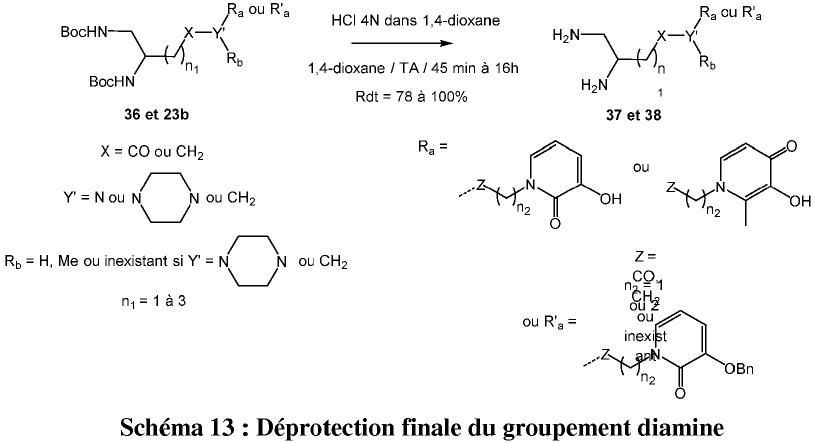

Le composé **36a-n** ou **23b** (0,2-1,7 mmol) est dissous dans du 1,4-dioxane (4-10 mL) en présence d'une solution commerciale de HCl 4N dans du 1,4-dioxane (20 eq). Le milieu réactionnel est alors placé sous agitation à température ambiante pendant 45 min à 16h. Le dioxane est évaporé sous pression réduite et le résidu est trituré dans de l'éther. Après évaporation sous vide (produits très hygroscopiques), le précipité obtenu est lyophilisé pour donner le dérivé **37a-n** ou **38** (78 à 100%) (Tableau 4).

Les composés 37d, 37f, 37i, 37j et 38 sont des composés de référence et ne font pas partie de l'invention.

**Tableau 4 : Composés 37 et 38**

| **Composé** | **n₁** | **X** | **Y'** | **R'ₐ** | **R_{b}** | **Rdt (%)** | **Aspect** |
|---|---|---|---|---|---|---|---|
| **37a** | 2 | CH₂ | N | 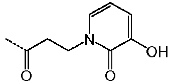 | H | 100 | Poudre jaune |
| **37b** | 3 | CH₂ | N | 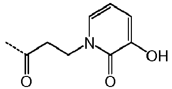 | H | 100 | Poudre jaune |
| **37c** | 2 | CO | N | 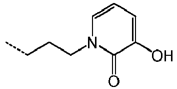 | H | 93 | Poudre blanche |
| **37d** | 1 | CO | 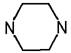 | 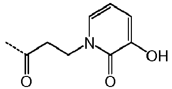 | Inexistant | 88 | Poudre blanche |
| **37e** | 2 | CO | 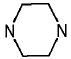 | 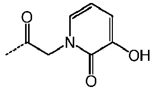 | Inexistant | 100 | Poudre beige |
| **37f** | 2 | CO | 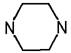 | 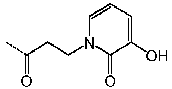 | Inexistant | 100 | Poudre jaune |
| **37g** | 2 | CH₂ | N | 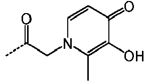 | H | 100 | Poudre blanche |
| **37h** | 3 | CH₂ | N | 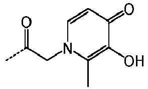 | H | 100 | Poudre blanche |
| **37i** | 3 | CH₂ | N | 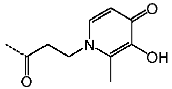 | H | 100 | Poudre blanche |
| **37j** | 2 | CO | N | 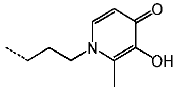 | H | 100 | Poudre blanche |
| **37k** | 2 | CO | 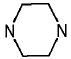 | 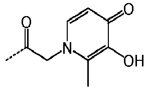 | Inexistant | 100 | Poudre blanche |
| **37l** | 2 | CH₂ | 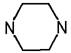 | 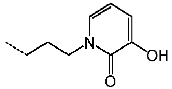 | Inexistant | 78 | Poudre blanche |
| **37m** | 2 | CH₂ | N | 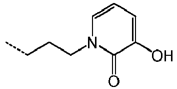 | CH₃ | 87 | Poudre blanche |
| **37n** | 1 | CH₂ | CH₂ | 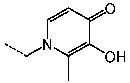 | Inexistant | 86 | Poudre blanche |
| **38** | 3 | CH₂ | N | 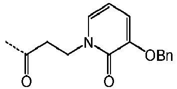 | H | 100 | Poudre orangée |

**Composé 37a** : ¹H RMN (D₂O, 400 MHz): *δ* (ppm) 7,16 (dd, *J₁* = 6,8 Hz et *J₂* = 1,4 Hz, 1H); 7,05 (dd, *J₁* = 7,5 Hz et *J₂* = 1,4 Hz, 1H); 6,41 (t, *J* = 7,1 Hz, 1H); 4,30 (t, *J* = 6,4 Hz, 2H); 3,67-3,64 (m, 1H); 3,35-3,34 (m, 2H); 3,18 (t, *J* = 6,7 Hz, 2H); 2,72 (t, *J* = 6,4 Hz, 2H); 1,76-1,57 (m, 4H).
¹³C RMN (D₂O, 100 MHz): *δ* (ppm) 173,3; 158,5; 145,5; 129,4; 118,8; 108,5; 49,0; 47,4; 40,9; 38,7; 35,3; 27,5; 24,1.
MS (ESI +): m/z = [M + H] 283,1.
HRMS (ESI +): m/z calculée pour C₁₃H₂₃N₄O₃ [M + H] = 283,1770; trouvée = 283.1765.
**Composé 37b :** ¹H RMN (d₆-DMSO, 300 MHz): *δ* (ppm) 8,59 (s, 4H); 8,10-8,07 (s, 1H); 7,08 (dd, *J₁* = 6,9 Hz et *J₂* = 1,5 Hz, 1H); 6,70 (dd, *J₁* = 7,2 Hz et *J₂* = 1,5 Hz, 1H); 6,07 (t, *J* = 7,2 Hz, 1H); 4,10 (t, *J* = 6,9 Hz, 2H); 3,40-3,38 (m, 1H); 3,09-3,00 (m, 4H); 2,53-2,49 (m, 2H); 1,63-1,58 (m, 2H); 1,19 (m, 4H).
¹³C RMN (d₆-DMSO, 75 MHz): *δ* (ppm) 169,6; 157,8; 146,8; 128,6; 115,0; 105,4; 49,1; 45,9; 40,6; 38,2; 34,8; 29,7; 28,6; 21,9.
MS (ESI +): m/z = [M + H] 296,9.
HRMS (ESI +): m/z calculée pour C₁₄H₂₅N₄O₃ [M + H] = 297,1927; trouvée = 297.1914.
**Composé 37c :** ¹H RMN (d₆-DMSO, 300 MHz): *δ* (ppm) 8,63 (s, 2H); 8,54 (s, 2H); 8,30 (s, 1H); 7,20 (dd, *J₁* = 6,8 Hz et *J₂* = 1,5 Hz, 1H); 6,72 (dd, *J₁* = 7,2 Hz et *J₂* = 1,8 Hz, 1H); 6,11 (t, *J* = 6,9 Hz, 1H); 3,96-3,87 (m, 2H); 3,49-3,46 (m, 1H); 3,11 - 3,04 (m, 4H); 2,32-2,31 (m, 2H); 1,90-1,75 (m, 4H).
¹³C RMN (d₆-DMSO, 75 MHz): *δ* (ppm) 171,2; 157,6; 146,6; 128,2; 114,8; 105,5; 48,7; 46,6; 40,3; 35,8; 30,8; 28,7; 26,1.
MS (ESI +): m/z = [M + H] 283,0.
HRMS (ESI +): m/z calculée pour C₁₃H₂₃N₄O₃ [M + H] = 283,1810; trouvée = 283,1800. **Composé 37d** : ¹H RMN (D₂O, 400 MHz): *δ* (ppm) 7,20 (d, *J* = 6,4 Hz, 1H); 7,03 (d, *J* = 6,6 Hz, 1H); 6,39 (t, *J* = 6,9 Hz, 1H); 4,32-4,28 (m, 1H); 4,06-4,02 (m, 2H); 3,69-3,45 (m, 8H); 3,17-3,10 (m, 2H); 3,04-3,01 (m, 2H); 2,99-2,95 (m, 2H).
¹³C RMN (D₂O, 100 MHz): *δ* (ppm) 172,8; 171,5; 168,8; 158,5; 129,5; 118,9; 108,3; 47,1; 45,5; 45,3; 44,7; 44,3; 41,5; 40,7; 33,2; 31,5.
MS (ESI +): m/z = [M + H] 352,2.
HRMS (ESI +): m/z calculée pour C₁₆H₂₆N₅O₄ [M + H] = 352,1985; trouvée = 352,1977. **Composé 37e** : ¹H RMN (D₂O, 400 MHz): *δ* (ppm) 7,14-7,08 (m, 2H); 6,44 (m, 1H); 5,01 (s, 2H); 3,94-3,85 (m, 1H); 3,66 (m, 8H); 3,17 (m, 2H); 2,78-2,76 (m, 2H); 2,13-2,08 (m, 2H).
¹³C RMN (D₂O, 100 MHz): *δ* (ppm) 172,5; 167,2; 158,7; 145,4; 130,1; 119,2; 108,3; 51,2; 49,2; 44,6; 44,1; 42,9; 41,7; 41,3; 28,5; 25,3.
MS (ESI +): m/z = [M + H] 352,2.
HRMS (ESI +): m/z calculée pour C₁₆H₂₆N₅O₄ [M + H] = 352,1985; trouvée = 352,1981. **Composé 37f :** ¹H RMN (D₂O, 400 MHz): *δ* (ppm) 7,20 (d, *J* = 6,3 Hz, 1H); 7,03 (d, *J* = 6,4 Hz, 1H); 6,39 (t, *J* = 6,8 Hz, 1H); 4,30 (t, *J* = 6,3 Hz, 2H); 3,75-3,60 (m, 4H); 3,55-3,52 (m, 5H); 3,37-3,35 (m, 2H); 2,98-2,95 (m, 2H); 2,74-2,68 (m, 2H); 2,11-2,03 (m, 2H).
¹³C RMN (D₂O, 100 MHz): *δ* (ppm) 172,7; 161,7; 158,8; 145,3; 129,5; 118,6; 108,3; 49,0; 47,0; 45,1; 44,4; 41,5; 41,2; 40,6; 31,4; 28,3; 25,2.
MS (ESI +): m/z = [M + H] 366,2.
HRMS (ESI +): m/z calculée pour C₁₇H₂₈N₅O₄ [M + H] = 366,2146; trouvée = 366,2134. **Composé 37g :** ¹H RMN (CD₃OD, 400 MHz): *δ* (ppm) 8,22 (d, *J* = 5,8 Hz, 1H); 7,16 (d, *J* = 5,8 Hz, 1H); 5,29 (s, 2H); 3,66-3,61 (m, 1H); 3,35-3,31 (m, 4H); 2,53 (s, 3H); 1,90-1,73 (m, 4H).
¹³C RMN (CD₃OD, 100 MHz): *δ* (ppm) 167,1; 160,7; 145,0; 144,3; 141,2; 111,5; 59,4; 51,0; 42,4; 40,1; 29,1; 26,2; 13.3.
MS (ESI +): m/z = [M + H] 283,2.
HRMS (ESI +): m/z calculée pour C₁₃H₂₃N₄O₃ [M + H] = 283,1770; trouvée = 283,1772. **Composé 37h** : ¹H RMN (CD₃OD, 400 MHz): *δ* (ppm) 8,15 (d, *J* = 6,8 Hz, 1H); 7,12 (d, *J* = 6,6 Hz, 1H); 5,22 (s, 2H); 3,58-3,56 (m, 1H); 3,29-3,25 (m, 4H); 2,48 (s, 3H); 1,81-1,73 (m, 2H); 1,62-1,49 (m, 4H).
¹³C RMN (CD₃OD, 100 MHz): *δ* (ppm) 166,8; 160,4; 144,8; 143,9; 140,8; 111,4; 59,1; 50,9; 42,9; 40,3; 31,0; 29,7; 23,2; 12.9.
MS (ESI +): m/z = [M + H] 297,2; [M + Na] 319,2.
HRMS (ESI +): m/z calculée pour C₁₄H₂₅N₄O₃ [M + H] = 297,1927; trouvée = 297,1913.
**Composé 37i** : ¹H RMN (D₂O, 400 MHz): *δ* (ppm) 7,97 (d, *J* = 6,6 Hz, 1H); 7,18 (d, *J* = 7,0 Hz, 1H); 4,68 (t, *J* = 6,4 Hz, 2H); 3,71-3,65 (m, 1H); 3,38-3,37 (m, 2H); 3,16 (t, *J* = 6,7 Hz, 2H); 3,08-3,04 (s, 1H); 2,88 (t, *J* = 6,5 Hz, 2H); 2,56 (s, 3H) ; 1,85-1,74 (m, 2H); 1,52-1,38 (m, 4H).
¹³C RMN (D₂O, 100 MHz): *δ* (ppm) 171,3 (2C); 158,2; 142,4; 138,7; 110,9; 52,7; 49,2; 40,8; 38,9; 35,8; 29,6; 27,9; 21,6; 12,3.
MS (ESI +): m/z = [M + H] 311,2.
HRMS (ESI +): m/z calculée pour C₁₅H₂₇N₄O₃ [M + H] = 311.2083; trouvée = 311.2075. **Composé 37j** : ¹H RMN (D₂O, 400 MHz): *δ* (ppm) 8,28 (d, *J* = 6,5 Hz, 1H); 7,13 (d, *J* = 6,5 Hz, 1H); 4,44 (m, 2H); 3,33-3,29 (m, 5H); 2,64 (s, 3H); 2,57 (m, 2H); 2,08-2,07 (m, 4H).
¹³C RMN (D₂O, 100 MHz): *δ* (ppm) 174,9; 159,8; 145,2; 143,4; 139,4; 111,7; 55,7; 50,6; 41,9; 37,3; 32,2; 31,0; 27,1; 12,8.
MS (ESI +): m/z = [M + H] 297,2.
HRMS (ESI +): m/z calculée pour C₁₄H₂₅N₄O₃ [M + H] = 297,1927; trouvée = 297,1931. **Composé 37k** : ¹H RMN (D₂O, 400 MHz): *δ* (ppm) 8,01 (d, *J* = 6,8 Hz, 1H); 7,19 (d, *J* = 6,9 Hz, 1H); 5,53 (s, 2H); 3,72-3,55 (m, 9H); 3,41-3,39 (m, 2H); 2,81-2.75 (m, 2H); 2,47 (s, 3H); 2,16-2,08 (m, 2H).
¹³C RMN (D₂O, 100 MHz): *δ* (ppm) 172,4; 165,2; 159,4; 143,4; 142,5; 139,9; 111,1; 57,2; 49,1; 44,5; 44,1; 42,2; 41,4; 40,7; 28,5; 25,3; 12,5.
MS (ESI +): m/z = [M + H] 366,2.
HRMS (ESI +): m/z calculée pour C₁₇H₂₈N₅O₄ [M + H] = 366,2141; trouvée = 366,2133.
**Composé 37l :** ¹H RMN (d₆-DMSO, 400 MHz): *δ* (ppm) 6,83 (d, *J* = 6,8 Hz, 1H); 6,76 (d, *J* = 7,3 Hz, 1H); 6,11 (t, *J* = 7,1 Hz, 1H); 5,17-5,08 (s, 2H); 4,02 (t, *J* = 6,9 Hz, 2H); 3,56 (m, 1H); 3,15-3,12 (m, 4H); 2,91-2,83 (m, 6H); 2,57-2,56 (m, 2H); 2,12 (m, 2H); 1,80-1,78 (m, 2H); 1,37 (m, 22H).
¹³C RMN (d₆-DMSO, 100 MHz): *δ* (ppm) 159,0; 157,2; 156,3; 147,0; 127,4; 114,3; 107,1; 79,7 (2C); 56,9; 54,4; 51,1; 50,2 (2C); 48,4; 44,7 (2C); 30,1; 28,6 (6C); 27,3; 25,5; 21,2. MS (ESI +): m/z = [M + H] 338,3.
HRMS (ESI +): m/z calculée pour C₁₇H₃₂N₅O₂ [M + H] = 338,2556; trouvée = 338,2552. **Composé 37m :** ¹H RMN (CD₃OD, 400 MHz): *δ* (ppm) 7,49 (m, 1H); 7,14 (m, 1H); 6,60-6,59 (m, 1H); 4,34-4,30 (m, 2H); 3,82-3,78 (m, 1H); 3,40-3,34 (m, 4H); 3,31-3,26 (m, 2H); 2,95 (s, 3H); 2,35-2,31 (m, 2H); 2,03-1,91 (m, 4H).
¹³C RMN (CD₃OD, 100 MHz): *δ* (ppm) 158,8; 147,8; 130,1; 121,2; 112,2; 56,8; 54,6; 50,3; 49,8; 42,1; 40,9; 28,5; 25,4; 21,2.
MS (ESI +): m/z = [M + H] 283,2.
HRMS (ESI +): m/z calculée pour C₁₄H₂₇N₄O₂ [M +] = 283,2134; trouvée = 283,2133. **Composé 37n** : ¹H RMN (CD₃OD, 400 MHz): *δ* (ppm) 8,25 (d, *J* = 7,0 Hz, 1H); 7,13 (d, *J* = 6,9 Hz, 1H); 4,42 (t, *J* = 7,6 Hz, 2H); 3,64-3,61 (m, 1H); 3,31-3,27 (m, 2H); 2,64 (s, 3H); 1,93-1,82 (m, 4H); 1,57-1,56 (m, 2H).
¹³C RMN (CD₃OD, 100 MHz): *δ* (ppm) 159,5; 145,0; 143,1; 139,6; 111,8; 57,4; 50,6; 42,0; 31,0; 30,7; 22,7; 12,7.
MS (ESI +): m/z = [M + H] 240,2.
HRMS (ESI +): m/z calculée pour C₁₂H₂₂N₃O₂ [M + H] = 240,1712; trouvée = 240,1713. **Composé 38 :** ¹H RMN (CD₃OD, 400 MHz): *δ* (ppm) 7,46 (s, 2H); 7,37-7,27 (m, 3H); 6,58 (m, 1H); 5,21 (m, 2H); 4,40 (m, 2H); 3,62 (m, 1H); 3,32-3,31 (m, 2H); 3,19 (m, 2H); 2,77 (m, 2H); 1,80 (m, 2H); 1,53 (m, 4H).
¹³C RMN (CD₃OD, 100 MHz): *δ* (ppm) 171,4;157,1; 147,2; 135,7; 130,3; 128,6 (2C); 128,2; 127,8 (2C); 119,3; 109,5; 71,0; 49,6; 40,8; 38,5; 34,7; 32,4; 29,7; 28,3; 21,8.
MS (ESI +): m/z = [M + H] 387,2.
HRMS (ESI +): m/z calculée pour C₂₁H₃₁N₄O₃ [M + H] = 387,2396; trouvée = 387,2382.

### B. ÉTUDES PHYSICOCHIMIQUES

### I) Évaluation de la formation d'adduits entre les composés selon l'invention (29a, 29b, 30a, 30b ; 37a, 37b, 37c, 37d, 37f, 37i, 37j) et des α-oxoaldéhydes ou des aldéhydes α,β-insaturés par analyse HPLC

### 1- Principe

Les composés à tester sont mis à incuber à 37°C en présence de MGO ou de MDA. Une étude cinétique visant à rendre compte de la formation d'adduits avec le MGO ou le MDA est alors réalisée par analyse HPLC.

### 2- Méthode

### a) Préparation des solutions

Les composés à tester (30 µmol) sont dissous dans du PBS (1,5 mL). Une solution aqueuse (qsp 1,25 mL) de MGO à 40% dans l'eau (250 µmol) est ensuite préparée et en parallèle, du MDA bis-(diOEt)-acétal (250 µmol) est mis à réagir avec une solution de HCl 1N (2 eq) dans l'eau (qsp 1,25 mL) pendant 1h à température ambiante. Une solution aqueuse de NaOH 0,05N est également nécessaire pour neutraliser le milieu réactionnel (5 mL).

### b) Incubation des mélanges à 37°C

La solution de composé à tester (625 µL - concentration finale dans le milieu = 10 mM) est ensuite mise à réagir avec la solution de MGO ou de MDA préparé extemporanément (125 µL - concentration finale dans le milieu = 20 mM) en présence de NaOH 0,05 N (0,5 mL).

Les différents mélanges sont ensuite mis à incuber à l'étuve à 37°C pendant 24h.

### c) Analyse par HPLC

Un prélèvement de chaque mélange (100 µL) est réalisé à intervalles de temps réguliers (0,25; 0,5; 1; 5 et 24h) et stocké à -20°C pour arrêter la réaction. Après dilution dans un mélange MeCN/H₂O 98:2 à température ambiante, l'analyse HPLC est réalisée sur un appareil Shimadzu LCMS-2020 (chromatogramme UV à 190 nm et spectre de masse en mode d'ionisation électrospray positif (ESI +)) après séparation sur une colonne Waters Acquity à l'aide d'un gradient de solvants H₂O + HCOOH 0,1% / MeCN + HCOOH 0,1% (98/2 pendant 2 min, puis 55/45 pendant 2 min et 45/55 pendant 3 min) avec un débit de 0,3 mL/min à 40°C et un volume d'injection de 1 µL (« détection mode: scan, interface voltage: tuning file, DL voltage: 100 V, Q-array DC: 40 V, Q-array RF: 40 V»). On réalise tout d'abord un blanc à l'aide d'une solution contenant le milieu réactionnel sans piégeur. Puis, on utilise une solution de piégeur libre à 10 mM comme témoin négatif et la carnosine comme référence de la littérature. La proportion d'adduits formés avec le MGO ou le MDA (généralement, t_{R} = 4,1 à 6,8 min) est ensuite mesurée par rapport à la quantité de piégeur libre restant (généralement, t_{R} = 0,8 à 4,6 min) après mesure de l'aire sous la courbe des pics correspondants sur le spectre UV (mesures réalisées pour un échantillon représentatif).

### 3- Résultats et discussion

### a) Évaluation de la formation d'adduits avec le MGO (Figure 2)

Tout d'abord, on observe une disparition du piégeur libre au profit des différents adduits avec le MGO beaucoup plus rapide avec les nouveaux composés selon l'invention qu'avec les dérivés de 2^{ème} génération (Dap-Pip et Dap-(nBu)Pip) ou la carnosine (Figure 2). L'efficacité accrue de cette nouvelle série de dérivés diaminés en tant que piégeurs de MGO, liée à l'éloignement du groupement carbonyle par rapport à la fonction diamine, a donc pu être démontrée. Les composés **37a, 37b, 37c, 37f, 37i** et **37j** porteurs d'un groupement hydroxypyridinone apparaissent de plus comme les plus réactifs avec une activité comparable à celle de l'hydralazine et une disparition presque totale du piégeur libre dès 15 min d'incubation. Nous avons également pu identifier trois types d'adduits possibles avec le MGO (Adduit A (AdA) de type 1:1 soit une molécule de piégeur pour deux molécules de MGO ; Adduit B (AdB) de type 1:2; Adduit C (AdC) de type 2:1) avec une évolution fréquente vers l'observation d'un adduit majoritaire de type 1:2, porteur d'un cycle pyrazine (AdB) (Figure 3). Enfin, il faut noter que les résultats obtenus avec le composé **29a** n'ont pas pu être représentés graphiquement (ND = Non déterminé) car il n'a pas été possible d'obtenir de séparation entre le piégeur libre et les adduits avec le MGO dans ce cas lors de l'analyse HPLC. Toutefois, une tendance vers une présence majoritaire de AdB vis-à-vis du piégeur libre semble se dégager après 5h de réaction.

### b) Évaluation de la formation d'adduits avec le MDA (Figure 4)

On observe notamment la formation d'un adduit porteur d'un cycle 2,3-dihydro-1*H*-1,4-diazépine (Adduit D (AdD)) avec l'ensemble des composés selon l'invention (Figure 4 et Figure 5). Les dérivés **30b, 37c, 37d, 37f, 37i** et **37j** se sont révélés être les plus réactifs avec une disparition du piégeur testé conséquente au profit des adduits avec le MDA au bout de 1h (> 81% d'adduits). Notons que les résultats obtenus avec le composé **29a** n'ont une nouvelle fois pas pu être représentés graphiquement (ND = Non déterminé) car comme avec le MGO, nous n'avons pas pu retrouver de séparation entre le piégeur libre et les adduits avec le MDA lors de l'analyse HPLC. Dans ce cas, une tendance vers une présence majoritaire de AdD vis-à-vis du piégeur libre semble se dégager seulement après 24h de réaction.

### II) Évaluation des propriétés chélatrices du Cu²⁺ des composés selon l'invention ((29a, 29b, 30a, 30b ; 37a, 37b, 37c, 37d, 37f, 37i, 37j) par spectrophotométrie UV/Visible

### 1- Principe :

Les composés à tester sont mis à incuber à TA en présence de Cu²⁺ pendant 10 min. La quantité de Cu²⁺ libre restante est déterminée après complexation par le murexide *(*Int. J. Mol. Sci., 2009, 10, 5485-5497 ; Molecules, 2012, 17, 13457-13472). Une mesure de l'absorbance à 485 nm (A₄₈₅) du complexe Cu²⁺/murexide (orangé) et de l'absorbance à 520 nm (A₅₂₀) du murexide libre (rose) est en effet réalisée par spectrophotométrie UV/Visible. La quantité de Cu²⁺ libre restante est alors évaluée à l'aide de l'équation de la droite de calibration donnant le rapport A₄₈₅/A₅₂₀ en fonction de la concentration en Cu²⁺ préalablement obtenue. On en déduit au final le pourcentage de complexation du Cu²⁺ par les composés testés.

### 2- Méthode :

### a) Préparation des solutions

Un tampon Hexamine 0,01 M / KCl 0,01 M (qsp 200 mL) dont le pH est ajusté à 5 à l'aide d'une solution d'HCl 1N est tout d'abord préparé. Des solutions de CuSO₄.5H₂O à 0,5 mM (20 mL) et 0,25 mM (100 mL) sont ensuite réalisées dans ce tampon, la première étant utilisée pour l'obtention de la courbe de calibration. Une solution aqueuse de murexide à 1 mM doit également être préparée extemporanément (10 mL) ainsi qu'une solution mère des composés à tester à 4,2 mM dans du tampon Hexamine 0,01 M / KCl 0,01 M ou dans un mélange de tampon et de MeOH 75/25 (6,2 mL).

### b) Incubation des mélanges à TA

Différentes concentrations des composés à tester sont réparties dans du tampon Hexamine 0,01M / KCl 0,01M (pH = 5) (Tableau 5) ou dans un mélange de tampon et de MeOH 75/25 dans des tubes à hémolyse (Tableau 6). On ajoute ensuite la solution de CuSO₄.5H₂O à 0,25 mM (1 mL) et on laisse incuber à TA pendant 10 min. Enfin, on introduit la solution aqueuse de murexide à 1 mM (0,1 mL) et on laisse à nouveau incuber à TA pendant 1 min. On mesure alors les absorbances à 485 nm (A₄₈₅) et 520 nm (A₅₂₀) par spectrophotométrie UV/Visible (Spectromètre V-650 JASCO) (mesures réalisées en triplicatas). On utilise un blanc contenant du tampon Hexamine 0,01 M / KCl 0,01 M (pH = 5) (2 mL) et de l'eau (0,1 mL), et l'éthylènediamine (EDA) comme référence de la littérature en tant que chélateur du Cu²⁺.

**Tableau 5 : Préparation des différentes concentrations de composé à tester dans du tampon Hexamine 0,01M / KCl 0,01M (pH = 5)**

| Concentration finale de composé à tester (mM) | Volume de solution mère de composé à tester (µL) | Volume de tampon Hexamine 0,01M / KCl 0,01M (pH = 5) (µL) |
|---|---|---|
| 0 | 0 | 1000 |
| 0,02 | 10 | 990 |
| 0,05 | 25 | 975 |
| 0,08 | 40 | 960 |
| 0,10 | 50 | 950 |
| 0,20 | 100 | 900 |
| 0,50 | 250 | 750 |
| 1 | 500 | 500 |
| 2 | 1000 | 0 |

**Tableau 6 : Préparation des différentes concentrations de composé à tester dans un mélange de tampon Hexamine 0,01 M / KCl 0,01 M (pH = 5) et de MeOH 75/25**

| Concentration finale de composé à tester (mM) | Volume de solution mère de composé à tester (µL) | Volume de tampon Hexamine 0,01 M / KCl 0,01M (pH = 5) (µL) | Volume de MeOH (µL) |
|---|---|---|---|
| 0 | 0 | 750 | 250 |
| 0,02 | 10 | 743 | 247 |
| 0,05 | 25 | 731 | 244 |
| 0,08 | 40 | 720 | 240 |
| 0,10 | 50 | 713 | 237 |
| 0,20 | 100 | 675 | 225 |
| 0,50 | 250 | 563 | 187 |
| 1 | 500 | 375 | 125 |
| 2 | 1000 | 0 | 0 |

Des courbes de calibration sont préalablement réalisées après répartition de différentes concentrations de CuSO₄.5H₂O dans du tampon Hexamine 0,01 M / KCl 0,01 M (pH = 5) (Tableau 7) ou dans un mélange de tampon et de MeOH 75/25 (Tableau 8) dans des tubes à hémolyse (mesures réalisées en triplicatas). Après introduction de la solution aqueuse de murexide à 1 mM (0,1 mL), on laisse incuber à TA pendant 1 min et on mesure finalement les absorbances à 485 nm (A₄₈₅) et 520 nm (A₅₂₀) par spectrophotométrie UV/Visible.

**Tableau 7 : Préparation des différentes concentrations de CuSO₄.5H₂O dans du tampon Hexamine 0,01M / KCl 0,01M (pH = 5) pour la réalisation des courbes de calibration**

| Concentration finale de CuSO₄.5H₂O (mM) | Volume de solution de CuSO₄.5H₂O à 0,5 mM (µL) | Volume de tampon Hexamine 0,01 M / KCl 0,01 M (pH = 5) (µL) |
|---|---|---|
| 0 | 0 | 2000 |
| 0,025 | 105 | 1895 |
| 0,050 | 210 | 1790 |
| 0,075 | 315 | 1685 |
| 0,100 | 420 | 1580 |
| 0,125 | 525 | 1475 |

**Tableau 8 : Préparation des différentes concentrations de CuSO₄.5H₂O dans un mélange de tampon Hexamine 0,01 M / KCl 0,01 M (pH = 5) et de MeOH 75/25 pour la réalisation des courbes de calibration**

| Concentration finale de CuSO₄.5H₂O (mM) | Volume de solution de CuSO₄.5H₂O à 0,5 mM (µL) | Volume de tampon Hexamine 0,01 M / KCl 0,01 M (pH = 5) (µL) | Volume de MeOH (µL) |
|---|---|---|---|
| 0 | 0 | 1750 | 0,250 |
| 0,025 | 105 | 1645 | 0,250 |
| 0,050 | 210 | 1540 | 0,250 |
| 0,075 | 315 | 1435 | 0,250 |
| 0,100 | 420 | 1330 | 0,250 |
| 0,125 | 525 | 1225 | 0,250 |

### 3- Résultats et discussion :

### a) Courbes de calibration

On obtient des droites (Figure 6, Figure 7) dont les équations permettront de déterminer la quantité de Cu²⁺ libre restante, après complexation par les composés à tester et d'évaluer au final leur pouvoir de complexation du Cu²⁺.

### b) Comparaison des propriétés chélatrices du Cu²⁺ des composés testés

Tout d'abord, les nouveaux composés selon l'invention apparaissent comme de meilleurs chélateurs du Cu²⁺ que les dérivés de 2^{ème} génération (Dap-Pip et Dap-(nBu)Pip) (Figure 8). Les composés **30b, 37b, 37i** et **37j** se sont ainsi révélés les plus actifs avec un pouvoir complexant proche, surtout pour le composé **30b,** de celui de l'EDA utilisée comme référence de la littérature. Au sein d'une même série, la comparaison des propriétés chélatrices du Cu²⁺ des composés **37a** et **37b** (composé **37b** meilleur complexant que le composé **37a**) montre que l'allongement de la chaîne carbonée porteuse du groupement diamine terminal semble comme attendu améliorer l'activité. L'éloignement de la fonction carbonyle permet donc bien une potentialisation de la capacité chélatrice du groupement diamine. Toutefois, le mauvais résultat obtenu avec le composé **29b** par rapport à ceux obtenus avec les composés **30b** et **37b** dont la chaîne carbonée est également dérivée de la lysine, suggère une intervention associée dans la complexation du Cu²⁺ du groupement dérivé de l'acide férulique, de l'acide gallique ou hydroxypyridinone introduit à l'extrémité opposée de la molécule. Afin de valider cette hypothèse, nous avons donc comparé les propriétés chélatrices du Cu²⁺ du composés **37b** et de deux composés apparentés présentant seulement une extrémité chélatrice du Cu²⁺ libre (composés **36b** et **38**) (Figure 9 et Figure 10). Ainsi, la perte du groupement diamine (composé **36b**) se révèle presque sans conséquence contrairement à celle du groupement hydroxypyridinone (composé **38**) qui apparaît donc bien essentiel à l'activité chélatrice du Cu²⁺ des nouveaux composés selon l'invention. De plus, la comparaison des résultats obtenus avec les composés **37c** et **37j** montre un pouvoir complexant du Cu²⁺ plus important du motif 3-hydroxy-2-méthylpyridin-4-one vis-à-vis du motif 3-hydroxypyridin-2-one. Finalement, compte tenu des mauvais résultats obtenus avec les composés **29a** et **29b,** le groupement dérivé de l'acide férulique semble présenter le pouvoir complexant du Cu²⁺ le moins intéressant.

### III) Évaluation des propriétés antioxydantes des composés selon l'invention (29a, 29b, 30a, 30b ; 37a, 37b, 37c, 37d, 37f, 37i, 37j) par un test ORAC (« Oxygen radical absorbance capacity »)

### 1- Principe :

Les propriétés antioxydantes des composés selon l'invention ont été évaluées grâce à un test ORAC_{FL} utilisant la fluorescéine (J. Agric. Food Chem.,2004 52, 48-54; J. Agric. Food Chem., 2005, 53, 4290-4302). Ainsi, des radicaux péroxydes, générés en présence de AAPH (2,2'-azobis(2-methylpropionamidine)dihydrochloride) à 37°C, réagissent avec cette sonde fluorescente pour donner un produit non fluorescent. L'effet protecteur des composés testés peut alors être déterminé en suivant la courbe de décroissance de la fluorescence de la fluorescéine dans le temps et en mesurant l'aire sous la courbe (AUC = « Area under the curve ») de l'échantillon par rapport à celle d'un contrôle correspondant à une absence d'antioxydant.

Le trolox (acide 6-hydroxy-2,5,7,8-tétraméthylchroman-2-carboxylique), un analogue hydrosoluble de la vitamine E, est utilisé comme standard pour le calcul de la capacité antioxydante (ORAC_{FL}) des composés testés à 10µM, exprimée en µmol d'équivalent trolox (TE = « Trolox équivalent »)/µmol de composé testé. Cette valeur est ainsi obtenue à l'aide de l'équation de la droite de calibration donnant l'aire sous la courbe en fonction de différentes concentrations de trolox (Bioorg. Med. Chem., 2015, 23, 1135-1148) (Figure 11).

### 2- Méthode :

Une solution de fluorescéine (FL) (12 nM; 150 µL) est introduite dans une plaque de 96 puits noire (Dutscher, Brumath, France). Les composés à tester (1-20 µM; 25 µL) ou le trolox (1-50 µM; 25 µL) dissous dans du D-PBS sont ajoutés dans chaque puits. Après équilibration de la plaque au cours d'une incubation à 37°C pendant un minimum de 30 min, les réactions radicalaires sont initiées à l'aide d'une solution de AAPH préparée extemporanément (30 mM; 25 µL). La fluorescence (λ_{Ex}: 485 nm; λ_{Em}: 520 nm) est mesurée toutes les 90 secondes pendant 60 cycles grâce à un lecteur de microplaques thermostaté Tecan Infinite® 200 PRO (mesures réalisées en triplicatas au cours de trois expériences indépendantes).

### 3- Résultats et discussion :

Les composés selon l'invention ont montré une importante capacité antioxydante (Tableau 9: ORAC_{FL} ≥ 1 µmol TE/µmol), comparée notamment à la très faible activité retrouvée pour la carnosine. De plus, l'introduction du groupement issu de l'acide férulique, de l'acide gallique ou du motif hydroxypyridinone apparaît indispensable à l'acquisition de ces propriétés antioxydantes compte tenu de l'absence d'activité retrouvée pour le dérivé Dap-Pip de 2^{ème} génération. Les composés **30b** et **37c** se sont ainsi révélés les plus actifs et seront de ce fait retenus comme les chefs de file de leurs familles respectives. Notons que la comparaison des résultats obtenus avec les composés **37c** et **37j** était dans ce cas en faveur d'une meilleure capacité antioxydante du motif 3-hydroxypyridin-2-one.

**Tableau 9 : Capacité antioxydante (ORAC_{FL}) des composés selon l'invention à 10 µM (Les valeurs sont exprimées par la moyenne ± SEM (« Standard error of the mean ») de trois expériences indépendantes réalisées en triplicatas).**

| Composé testé | ORAC_{FL} (µmol TE/µmol) |
|---|---|
| Trolox | 1 |
| Carnosine | 0,08 ± 0,05 |
| Dap-Pip | 0 |
| Composé **29a** | 2,05 ± 0,23 |
| Composé **29b** | 1,50 ± 0,10 |
| Composé **30a** | 0,89 ± 0,10 |
| Composé **30b** | 2,59 ± 0,35 |
| Composé **37a** | 0,58 ± 0,02 |
| Composé **37b** | 0,99 ± 0,09 |
| Composé **37c** | 1,03 ± 0,03 |
| Composé **37d** | 1,21 ± 0,07 |
| Composé **37f** | 1,96 ± 0,24 |
| Composé **37i** | 1,24 ± 0,02 |
| Composé **37j** | 1,01 ± 0,06 |

### C. ÉVALUATION BIOLOGIQUE IN VITRO

### I) Évaluation in vitro des propriétés antiradicalaires des composés selon l'invention (29a, 29b, 30a, 30b ; 37a, 37b, 37c)

### 1- Principe :

Les propriétés antiradicalaires des différents composés sont déterminées par inhibition de la peroxydation lipidique dans le modèle *in vitro* de l'oxydation des lipoprotéines de basse densité (LDL) (Free Radic. Biol. Med., 1992, 13, 341-390). La peroxydation lipidique est initiée par l'attaque par des radicaux libres d'une double liaison d'un acide gras polyinsaturé présent dans les LDL. Ceci résulte en l'élimination d'un atome d'hydrogène d'un groupement CH₂. Ce radical instable se réarrange pour aboutir alors à une configuration plus stable à savoir un diène conjugué. Une fois initiée, l'oxydation des LDL est une réaction en chaîne de peroxydation lipidique engendrée par les radicaux libres. L'oxydation *in vitro* des LDL est induite à 37°C par addition du composé hydrosoluble 2,2'-Azobis(2-methylpropionamidine)dihydrochloride (AAPH), qui génère des radicaux libres lors de sa décomposition thermique spontanée.

### 2- Méthode :

Brièvement, l'oxydation, réalisée en plaque de 96 puits, est induite à 37°C en ajoutant 20 µL d'une solution de AAPH à 2 mM dans du D-PBS à 160 µL de LDL (100 µg/mL) en présence ou absence de 20 µL des différentes solutions des composés à tester dissous dans du D-PBS (0,1 à 100 µM en concentrations finales). Les LDL seules sans ajout de AAPH sont utilisées comme contrôle négatif. Chaque oxydation est réalisée en double. Pendant l'oxydation, la formation des diènes conjugués est suivie par la mesure de la densité optique à 234 nm toutes les 10 minutes pendant 8h à 37°C à l'aide d'un spectrophotomètre thermostaté TECAN. La vitamine E (α-tocophérol) qui est un puissant antioxydant reconnu dans ce modèle *in vitro* est utilisée comme molécule de référence (J. Nutr. Biochem., 2012, 23, 845-51).

### 3- Résultats et discussion :

Le dérivé Dap-Pip de 2^{ème} génération ne montre aucun effet antiradicalaire même à forte concentration (100 µM) (Figure 27).

Les composés **29a** et **29b,** dérivés de l'acide férulique se comportent de façon similaire (Figure 28 et Figure 29). En effet, les résultats montrent que ces produits sont antioxydants pour des concentrations allant de 25 à 100 µM, mais deviennent pro-oxydants à des concentrations plus faibles (de 0,1 à 10 µM).

Le composé **30b,** dérivé de l'acide gallique est très pro-oxydant pour des concentrations allant de 50 à 100 µM, mais devient fortement antioxydant aux concentrations les plus faibles (0,1 à 10 µM) (Figure 30).

Les composés **37a** et **37c,** porteurs d'un groupement hydroxypyridinone se comportent de façon similaire. Ils ont un effet antioxydant croissant pour des concentrations allant de 1 à 100 µM (Figure 31 et Figure 32).

A des concentrations faibles de 1 à 10 µM, les composés **30b, 37a** et **37c** selon l'invention présentent des propriétés anti-radicalaires supérieures à celles du dérivé Dap-Pip de 2^{ème} génération, et même à celles de la vitamine E (Figure 33 et Figure 34). L'efficacité de cette 3^{ème} génération de composés antioxydants vis-à-vis à la fois de dérivés apparentés antérieurs, mais aussi d'un produit de référence, a donc pu être démontrée.

Le comportement assez atypique du composé **30b** (antioxydant à faibles concentrations et pro-oxydant à fortes concentrations) reste encore à étudier. Au contraire, le profil des composés **29a** et **29b** (pro-oxydants à faibles concentrations et antioxydants à fortes concentrations) peut trouver des échos dans certains travaux récents décrits dans la littérature (J. Agric. Food Chem., 2000, 48, 3597-3604 ; J. Agric. Food Chem., 2010, 58, 9273-9280).

Au final, les composés **37a** et **37c,** porteurs d'un groupement hydroxypyridinone semblent avoir les meilleures propriétés antioxydantes et ce à des concentrations faibles de l'ordre de 1 µM.

### II) Étude de la cytotoxicité des composés selon l'invention

Les lignées cellulaires utilisées aussi bien pour l'étude de la cytotoxicité des composés selon l'invention que pour l'évaluation de leurs propriétés antiapoptotiques ont été sélectionnées compte tenu des revendications précédemment exposées, en vue d'une utilisation en cosmétique ou dans le traitement et/ou la prévention notamment de l'athérosclérose et des maladies neurodégénératives des composés selon l'invention. Ainsi, ces différentes études ont pu être réalisées sur des fibroblastes humains (MRC-5), des cellules endothéliales cérébrales murines (bEnd.3) et des cellules de phéochromocytome de rat, assimilées à des cellules neuronales (PC12) *(*Eur. J. Med. Chem., 2014, 83, 355-365 ; Chem. Biol. Interact., 2014, 224, 108-116 ; Neurochem. Int., 2013, 62, 620-625).

### 1- Principe :

La viabilité cellulaire est évaluée par une méthode colorimétrique basée sur la détection de la conversion d'un sel du tétrazolium (WST-8) par les cellules métaboliquement actives (kit CCK-8, Sigma, Lyon, France) (Molecules, 2014, 19(8), 12048-12064 ; J. Pharmacol. Toxicol. Methods., 2007, 56(1), 58-62). Les cellules vivantes possèdent des déshydrogénases mitochondriales qui réduisent le WST-8 (2-(2-methoxy-4-nitrophenyl)-3-(4-nitrophenyl)-5-(2,4-disulfophenyl)-2H-tetrazolium), monosodium salt) en formazan de couleur orangée, qui se dissout directement dans le milieu de culture en présence de 1-Methoxy PMS (1-methoxy-5-methylphenazinium methyl sulphate). Après une incubation de 1h à 37°C, l'absorbance du formazan est mesurée à 450 nm. L'absorbance est directement proportionnelle au nombre de cellules vivantes.

### 2- Méthode :

Brièvement, les cellules sont ensemencées en plaques 96 puits à raison de 5.10³ cellules par puits dans 100 µL du milieu adéquat jusqu'à subconfluence. Les cellules sont ensuite lavées au D-PBS, puis traitées avec différentes concentrations (10 µM, 100 µM) des produits à tester en triplicatas pendant 24 et 48h. Un contrôle positif est réalisé avec 10% de DMSO. La solution CCK-8 (10 µL) est ensuite ajoutée dans chaque puits pour une incubation de 1h à 37°C. La mesure de l'absorbance est effectuée à 450 nm à l'aide d'un lecteur de microplaques Perkin Elmer 2103 Envision®.

La viabilité des cellules est exprimée en % du contrôle (cellules non traitées) par la moyenne ± écart-type de triplicatas formant une expérience représentative ou de trois expériences indépendantes réalisées en triplicatas.

### 3- Résultats et discussion :

**Tableau 10 : Étude de la cytotoxicité des composés selon l'invention sur trois lignées cellulaires (cellules endothéliales cérébrales murines (bEnd.3), cellules de phéochromocytome de rat, assimilées à des cellules neuronales (PC12) et fibroblastes humains (MRC-5)) après 24h de traitement. La viabilité des cellules est exprimée en pourcentage des cellules non traitées (100% conditions contrôle).**

| **Lignée cellulaire** | bEnd.3 | | PC12 | | MRC-5 | |
|---|---|---|---|---|---|---|
| **Concentration (µM)** | 10 | 100 | 10 | 100 | 10 | 100 |
| **Contrôle** | 100 | 100 | 100 | 100 | 100 | 100 |
| **Dap-Pip** | 123 | 103 | 114 | 120 | 125 | 128 |
| **Composé 29a** | 98 | 87 | 100 | 120 | 103 | 99 |
| **Composé 29b** | 92 | 86 | 108 | 112 | 106 | 102 |
| **Composé 30a** | 117 | 96 | 116 | 140 | 115 | 112 |
| **Composé 30b** | 93 | 99 | 110 | 82 | 98 | 101 |
| **Composé 37a** | 97 | 103 | 93 | 112 | 112 | 124 |
| **Composé 37b** | 126 | 115 | 138 | 124 | 113 | 113 |
| **Composé 37c** | 99 | 85 | 110 | 102 | 97 | 89 |

**Tableau 11 : Étude de la cytotoxicité des composés selon l'invention sur trois lignées cellulaires (cellules endothéliales cérébrales murines (bEnd.3), cellules de phéochromocytome de rat, assimilées à des cellules neuronales (PC12) et fibroblastes humains (MRC-5)) après 48h de traitement. La viabilité des cellules est exprimée en pourcentage des cellules non traitées (100% conditions contrôle).**

| **Lignée cellulaire** | bEnd.3 | | PC12 | | MRC-5 | |
|---|---|---|---|---|---|---|
| **Concentration (µM)** | 10 | 100 | 10 | 100 | 10 | 100 |
| **Contrôle** | 100 | 100 | 100 | 100 | 100 | 100 |
| **Dap-Pip** | 131 | 142 | 110 | 102 | 113 | 111 |
| **Composé 29a** | 90 | 83 | 94 | 87 | 104 | 103 |
| **Composé 29b** | 92 | 90 | 95 | 97 | 95 | 100 |
| **Composé 30a** | 95 | 108 | 96 | 113 | 101 | 103 |
| **Composé 30b** | 92 | 87 | 95 | 67 | 92 | 100 |
| **Composé 37a** | 99 | 93 | 96 | 99 | 107 | 105 |
| **Composé 37b** | 101 | 100 | 115 | 107 | 103 | 101 |
| **Composé 37c** | 86 | 84 | 91 | 94 | 94 | 90 |

La tendance générale qui se dégage de ces premiers résultats (mesures réalisées en triplicatas formant une expérience représentative) révèle a priori une faible cytotoxicité voire une absence de cytotoxicité des composés selon l'invention sur les trois lignées cellulaires testées à 10 et 100 µM après 24h (Tableau 10, Figure 35, Figure 36, Figure 37) ou 48h de traitement (Tableau 11, Figure 38, Figure 39, Figure 40). Quelques données moins satisfaisantes apparaissent toutefois surtout pour une concentration élevée de 100 µM pour notamment les composés **29a, 30b** et **37c.** Le composé **30b** est ainsi à l'origine d'une diminution non négligeable de la viabilité cellulaire à 100 µM et après 48h de traitement sur les cellules PC12 (67% contrôle). Toutefois, cette cytotoxicité pourrait s'expliquer par son caractère pro-oxydant déjà observé à forte concentration lors de l'évaluation *in vitro* de ses propriétés antiradicalaires (Figure 30). Au final, une activité antiradicalaire supérieure à celle de la vitamine E ayant été retrouvée pour les dérivés les plus intéressants pour des concentrations inférieures ou égales à 10 µM (Figure 33, Figure 34), ces premiers résultats sur la cytotoxicité des composés selon l'invention apparaissent très encourageants.

Finalement, l'absence de cytotoxicité des composés selon l'invention a pu être confirmée après 24h de traitement sur les cellules PC12 au cours de trois expériences indépendantes réalisées en triplicatas (Figure 41).

### III) Évaluation des propriétés antiapoptotiques des composés selon l'invention sur différentes lignées cellulaires (29a, 29b, 30a, 30b ; 37a, 37b, 37c)

### 1- Principe :

L'apoptose est un mécanisme cellulaire intrinsèquement programmé de mort cellulaire, hautement régulé, qui constitue une réponse de l'organisme à des stimuli physiologiques ou pathologiques provoquant un déséquilibre entre la production et l'élimination de cellules. Ce mécanisme de mort programmée permet de maintenir l'homéostasie des tissus. Morphologiquement, l'apoptose correspond à une rétraction progressive de la cellule, avec condensation de la chromatine et du cytoplasme, suivie d'une fragmentation caractéristique régulière de l'ADN aboutissant à la formation de fragments cellulaires (fragmentation internucléosomale) ou corps apoptotiques. Une régulation inappropriée de l'apoptose joue un rôle important dans de nombreuses conditions pathologiques comme le cancer, l'autoimmunité, la maladie d'Alzheimer... (The Lancet, 1993, 381, 1251-1254 ; Toxicol Pathol., 2007, 35(4), 495-516).

Diverses études ont montré que le méthyglyoxal (MGO) induit l'apoptose dans de nombreux types cellulaires (Int. J. Mol, Med., 2010, 26, 813-818).

L'apoptose des cellules est évaluée par une méthode ELISA utilisant deux anticorps monoclonaux murins dirigés l'un contre l'ADN et l'autre contre les histones (kit Cell Death Detection ELISAPLUS, Roche, Meylan, France). Cette technique permet de mesurer spécifiquement les mono- et oligo-nucléosomes dans la fraction cytoplasmique des lysats cellulaires.

### 2- Méthode :

Brièvement, les cellules sont ensemencées en plaques 96 puits ou 24 puits à raison de 5.10³ cellules/puits pour les Bend.3 et PC12 et 75.10³ cellules/puits pour les MRC5 dans le milieu adéquat jusqu'à subconfluence. Les cellules sont ensuite lavées au PBS, puis traitées avec différentes concentrations (10 µM, 100 µM) des produits à tester en triplicatas pendant 30 min à 1h (cellules PC12 et Bend.3) ou 1h (cellules MRC5) à 37°C. La solution de MGO (1 mM pour les cellules PC12, 2 mM pour les cellules Bend.3 et MRC5) est ensuite ajoutée pour une incubation de 24h. Les concentrations de MGO pour chaque type cellulaire ont été choisies selon la littérature et un test de cytotoxicité (CCK8) que nous avons réalisé. La solution de lyse est ensuite ajoutée dans chaque puits pendant 30 min à température ambiante. Les lysats cellulaires sont ensuite centrifugés à 200 g pendant 10 min. Vingt microlitres de ce lysat cellulaire est alors utilisé pour l'ELISA selon la procédure décrite par le fournisseur. Les lysats cellulaires sont ainsi appliqués sur une plaque recouverte d'anticorps anti-Histones permettant de visualiser la fragmentation de l'ADN. Une peroxydase associée à des anticorps anti-ADN réagit ensuite en présence de son substrat, l'ABTS (diammonium 2,2'-azino-bis(3-ethylbenzothiazoline-6-sulfonate) et de H₂O₂ pour donner un dérivé vert dont la densité optique reflète le niveau d'apoptose. La mesure de l'absorbance est effectuée à 405 nm à l'aide d'un lecteur de microplaques Perkin Elmer 2103 Envision®. Un contrôle positif est réalisé en présence de MGO seul.

L'apoptose des cellules est exprimée par la moyenne ± écart-type de triplicatas formant une expérience représentative ou de trois expériences indépendantes réalisées en triplicatas.

### 3- Résultats et discussion :

Les composés **30b** et **37c** ont montré des propriétés antiapoptotiques très intéressantes dès 10 µM suite à l'induction de l'apoptose par le MGO sur des cellules endothéliales cérébrales murines (bEnd.3) (Figure 42, Figure 43). Ils apparaissent donc capables de ralentir la cascade cellulaire néfaste liée aux stress oxydant et carbonylé en vue de l'utilisation des composés selon l'invention dans le traitement et/ou la prévention de l'athérosclérose.

Le composé **37c** a révélé des propriétés antiapoptotiques très intéressantes dès 10 µM suite à l'induction de l'apoptose par le MGO sur des cellules de phéochromocytome de rat, assimilées à des cellules neuronales (PC12) (Figure 44, Figure 45). Il apparaît donc capable de ralentir la cascade cellulaire néfaste liée aux stress oxydant et carbonylé en vue de l'utilisation des composés selon l'invention dans le traitement et/ou la prévention des maladies neurodégénératives. Pour le composé **30b,** cette tendance se confirme à 100 µM.

Les composés **37b** et **37c** présentent des propriétés antiapoptotiques prometteuses à 100 µM suite à l'induction de l'apoptose par le MGO sur des fibroblastes humains (MRC-5) (Figure 46, Figure 47). Ils apparaissent donc capables de ralentir la cascade cellulaire néfaste liée aux stress oxydant et carbonylé en vue d'une application des composés selon l'invention dans le domaine cosmétique pour une prévention du vieillissement prématuré de la peau.

Finalement, les propriétés antiapoptotiques du composé **37c** à 100 µM suite à l'induction de l'apoptose par le MGO sur des cellules de phéochromocytome de rat, assimilées à des cellules neuronales (PC12) ont pu être confirmées au cours de trois expériences indépendantes réalisées en triplicatas (Figure 48).

## Revendications

1. Composé de Formule I: 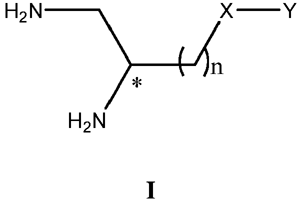
et ses sels,
dans laquelle
**n** est un nombre entier de 1 à 6 ;
**X** est CO ou CH₂ ;
**Y** est NR¹R² ou R² ou 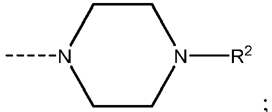
**R¹** est H ou alkyle ou alkyl-aryle ;
**R²** est **Z-L-R³** ;
**Z** est inexistant, CO ou CH₂ ;
**L** est inexistant, CH=CH ou (CH₂)ₘ ;
**m** est un nombre entier de 1 à 6 ;
**R³** est phényle, substitué par au moins un groupe OH et un ou plusieurs substituants choisis parmi OH, alkoxy en C1 à C4 et alkyle en C1 à C4, ou **R³** est N-pyridinonyle, substitué par au moins un groupe OH et éventuellement par un ou plusieurs substituants choisis parmi OH, alkoxy en C1 à C4 et alkyle en C1 à C4.

2. Composé ou sel selon la revendication 1, ayant la Formule II

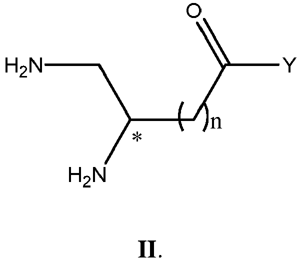

3. Composé ou sel selon la revendication 1, ayant la Formule III

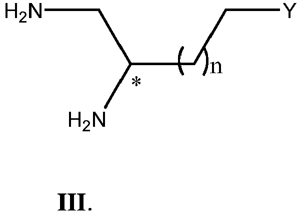

4. Composé ou sel selon la revendication 1, ayant la Formule IV

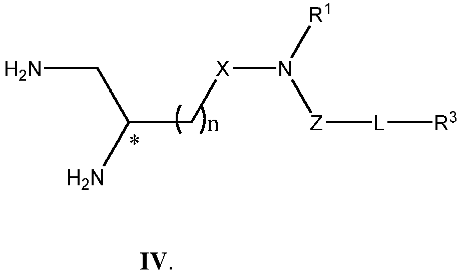

5. Composé ou sel selon la revendication 1, ayant la Formule V

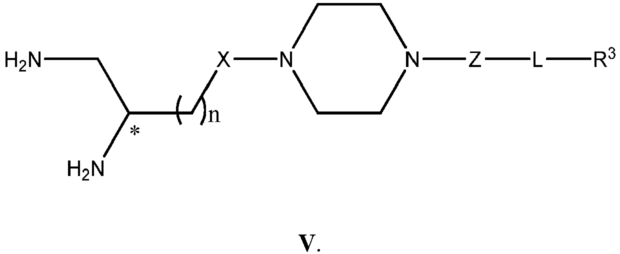

6. Composé ou sel selon la revendication 4 ou 5, dans lequel Z-L-R3 est choisi parmi : 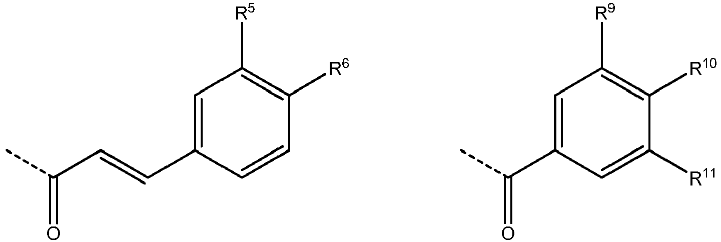 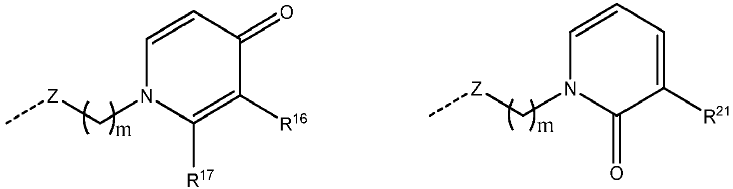 dans lesquels R⁵, R⁶, R⁹, R¹⁰, R¹¹, R¹⁶, R¹⁷ et R²¹ sont choisis, indépendamment les uns des autres, parmi OH, alkoxy en C1 à C4 et alkyle en C1 à C4

7. Composé ou sel selon la revendication 1, ayant la Formule VI

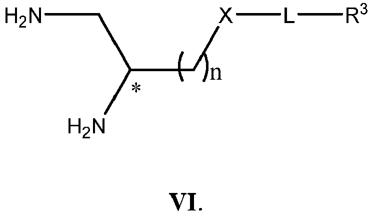

8. Composé ou sel selon la revendication 1 choisi parmi:
(E)-4,5-diamino- 1-(4-(3-(4-hydroxy-3-méthoxyphényl)acryloyl)pipérazin-1-yl)pentan-1-one,
(E)-*N*-(5,6-diaminohexyl)-3-(4-hydroxy-3-méthoxyphényl)acrylamide,
4,5-diamino-1-(4-(3,4,5-trihydroxybenzoyl)pipérazin-1-yl)pentan-1-one,
*N*-(5,6-diaminohexyl)-3,4,5-trihydroxybenzamide,
*N*-(4,5-diaminopentyl)-3-(3-hydroxy-2-oxopyridin-1(2*H*)-yl)propanamide,
*N*-(5,6-diaminohexyl)-3-(3-hydroxy-2-oxopyridin-1(2*H*)-yl)propanamide,
4,5-diamino-*N*-(3-(3-hydroxy-2-oxopyridin-1(2*H*)-yl)propyl)pentanamide.
1-(2-(4-(4,5-diaminopentanoyl)pipérazin-1-yl)-2-oxoéthyl)-3-hydroxypyridin-2(1*H*)-one
*N*-(4,5-diaminopentyl)-2-(3-hydroxy-2-méthyl-4-oxopyridin-1(4*H*)-yl)acetamide
*N*-(5,6-diaminohexyl)-2-(3-hydroxy-2-méthyl-4-oxopyridin-1(4*H*)-yl)acetamide
1-(2-(4-(4,5-diaminopentanoyl)pipérazin-1-yl)-2-oxoéthyl)-3-hydroxy-2-méthylpyridin-4(1*H*)-one
1-(3-(4-(4,5-diaminopentyl)pipérazin-1-yl)propyl)-3-hydroxypyridin-2(1*H*)-one
1-(3-((4,5-diaminopentyl)(méthyl)amino)propyl)-3-hydroxypyridin-2(1*H*)-one
1-(5,6-diaminohexyl)-3-hydroxy-2-méthylpyridin-4(1*H*)-one

9. Composition pharmaceutique comprenant au moins un composé ou l'un de ses sels pharmaceutiquement acceptables selon l'une quelconque des revendications 1 à 8 et au moins un excipient pharmaceutiquement acceptable.

10. Composé selon l'une quelconque des revendications 1 à 8 ou l'un de ses sels pharmaceutiquement acceptables pour utilisation en tant que médicament.

11. Composé ou sel pharmaceutiquement acceptable pour utilisation selon la revendication 10 dans le traitement et/ou la prévention de maladies ou affections associées à une accumulation de produits de glycation avancé et/ou de produits de peroxydation de lipides choisies parmi les maladies neurodégénératives, les pathologies liées à l'âge, les affections liées au diabète et le cancer.

12. Composé ou sel pharmaceutiquement acceptable pour utilisation selon la revendication 11 dans le traitement et/ou la prévention des maladies neurodégénératives, notamment parmi la maladie d'Alzheimer et la maladie de Parkinson.

13. Composé ou sel pharmaceutiquement acceptable pour utilisation selon la revendication 12 dans le traitement et/ou la prévention de la maladie d'Alzheimer ou de la maladie de Parkinson.

14. Composé ou sel pharmaceutiquement acceptable pour utilisation selon la revendication 11 dans le traitement et/ou la prévention des maladies les pathologies liées à l'âge choisies parmi la cataracte et les rhumatismes.

15. Composé ou sel pharmaceutiquement acceptable pour utilisation selon la revendication 11 dans le traitement et/ou la prévention des affections liées au diabète.

16. Composé ou sel pharmaceutiquement acceptable pour utilisation selon la revendication 15 dans le traitement et/ou la prévention de l'athérosclerose, de la rétinopathie, de la nephropathie, de la neuropathie, des micro et macroangiopathies, de la cataracte, l'amyloïdose, des troubles rhumatismaux et des ulcères variqueux et artériels.

17. Utilisation d'un composé selon l'une quelconque des revendications 1 à 8 en tant que principe actif cosmétique ou ingrédient agroalimentaire.

18. Composition cosmétique comprenant un composé selon l'une quelconque des revendications 1 à 8.

19. Composition agroalimentaire comprenant un composé selon l'une quelconque des revendications 1 à 8.

## Patentansprüche

1. Verbindung der Formel I: 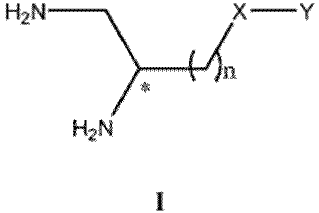 und ihre Salze,
wobei:
**n** eine ganze Zahl von 1 bis 6 ist;
**X** CO oder CH₂ ist;
**Y** NR¹R² oder R² oder ist 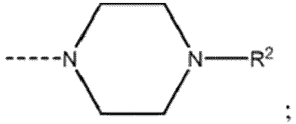
**R¹** H oder Alkyl oder Alkylaryl ist;
**R² Z-L-R³** ist;
**Z** fehlt, CO oder CH₂ ist;
**L** fehlt, CH=CH oder (CH₂)ₘ ist;
**m** eine ganze Zahl von 1 bis 6 ist;
**R³** Phenyl ist, substituiert durch mindestens eine OH-Gruppe und einen oder mehrere Substituenten, ausgewählt aus OH, C1- bis C4-Alkoxy und C1- bis C4-Alkyl, oder **R³** N-Pyridinonyl ist, substituiert durch mindestens eine OH-Gruppe und gegebenenfalls durch einen oder mehrere Substituenten, ausgewählt aus OH, C1- bis C4-Alkoxy und C1- bis C4-Alkyl.

2. Verbindung oder Salz nach Anspruch 1 mit der Formel II

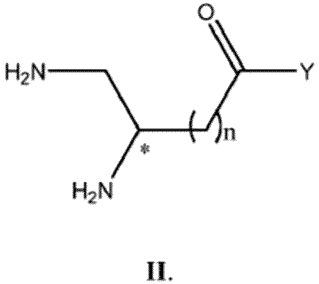

3. Verbindung oder Salz nach Anspruch 1 mit der Formel III

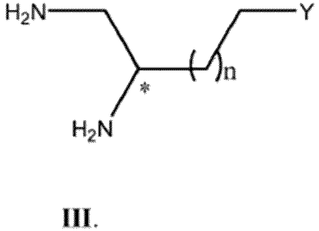

4. Verbindung oder Salz nach Anspruch 1 mit der Formel IV

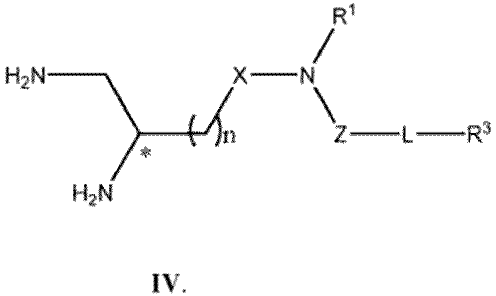

5. Verbindung oder Salz nach Anspruch 1 mit der Formel V

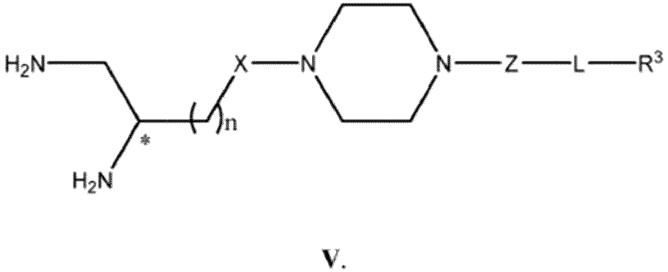

6. Verbindung oder Salz nach Anspruch 4 oder 5, wobei Z-L-R3 ausgewählt ist aus: 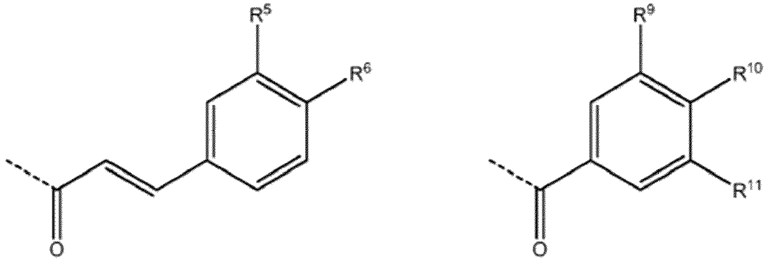 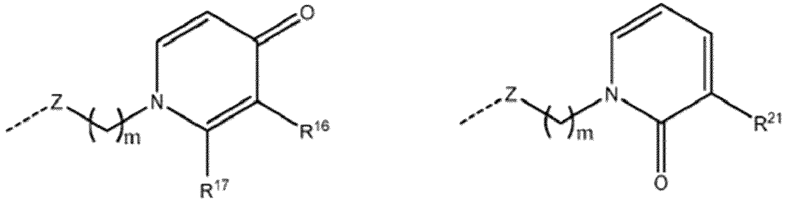 wobei R⁵, R⁶, R⁹, R¹⁰, R¹¹, R¹⁶, R¹⁷ und R²¹ unabhängig voneinander ausgewählt sind aus OH, C1- bis C4-Alkoxy und C1- bis C4-Alkyl.

7. Verbindung oder Salz nach Anspruch 1 mit der Formel VI

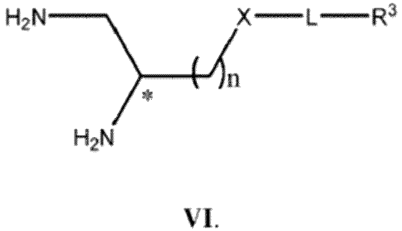

8. Verbindung oder Salz nach Anspruch 1, ausgewählt aus:
(E)-4,5-Diamino-1-(4-(3-(4-hydroxy-3-methoxyphenyl)acryloyl)piperazin-1-yl)pentan-1-on,
(E)-*N*-(5,6-Diaminohexyl)-3-(4-hydroxy-3-methoxyphenyl)acrylamid,
4,5-Diamino-1-(4-(3,4,5-trihydroxybenzoyl)piperazin-1yl)pentan-1-on,
*N*-(5,6-Diaminohexyl)-3,4,5-trihydroxybenzamid,
*N*-(4,5-Diaminopentyl)-3-(3-hydroxy-2-oxopyridin-1(2*H*)-yl)propanamid,
*N*-(5,6-Diaminohexyl)-3-(3-hydroxy-2-oxopyridin-1(2*H*)-yl)propanamid,
4,5-Diamino-*N*-(3-(3-hydroxy-2-oxopyridin-1(2*H*)-yl)propyl)pentanamid,
1-(2-(4-(4,5-Diaminopentanoyl)piperazin-1-yl)-2-oxoethyl)-3-hydroxypyridin-2(1*H*)-on
*N*-(4,5-Diaminopentyl)-2-(3-hydroxy-2-methyl-4-oxopyridin-1(4*H*)-yl)acetamid
*N*-(5,6-Diaminohexyl)-2-(3-hydroxy-2-methyl-4-oxopyridin-1(4*H*)-yl)acetamid
1-(2-(4-(4,5-Diaminopentanoyl)piperazin-1-yl)-2-oxoethyl)-3-hydroxy-2-methylpyridin-4(1*H*)-on
1-(3-(4-(4,5-Diaminopentyl)piperazin-1-yl)propyl)-3-hydroxypyridin-2(1*H*)-on
1-(3-((4,5-Diaminopentyl)(methyl)amino)propyl)-3-hydroxypyridin-2(1*H*)-on
1-(5,6-Diaminohexyl)-3-hydroxy-2-methylpyridin-4(1*H*)-on

9. Pharmazeutische Zusammensetzung, umfassend mindestens eine Verbindung oder eines ihrer pharmazeutisch unbedenklichen Salze nach einem der Ansprüche 1 bis 8 und mindestens einen pharmazeutisch unbedenklichen Hilfsstoff.

10. Verbindung nach einem der Ansprüche 1 bis 8 oder eines ihrer pharmazeutisch unbedenklichen Salze zur Verwendung als Medikament.

11. Verbindung oder pharmazeutisch unbedenkliches Salz zur Verwendung nach Anspruch 10 bei der Behandlung und/oder Vorbeugung von Erkrankungen oder Störungen, die mit einer Akkumulation von Produkten einer fortgeschrittenen Glykation und/oder Produkten der Peroxidation von Lipiden verbunden sind, ausgewählt aus neurodegenerativen Erkrankungen, mit dem Alter verbundenen Pathologien sowie mit Diabetes und Krebs verbundenen Störungen.

12. Verbindung oder pharmazeutisch unbedenkliches Salz zur Verwendung nach Anspruch 11 bei der Behandlung und/oder Vorbeugung von neurodegenerativen Erkrankungen, insbesondere Morbus Alzheimer und Morbus Parkinson.

13. Verbindung oder pharmazeutisch unbedenkliches Salz zur Verwendung nach Anspruch 12 bei der Behandlung und/oder Vorbeugung von Morbus Alzheimer oder Morbus Parkinson.

14. Verbindung oder pharmazeutisch unbedenkliches Salz zur Verwendung nach Anspruch 11 bei der Behandlung und/oder Vorbeugung von Erkrankungen von mit dem Alter verbundenen Pathologien, ausgewählt aus grauem Star und Rheumatismus.

15. Verbindung oder pharmazeutisch unbedenkliches Salz zur Verwendung nach Anspruch 11 bei der Behandlung und/oder Vorbeugung von mit Diabetes verbundenen Störungen.

16. Verbindung oder pharmazeutisch unbedenkliches Salz zur Verwendung nach Anspruch 15 bei der Behandlung und/oder Vorbeugung von Atherosklerose, Retinopathie, Nephropathie, Neuropathie, Mikro- und Makroangiopathien, grauem Star, Amyloidose, rheumatischen Beschwerden und varikösen und arteriellen Geschwüren.

17. Verwendung einer Verbindung nach einem der Ansprüche 1 bis 8 als kosmetischer Wirkstoff oder Nahrungsmittelbestandteil.

18. Kosmetische Zusammensetzung, umfassend eine Verbindung nach einem der Ansprüche 1 bis 8.

19. Nahrungsmittelzusammensetzung, umfassend eine Verbindung nach einem der Ansprüche 1 bis 8.

## Claims

1. Compound of Formula I: 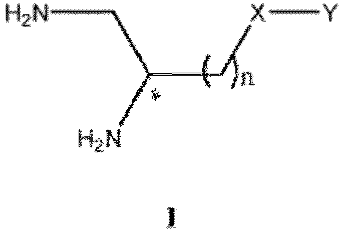
and salts thereof,
wherein
**n** is an integer from 1 to 6;
**X** is CO or CH₂;
**Y** is NR¹R² or R² or 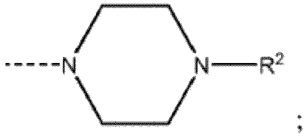
**R¹** is H or alkyl or alkyl-aryl;
**R²** is **Z-L-R³;**
**Z** is non-existent, CO or CH₂;
**L** is non-existent, CH=CH or (CH₂)ₘ;
**m** is an integer from 1 to 6;
**R³** is phenyl, substituted by at least one OH group and one or more substituents selected from OH, C1 to C4 alkoxy and C1 to C4 alkyl, or **R³** is N-pyridinonyl, substituted by at least one OH group and optionally by one or more substituents selected from OH, C1 to C4 alkoxy and C1 to C4 alkyl.

2. Compound or salt according to claim 1, having Formula II

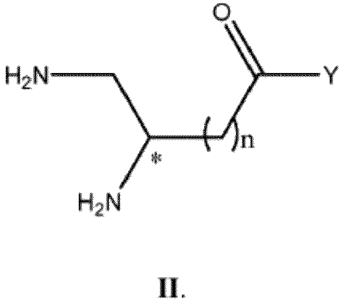

3. Compound or salt according to claim 1, having Formula III

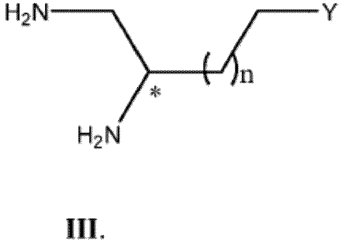

4. Compound or salt according to claim 1, having Formula IV

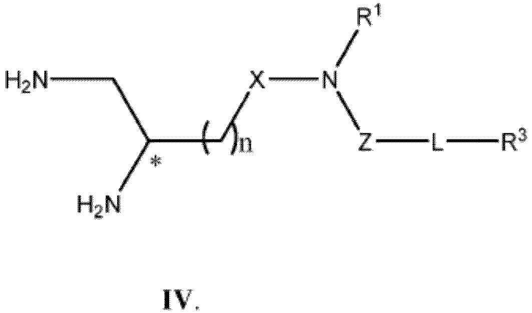

5. Compound or salt according to claim 1, having Formula V

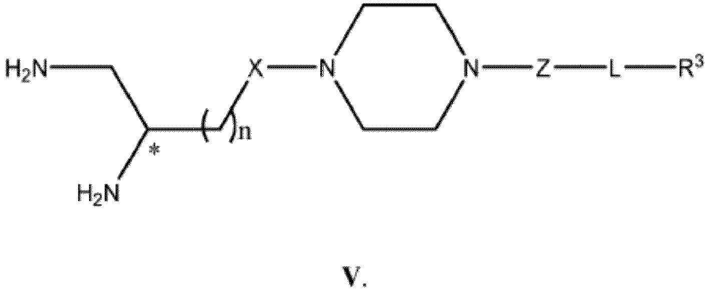

6. Compound or salt according to claim 4 or 5, wherein Z-L-R³ is selected from: 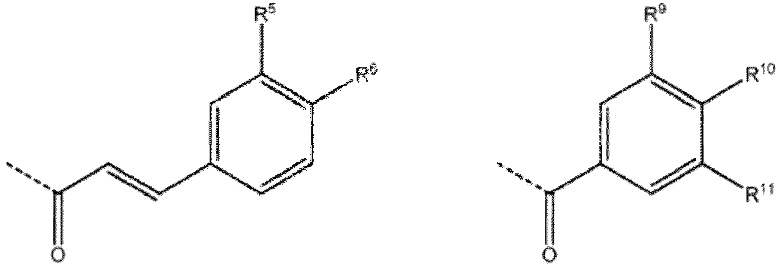 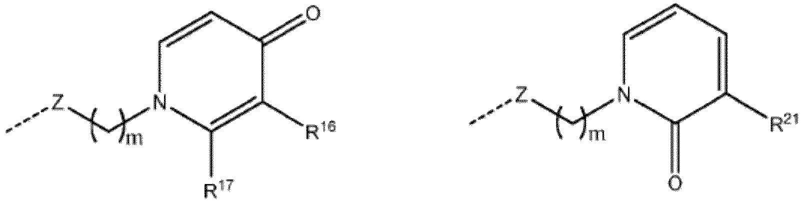 wherein R⁵, R⁶, R⁹, R¹⁰, R¹¹, R¹⁶, R¹⁷ and R²¹ are selected, independently of each other, from OH, C1 to C4 alkoxy and C1 to C4 alkyl.

7. Compound or salt according to claim 1, having Formula VI

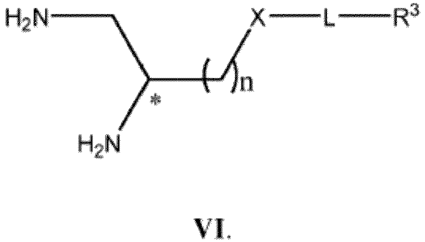

8. Compound or salt according to claim 1, selected from:
(E)-4,5-diamino-1-(4-(3-(4-hydroxy-3-methoxyphenyl)acryloyl)piperazin-1-yl)pentan-1-one,
(E)-*N*-(5,6-diaminohexyl)-3-(4-hydroxy-3-methoxyphenyl)acrylamide,
4,5-diamino-1-(4-(3,4,5-trihydroxybenzoyl)piperazin-1-yl)pentan-1-one,
*N*-(5,6-diaminohexyl)-3,4,5-trihydroxybenzamide,
*N*-(4,5-diaminopentyl)-3-(3-hydroxy-2-oxopyridin-1(2*H*)-yl)propanamide,
*N*-(5,6-diaminohexyl)-3-(3-hydroxy-2-oxopyridin-1(2*H*)-yl)propanamide,
4,5-diamino-*N*-(3-(3-hydroxy-2-oxopyridin-1(2*H*)-yl)propyl)pentanamide,
1-(2-(4-(4,5-diaminopentanoyl)piperazin-1-yl)-2-oxoethyl)-3-hydroxypyridin-2(1*H*)-one
*N*-(4,5-diaminopentyl)-2-(3-hydroxy-2-methyl-4-oxopyridin-1(4*H*)-yl)acetamide
*N*-(5,6-diaminohexyl)-2-(3-hydroxy-2-methyl-4-oxopyridin-1(4*H*)-yl)acetamide
1-(2-(4-(4,5-diaminopentanoyl)piperazin-1-yl)-2-oxoethyl)-3-hydroxy-2-methylpyridin-4(1*H*)-one
1-(3-(4-(4,5-diaminopentyl)piperazin-1-yl)propyl)-3-hydroxypyridin-2(1*H*)-one
1-(3-((4,5-diaminopentyl)(methyl)amino)propyl)-3-hydroxypyridin-2(1*H*)-one
1-(5,6-diaminohexyl)-3-hydroxy-2-methylpyridin-4(1*H*)-one.

9. Pharmaceutical composition comprising at least one compound or one of the pharmaceutically acceptable salts thereof according to any one of claims 1 to 8 and at least one pharmaceutically acceptable excipient.

10. Compound according to any one of claims 1 to 8 or one of the pharmaceutically acceptable salts thereof for use as a drug.

11. Compound or pharmaceutically acceptable salt for use according to claim 10 in treating and/or preventing illnesses or diseases associated with an accumulation of advanced glycation products and/or lipid peroxidation products selected from neurodegenerative illnesses, age-related pathologies, diseases related to diabetes and cancer.

12. Compound or pharmaceutically acceptable salt for use according to claim 11 in treating and/or preventing neurodegenerative illnesses, in particular from Alzheimer's disease and Parkinson's disease.

13. Compound or pharmaceutically acceptable salt for use according to claim 12 in treating and/or preventing Alzheimer's disease or Parkinson's disease.

14. Compound or pharmaceutically acceptable salt for use according to claim 11 in treating and/or preventing illnesses and age-related pathologies selected from cataract and rheumatism.

15. Compound or pharmaceutically acceptable salt for use according to claim 11 in treating and/or preventing diseases related to diabetes.

16. Compound or pharmaceutically acceptable salt for use according to claim 15 in treating and/or preventing atherosclerosis, retinopathy, nephropathy, neuropathy, micro- and macroangiopathies, cataract, amyloidosis, rheumatic illnesses and varicose and arterial ulcers.

17. Use of a compound according to any one of claims 1 to 8 as a cosmetic active principle or food ingredient.

18. Cosmetic composition comprising a compound according to any one of claims 1 to 8.

19. Food composition comprising a compound according to any one of claims 1 to 8.
